# EUROPEAN PATENT APPLICATION

(11) **EP 4 141 000 A1**
(43) Date of publication of application: **01.03.2023**
(21) Application number: 21793293.8
(22) Date of filing: 15.04.2021
(51) Int. Cl.: C07D 403/14, C07D 405/14, C07D 409/14, C07D 403/04, C07F 7/08, H01L 51/00

(54) **HETEROCYCLIC COMPOUND, ORGANIC LIGHT-EMITTING DEVICE COMPRISING SAME, MANUFACTURING METHOD THEREFOR, AND COMPOSITION FOR ORGANIC LAYER**

(30) Priority: 21.04.2020 KR 20200048060
(71) Applicant: LT Materials Co., Ltd., Yongin-si, Gyeonggi-do 17118 (KR)
(72) Inventor: NO, Young-Seok, Yongin-si, Gyeonggi-do 17118 (KR); PARK, Geon-Yu, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2021/004764
(87) International publication number: WO 2021/215742

(57) **Abstract**

The present specification relates to a heterocyclic compound represented by Chemical Formula 1, an organic light emitting device including the same, a method for manufacturing the same, and a composition for an organic material layer.

## Description

### [Technical Field]

The present specification relates to a heterocyclic compound, an organic light emitting device including the same, a method for manufacturing the same, and a composition for an organic material layer.

The present specification claims priority to and the benefits of Korean Patent Application No. 10-2020-0048060, filed with the Korean Intellectual Property Office on April 21, 2020, the entire contents of which are incorporated herein by reference.

### [Background Art]

An electroluminescent device is one type of self-emissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a host-dopant-based light emitting layer may also be used. In addition thereto, compounds capable of performing roles of hole injection, hole transfer, electron blocking, hole blocking, electron transfer, electron injection and the like may also be used as a material of the organic thin film.

Development of an organic thin film material has been continuously required for enhancing performance, lifetime or efficiency of an organic light emitting device.

### [Disclosure]

### [Technical Problem]

The present disclosure is directed to providing a heterocyclic compound, an organic light emitting device including the same, a method for manufacturing the same, and a composition for an organic material layer.

### [Technical Solution]

One embodiment of the present application provides a heterocyclic compound represented by the following Chemical Formula 1.

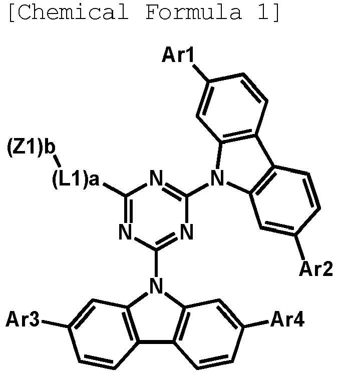

In Chemical Formula 1,
Ar1 to Ar4 are the same as or different from each other, and each independently hydrogen; deuterium; a substituted or unsubstituted C1 to C60 alkyl group; a substituted or unsubstituted C6 to C60 aryl group; or a substituted or unsubstituted C2 to C60 heteroaryl group,
L1 is a direct bond; a substituted or unsubstituted C6 to C60 arylene group; or a substituted or unsubstituted C2 to C60 heteroarylene group,
Z1 is selected form the group consisting of halogen; -CN; a substituted or unsubstituted C1 to C60 alkyl group; a substituted or unsubstituted C10 to C60 aryl group; a C2 to C60 heteroaryl group substituted or unsubstituted and including O or S as a heteroatom; -P(=O)RR'; -SiRR'R"; and a substituted or unsubstituted amine group,
when Z1 is a dicyclic or tetracyclic C15 to C60 aryl group substituted or unsubstituted and having the number of protons of 9 or less, L1 is a substituted or unsubstituted C6 to C60 arylene group; or a substituted or unsubstituted C2 to C60 heteroarylene group,
R, R' and R" are the same as or different from each other, and each independently a substituted or unsubstituted C1 to C60 alkyl group; a substituted or unsubstituted C6 to C60 aryl group; or a substituted or unsubstituted C2 to C60 heteroaryl group,
a is an integer of 1 to 4,
b is an integer of 1 to 6, and
when a and b are each 2 or greater, substituents in each parenthesis are the same as or different from each other.

In addition, one embodiment of the present application provides an organic light emitting device including a first electrode; a second electrode provided opposite to the first electrode; and one or more organic material layers provided between the first electrode and the second electrode, wherein one or more layers of the organic material layers include the heterocyclic compound represented by Chemical Formula 1.

In addition, in the organic light emitting device provided in one embodiment of the present application, the organic material layer including the heterocyclic compound of Chemical Formula 1 further includes a heterocyclic compound represented by the following Chemical Formula 2.

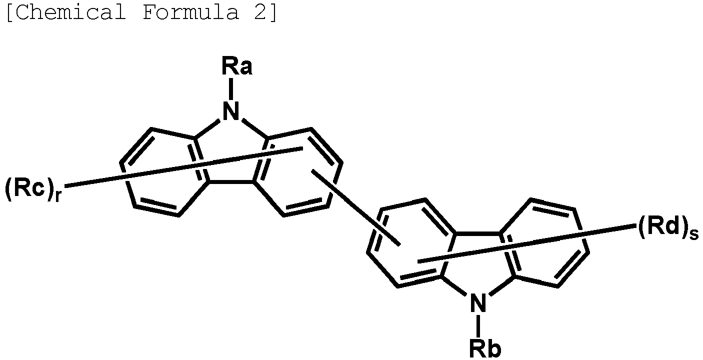

In Chemical Formula 2,
Rc and Rd are the same as or different from each other, and each independently selected from the group consisting of hydrogen; deuterium; a halogen group; -CN; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -Si(R10) (R11) (R12); - P(=O)(R10)(R11); and a substituted or unsubstituted amine group, or two or more groups adjacent to each other bond to each other to form a substituted or unsubstituted C6 to C60 aromatic hydrocarbon ring or a substituted or unsubstituted C2 to C60 heteroring,
R10, R11 and R12 are the same as or different from each other, and each independently hydrogen; deuterium; -CN; a substituted or unsubstituted C1 to C60 alkyl group; a substituted or unsubstituted C6 to C60 aryl group; or a substituted or unsubstituted C2 to C60 heteroaryl group,
Ra and Rb are the same as or different from each other, and each independently a substituted or unsubstituted C6 to C60 aryl group; or a substituted or unsubstituted C2 to C60 heteroaryl group,
r and s are each an integer of 0 to 7, and
when r and s are each 2 or greater, substituents in each parenthesis are the same as or different from each other.

In addition, another embodiment of the present application provides a composition for an organic material layer of an organic light emitting device, the composition including the heterocyclic compound represented by Chemical Formula 1 and the heterocyclic compound represented by Chemical Formula 2.

Lastly, one embodiment of the present application provides a method for manufacturing an organic light emitting device, the method including preparing a substrate; forming a first electrode on the substrate; forming one or more organic material layers on the first electrode; and forming a second electrode on the organic material layer, wherein the forming of organic material layers includes forming one or more organic material layers using the composition for an organic material layer according to one embodiment of the present application.

### [Advantageous Effects]

A compound described in the present specification can be used as a material of an organic material layer of an organic light emitting device. In the organic light emitting device, the compound is capable of performing a role of a hole injection material, a hole transfer material, a light emitting material, an electron transfer material, an electron injection material or the like. Particularly, the compound can be used as a light emitting layer material of an organic light emitting device.

Specifically, the compound can be used alone as a light emitting material, or as a host material or a dopant material of a light emitting layer. Using the compound represented by Chemical Formula 1 in an organic material layer can lower a driving voltage, enhance light efficiency, and enhance lifetime properties of a device by thermal stability of the compound.

In addition, by the heterocyclic compound represented by Chemical Formula 1 having a substituent of -(L1)a-(Z1)b, the LUMO orbital is delocalized sufficiently long to the triazine group which is a core structure, and the substituent of -(L1)a-(Z1)b, and electrons are effectively stabilized, and as a result, an organic light emitting device including the same has properties of superior lifetime properties.

Particularly, the heterocyclic compound represented by Chemical Formula 1 and the heterocyclic compound represented by Chemical Formula 2 can be used as a material of a light emitting layer of an organic light emitting device at the same time. In this case, a driving voltage of the device can be lowered, light efficiency can be enhanced, and lifetime properties of the device can be particularly enhanced by thermal stability of the compound.

### [Description of Drawings]

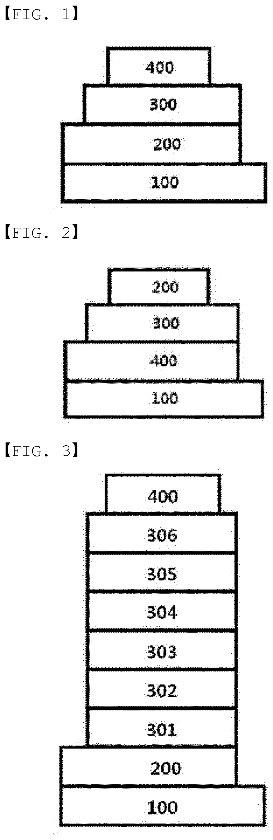

FIG. 1 to FIG. 3 are diagrams each schematically illustrating a lamination structure of an organic light emitting device according to one embodiment of the present application.

### [Reference Numeral]

100: Substrate
200: Anode
300: Organic Material Layer
301: Hole Injection Layer
302: Hole Transfer Layer
303: Light Emitting Layer
304: Hole Blocking Layer
305: Electron Transfer Layer
306: Electron Injection Layer
400: Cathode

### [Mode for Disclosure]

Hereinafter, the present application will be described in detail.

In the present specification, a term "substitution" means a hydrogen atom bonding to a carbon atom of a compound being changed to another substituent, and the position of substitution is not limited as long as it is a position at which the hydrogen atom is substituted, that is, a position at which a substituent can substitute, and when two or more substituents substitute, the two or more substituents may be the same as or different from each other.

In the present specification, "substituted or unsubstituted" means being substituted with one or more substituents selected from the group consisting of C1 to C60 linear or branched alkyl; C2 to C60 linear or branched alkenyl; C2 to C60 linear or branched alkynyl; C3 to C60 monocyclic or polycyclic cycloalkyl; C2 to C60 monocyclic or polycyclic heterocycloalkyl; C6 to C60 monocyclic or polycyclic aryl; C2 to C60 monocyclic or polycyclic heteroaryl; -SiRR'R"; - P(=O)RR'; C1 to C20 alkylamine; C6 to C60 monocyclic or polycyclic arylamine; and C2 to C60 monocyclic or polycyclic heteroarylamine, or being unsubstituted, or being substituted with a substituent linking two or more substituents selected from among the substituents illustrated above, or being unsubstituted, and
R, R' and R" are the same as or different from each other, and each independently a substituted or unsubstituted C1 to C60 alkyl group; a substituted or unsubstituted C6 to C60 aryl group; or a substituted or unsubstituted C2 to C60 heteroaryl group.

In the present specification, "substituted or unsubstituted" means being substituted with one or more substituents selected from the group consisting of deuterium; halogen; a cyano group; C1 to C60 linear or branched alkyl; C2 to C60 linear or branched alkenyl; C2 to C60 linear or branched alkynyl; C3 to C60 monocyclic or polycyclic cycloalkyl; C2 to C60 monocyclic or polycyclic heterocycloalkyl; C6 to C60 monocyclic or polycyclic aryl; C2 to C60 monocyclic or polycyclic heteroaryl; -SiRR'R"; -P(=O)RR'; C1 to C20 alkylamine; C6 to C60 monocyclic or polycyclic arylamine; and C2 to C60 monocyclic or polycyclic heteroarylamine, or being unsubstituted, or being substituted with a substituent linking two or more substituents selected from among the substituents illustrated above, or being unsubstituted, and
R, R' and R" are the same as or different from each other, and each independently a substituted or unsubstituted C1 to C60 alkyl group; a substituted or unsubstituted C6 to C60 aryl group; or a substituted or unsubstituted C2 to C60 heteroaryl group.

In the present specification, "the number of protons" means the number of substituents that a specific compound may have, and specifically, the number of protons may mean the number of hydrogens. For example, the number of protons in unsubstituted benzene may be expressed as 5, the number of protons in an unsubstituted naphthyl group may be expressed as 7, the number of protons in a naphthyl group substituted with a phenyl group may be expressed as 6, and the number of protons in an unsubstituted biphenyl group may be expressed as 9.

In the present specification, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present application, a "case of a substituent being not indicated in a chemical formula or compound structure" may mean that positions that may come as a substituent may all be hydrogen or deuterium. In other words, since deuterium is an isotope of hydrogen, some hydrogen atoms may be deuterium that is an isotope, and herein, a content of the deuterium may be from 0% to 100%.

In one embodiment of the present application, in a "case of a substituent being not indicated in a chemical formula or compound structure", hydrogen and deuterium may be mixed in compounds when deuterium is not explicitly excluded such as a deuterium content being 0%, a hydrogen content being 100% or substituents being all hydrogen.

In one embodiment of the present application, deuterium is one of isotopes of hydrogen, is an element having deuteron formed with one proton and one neutron as a nucleus, and may be expressed as hydrogen-2, and the elemental symbol may also be written as D or ²H.

In one embodiment of the present application, an isotope means an atom with the same atomic number (Z) but with a different mass number (A), and may also be interpreted as an element with the same number of protons but with a different number of neutrons.

In one embodiment of the present application, a meaning of a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 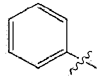 means that the total number of substituents that the phenyl group may have is 5 (T1 in the formula), and the number of deuterium among these is 1 (T2 in the formula). In other words, having a deuterium content of 20% in a phenyl group may be represented by the following structural formulae.

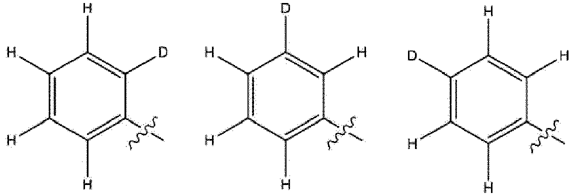

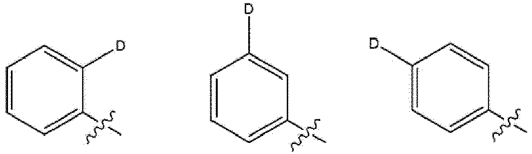

In addition, in one embodiment of the present application, "a phenyl group having a deuterium content of 0%" may mean a phenyl group that does not include a deuterium atom, that is, a phenyl group that has 5 hydrogen atoms.

In the present specification, the halogen may be fluorine, chlorine, bromine or iodine.

In the present specification, the alkyl group includes linear or branched having 1 to 60 carbon atoms, and may be further substituted with other substituents. The number of carbon atoms of the alkyl group may be from 1 to 60, specifically from 1 to 40 and more specifically from 1 to 20. Specific examples thereof may include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethylbutyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group and the like, but are not limited thereto.

In the present specification, the alkenyl group includes linear or branched having 2 to 60 carbon atoms, and may be further substituted with other substituents. The number of carbon atoms of the alkenyl group may be from 2 to 60, specifically from 2 to 40 and more specifically from 2 to 20. Specific examples thereof may include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group includes linear or branched having 2 to 60 carbon atoms, and may be further substituted with other substituents. The number of carbon atoms of the alkynyl group may be from 2 to 60, specifically from 2 to 40 and more specifically from 2 to 20.

In the present specification, the alkoxy group may be linear, branched or cyclic. The number of carbon atoms of the alkoxy group is not particularly limited, but is preferably from 1 to 20. Specific examples thereof may include methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy and the like, but are not limited thereto.

In the present specification, the cycloalkyl group includes a monocyclic or polycyclic aryl group having 3 to 60 carbon atoms, and may be further substituted with other substituents. Herein, the polycyclic means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon groups of the cycloalkyl group may be from 3 to 60, specifically from 3 to 40 and more specifically from 5 to 20. Specific examples thereof may include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group includes O, S, Se, N or Si as a heteroatom, includes monocyclic or polycyclic having 2 to 60 carbon atoms, and may be further substituted with other substituents. Herein, the polycyclic means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group includes monocyclic or polycyclic having 6 to 60 carbon atoms, and may be further substituted with other substituents. Herein, the polycyclic means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The aryl group includes a spiro group. The number of carbon atoms of the aryl group may be from 6 to 60, specifically from 6 to 40 and more specifically from 6 to 25. Specific examples of the aryl group may include a phenyl group, a biphenyl group, a terphenyl group, a quarterphenyl group, a quinquephenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused ring group thereof, and the like, but are not limited thereto.

In the present specification, the phosphine oxide group is represented by -P(=O)R₁₀₁R₁₀₂, R₁₀₁ and R₁₀₂ are the same as or different from each other and may be each independently a substituent formed with at least one of hydrogen; deuterium; a halogen group; an alkyl group; an alkenyl group; an alkoxy group; a cycloalkyl group; an aryl group; and a heterocyclic group. Specifically, the phosphine oxide group may be substituted with an aryl group, and as the aryl group, the examples described above may be used. Examples of the phosphine oxide group may include a diphenylphosphine oxide group, a dinaphthylphosphine oxide group and the like, but are not limited thereto.

In the present specification, the silyl group is a substituent including Si, having the Si atom directly linked as a radical, and is represented by -SiR₁₀₄R₁₀₅R₁₀₆. R₁₀₄ to R₁₀₆ are the same as or different from each other, and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present specification, a fluorenyl group may be substituted, and adjacent substituents may bond to each other to form a ring.

When the fluorenyl group is substituted, 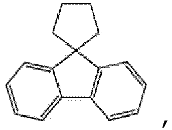 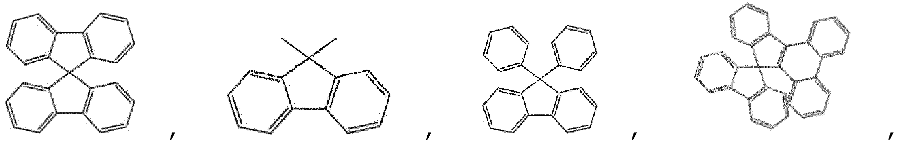 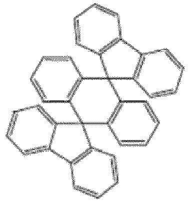 and the like may be included, however, the structure is not limited thereto.

In the present specification, the heteroaryl group includes S, O, Se, N or Si as a heteroatom, includes monocyclic or polycyclic having 2 to 60 carbon atoms, and may be further substituted with other substituents. Herein, the polycyclic means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. Specific examples of the heteroaryl group may include a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a qninozolinyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi(dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiathiazinyl group, a phthalazinyl group, a naphthylidinyl group, a phenanthrolinyl group, a benzo[c][1,2,5]thiadiazolyl group, a 5,10-dihydrobenzo[b,e][1,4]azasilinyl group, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group and the like, but are not limited thereto.

In the present specification, the amine group is represented by -NR₁₀₇R₁₀₈, and R₁₀₇ and R₁₀₈ are the same as or different from each other, and may be each independently a substituent formed with at least one of hydrogen; deuterium; a halogen group; an alkyl group; an alkenyl group; an alkoxy group; a cycloalkyl group; an aryl group; and a heterocyclic group. The amine group may be selected from the group consisting of -NH₂; a monoalkylamine group; a monoarylamine group; a monoheteroarylamine group; a dialkylamine group; a diarylamine group; a diheteroarylamine group; an alkylarylamine group; an alkylheteroarylamine group; and an arylheteroarylamine group, and although not particularly limited thereto, the number of carbon atoms is preferably from 1 to 30. Specific examples of the amine group may include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the arylene group means the aryl group having two bonding sites, that is, a divalent group. The descriptions on the aryl group provided above may be applied thereto except for those that are each a divalent group. In addition, the heteroarylene group means the heteroaryl group having two bonding sites, that is, a divalent group. The descriptions on the heteroaryl group provided above may be applied thereto except for those that are each a divalent group.

In the present specification, the "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

One embodiment of the present application provides a heterocyclic compound represented by Chemical Formula 1.

When Z1 is a dicyclic or tetracyclic C15 to C60 aryl group substituted or unsubstituted and having the number of protons of 9 or less in Chemical Formula 1, L1 may be a substituted or unsubstituted C6 to C60 arylene group; or a substituted or unsubstituted C2 to C60 heteroarylene group.

In other words, when Z1 is a dicyclic or tetracyclic C15 to C60 aryl group substituted or unsubstituted and having the number of protons of 9 or less in Chemical Formula 1, it is linked through a linker (L1), and the dicyclic or tetracyclic C15 to C60 aryl group substituted or unsubstituted and having the number of protons of 9 or less may be a biphenyl group or a pyrenyl group.

In the present application, when a pyrenyl group belonging to the dicyclic or tetracyclic C15 to C60 aryl group substituted or unsubstituted and having the number of protons of 9 or less directly bonds to the triazine group, light energy (approximately 2.4 eV) emitted from a green dopant is absorbed again due to a low T₁ level (energy level value in triplet state), and as a result, device efficiency and lifetime may decrease. Accordingly, device efficiency and lifetime may increase in the present application since the dicyclic or tetracyclic C15 to C60 aryl group substituted or unsubstituted and having the number of protons of 9 or less is linked to the triazine through a linker (L1).

In other words, when Z1 is a biphenyl group or a pyrenyl group in Chemical Formula 1, L1 may be a substituted or unsubstituted C6 to C60 arylene group; or a substituted or unsubstituted C2 to C60 heteroarylene group.

In one embodiment of the present application, Chemical Formula 1 may be represented by any one of the following Chemical Formula 3 to Chemical Formula 8.

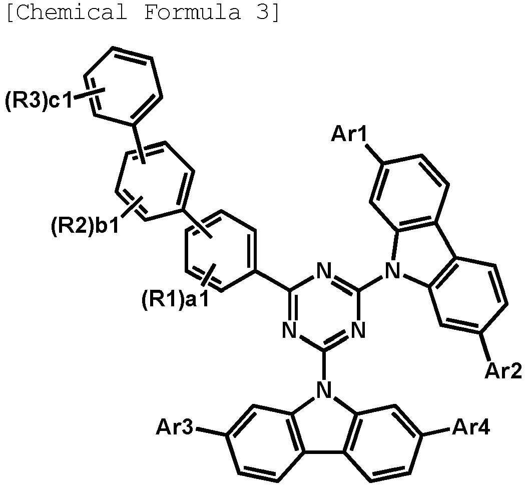

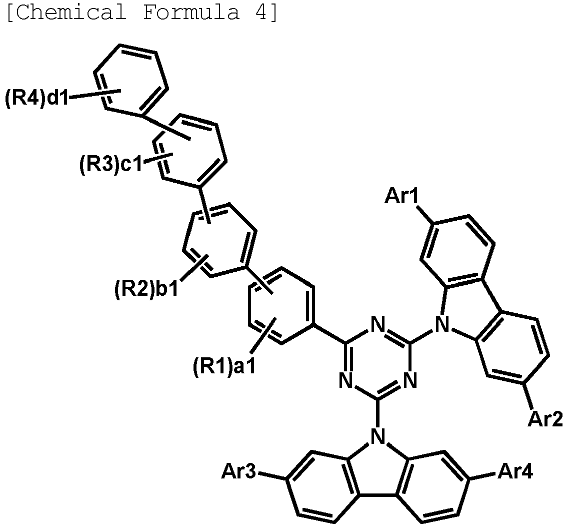

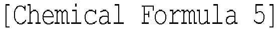

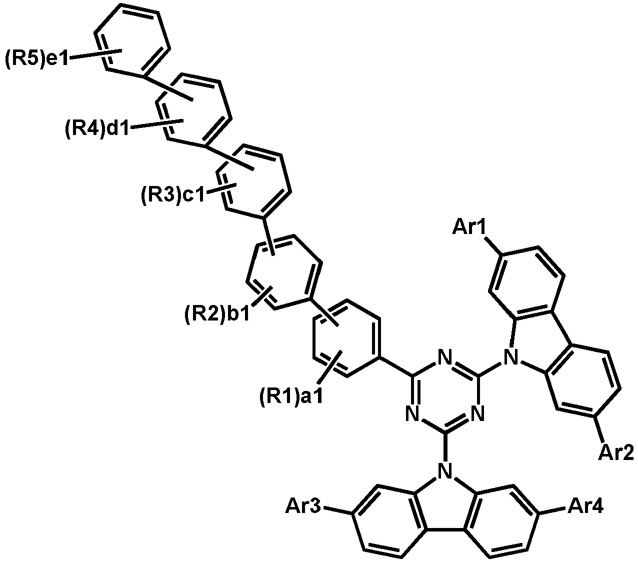

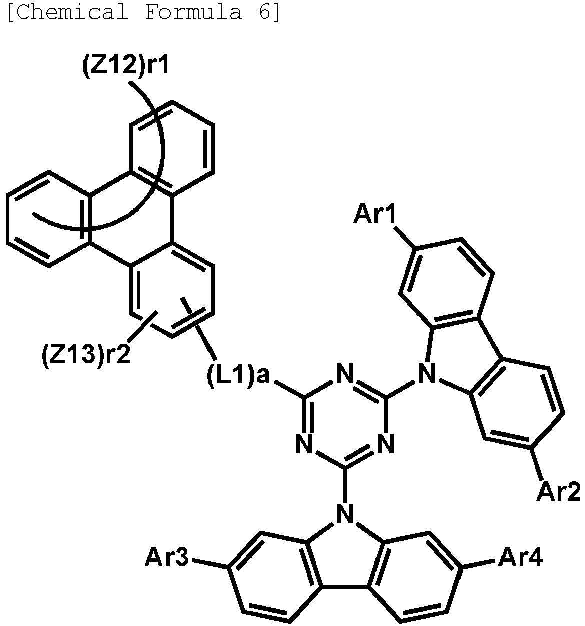

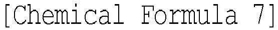

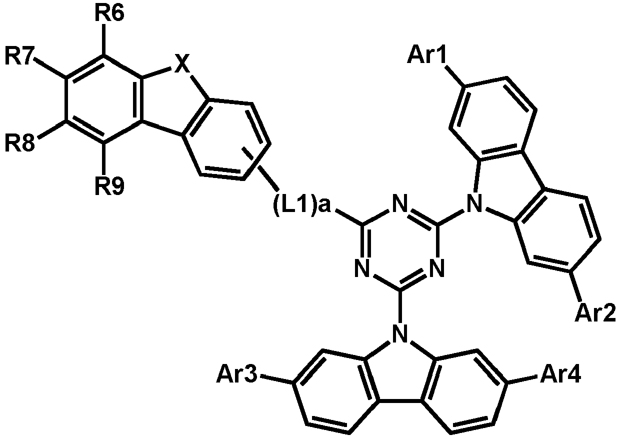

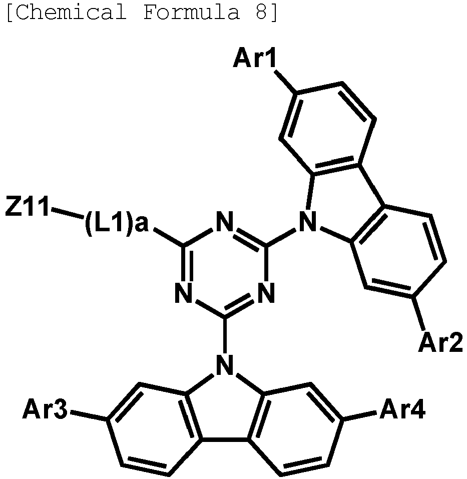

In Chemical Formulae 3 to 8,
Ar1 to Ar4, L1 and a have the same definitions as in Chemical Formula 1,
X is O; S; or C(R21)(R22),
R1 to R5 are the same as or different from each other, and each independently hydrogen; a substituted or unsubstituted C1 to C60 alkyl group; a substituted or unsubstituted C6 to C60 aryl group; or a substituted or unsubstituted C2 to C60 heteroaryl group,
R6 to R9 are the same as or different from each other, and each independently hydrogen; deuterium; a halogen group; - CN; a substituted or unsubstituted C1 to C60 alkyl group; a substituted or unsubstituted C6 to C60 aryl group; a C2 to C60 heteroaryl group substituted or unsubstituted and including O or S as a heteroatom,
Z11 is -P(=O)RR'; -SiRR'R"; or a substituted or unsubstituted amine group,
Z12 and Z13 are the same as or different from each other, and each independently a substituted or unsubstituted C1 to C60 alkyl group; a substituted or unsubstituted C6 to C60 aryl group; a C2 to C60 heteroaryl group substituted or unsubstituted and including O or S as a heteroatom,
R, R' and R" have the same definitions as in Chemical Formula 1,
R21 and R22 are the same as or different from each other, and each independently a substituted or unsubstituted C1 to C60 alkyl group; a substituted or unsubstituted C6 to C60 aryl group; or a substituted or unsubstituted C2 to C60 heteroaryl group, or two or more groups adjacent to each other bond to each other to form a substituted or unsubstituted C6 to C60 aromatic hydrocarbon ring or a substituted or unsubstituted C2 to C60 heteroring,
a1 to d1 are each an integer of 1 to 4,
e1 is an integer of 1 to 5,
when a1 to e1 are each 2 or greater, substituents in each parenthesis are the same as or different from each other,
r1 and r2 are each an integer of 0 or 1, and
r1+r2<2.

Particularly, as in Chemical Formulae 3 to 8, the heterocyclic compound according to the present application has, a sufficiently long substituent or a substituent with a large molecular weight, which may effectively stabilize electrons since the LUMO is delocalized from the triazine core to the substituent, and as a result, an organic light emitting device including the same has superior lifetime properties.

In addition, a dipole moment is different depending on the type of a terminal substituent of triazine which is the core structure, and molecular mobility may be adjusted. This allows to select a proper triazine terminal substituent depending on the charge balance of a device and to adjust a sublimation temperature by affecting molecular rigidity depending on the terminal substituent, and as a result, an organic light emitting device including the same has superior lifetime properties. In other words, a sublimation temperature that is too high causes molecular deterioration before the molecules are sublimated, which inhibits efficiency and lifetime of a device.

In one embodiment of the present application, Chemical Formula 1 may be represented by any one of the following Chemical Formula 9 to Chemical Formula 14.

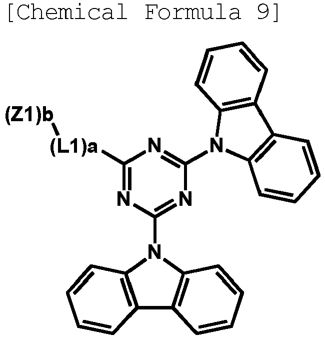

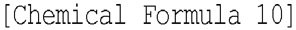

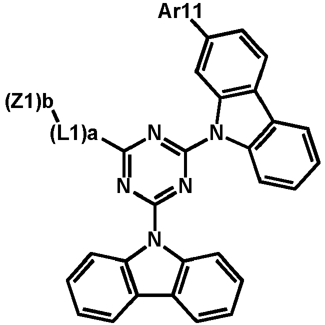

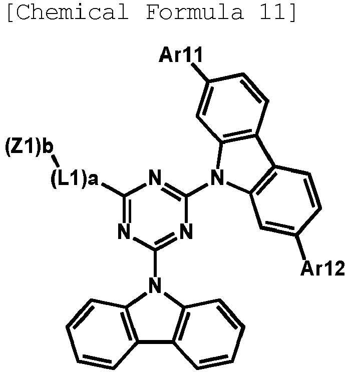

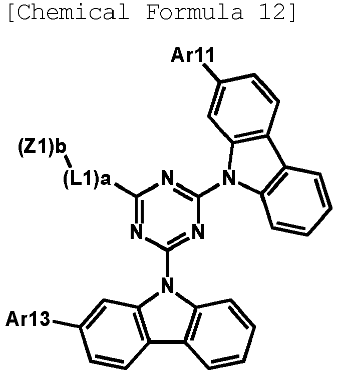

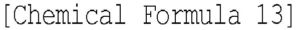

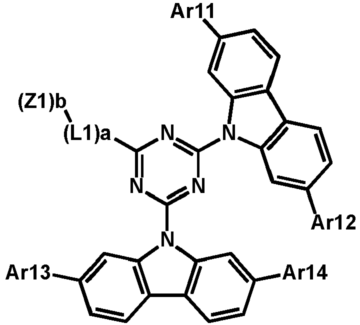

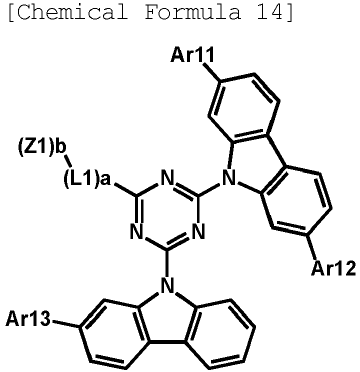

In Chemical Formulae 9 to 14,
L1, Z1, a and b have the same definitions as in Chemical Formula 1, and
Ar11 to Ar14 are the same as or different from each other, and each independently deuterium; a substituted or unsubstituted C6 to C60 aryl group; or a substituted or unsubstituted C2 to C60 heteroaryl group.

In one embodiment of the present application, Ar1 to Ar4 are the same as or different from each other, and may be each independently hydrogen; deuterium; a substituted or unsubstituted C1 to C60 alkyl group; a substituted or unsubstituted C6 to C60 aryl group; or a substituted or unsubstituted C2 to C60 heteroaryl group.

In another embodiment, Ar1 to Ar4 are the same as or different from each other, and may be each independently hydrogen; deuterium; a substituted or unsubstituted C1 to C40 alkyl group; a substituted or unsubstituted C6 to C40 aryl group; or a substituted or unsubstituted C2 to C40 heteroaryl group.

In another embodiment, Ar1 to Ar4 are the same as or different from each other, and may be each independently hydrogen; deuterium; or a substituted or unsubstituted C6 to C40 aryl group.

In another embodiment, Ar1 to Ar4 are the same as or different from each other, and may be each independently hydrogen; deuterium; or a substituted or unsubstituted C6 to C20 aryl group.

In another embodiment, Ar1 to Ar4 are the same as or different from each other, and may be each independently hydrogen; deuterium; or a substituted or unsubstituted C6 to C20 monocyclic or polycyclic aryl group.

In another embodiment, Ar1 to Ar4 are the same as or different from each other, and may be each independently hydrogen; deuterium; or a substituted or unsubstituted C6 to C20 monocyclic aryl group.

In another embodiment, Ar1 to Ar4 are the same as or different from each other, and may be each independently hydrogen; deuterium; or a phenyl group.

In one embodiment of the present application, Ar11 to Ar14 are the same as or different from each other, and may be each independently deuterium; a substituted or unsubstituted C6 to C60 aryl group; or a substituted or unsubstituted C2 to C60 heteroaryl group.

In another embodiment, Ar11 to Ar14 are the same as or different from each other, and may be each independently deuterium; or a substituted or unsubstituted C6 to C60 aryl group.

In another embodiment, Ar11 to Ar14 are the same as or different from each other, and may be each independently deuterium; or a substituted or unsubstituted C6 to C20 monocyclic aryl group.

In another embodiment, Ar11 to Ar14 may be deuterium; or a phenyl group.

In one embodiment of the present application, L1 may be a direct bond; a substituted or unsubstituted C6 to C60 arylene group; or a substituted or unsubstituted C2 to C60 heteroarylene group.

In another embodiment, L1 may be a direct bond; a substituted or unsubstituted C6 to C40 arylene group; or a substituted or unsubstituted C2 to C40 heteroarylene group.

In another embodiment, L1 may be a direct bond; a C6 to C40 arylene group; or a C2 to C40 heteroarylene group.

In another embodiment, L1 may be a direct bond; or a C6 to C40 arylene group.

In another embodiment, L1 may be a direct bond; or a C6 to C40 monocyclic or polycyclic arylene group.

In another embodiment, L1 may be a direct bond; a phenylene group; or a biphenylene group.

In one embodiment of the present application, Z1 may be selected from the group consisting of halogen; -CN; a substituted or unsubstituted C1 to C60 alkyl group; a substituted or unsubstituted C10 to C60 aryl group; a C2 to C60 heteroaryl group substituted or unsubstituted and including O or S as a heteroatom; -P(=O)RR'; -SiRR'R"; and a substituted or unsubstituted amine group.

In another embodiment, Z1 may be selected from the group consisting of a substituted or unsubstituted C10 to C60 aryl group; a C2 to C60 heteroaryl group substituted or unsubstituted and including O or S as a heteroatom; and - SiRR'R".

In another embodiment, Z1 may be selected from the group consisting of a substituted or unsubstituted C10 to C40 aryl group; a C2 to C40 heteroaryl group substituted or unsubstituted and including O or S as a heteroatom; and - SiRR'R".

In another embodiment, Z1 may be selected from the group consisting of a C10 to C40 aryl group unsubstituted or substituted with a C1 to C20 alkyl group or a C6 to C20 aryl group; a C2 to C40 heteroaryl group including O or S as a heteroatom; and -SiRR'R".

In another embodiment, Z1 may be a biphenyl group; a terphenyl group unsubstituted or substituted with a C6 to C20 aryl group; a quarterphenyl group unsubstituted or substituted with a C6 to C20 aryl group; a quinquephenyl group; a dimethylfluorenyl group; a diphenylfluorenyl group; a spirobifluorenyl group; a triphenylenyl group; a dibenzofuran group; a dibenzothiophene group; or -SiRR'R".

In another embodiment, Z1 may be a biphenyl group; a terphenyl group; a dimethylfluorenyl group; a diphenylfluorenyl group; a spirobifluorenyl group; a triphenylenyl group; a dibenzofuran group; a dibenzothiophene group; or -SiRR'R".

In one embodiment of the present application, X may be O.

In one embodiment of the present application, X may be S.

In one embodiment of the present application, X may be C(R21)(R22).

In one embodiment of the present application, R1 to R5 are the same as or different from each other, and may be each independently hydrogen; a substituted or unsubstituted C1 to C60 alkyl group; a substituted or unsubstituted C6 to C60 aryl group; or a substituted or unsubstituted C2 to C60 heteroaryl group.

In another embodiment, R1 to R5 are the same as or different from each other, and may be each independently hydrogen; or a substituted or unsubstituted C6 to C60 aryl group.

In another embodiment, R1 to R5 are the same as or different from each other, and may be each independently hydrogen; or a substituted or unsubstituted C6 to C40 aryl group.

In another embodiment, R1 to R5 are the same as or different from each other, and may be each independently hydrogen; or a substituted or unsubstituted C6 to C20 aryl group.

In another embodiment, R1 to R5 are the same as or different from each other, and may be each independently hydrogen; or a C6 to C20 aryl group.

In another embodiment, R1 to R5 are the same as or different from each other, and may be each independently hydrogen; a phenyl group; or a biphenyl group.

In another embodiment, R3 of Chemical Formula 3 may be hydrogen.

In another embodiment, R4 of Chemical Formula 4 may be hydrogen.

In one embodiment of the present application, R6 to R9 are the same as or different from each other, and may be each independently hydrogen; deuterium; a halogen group; -CN; a substituted or unsubstituted C1 to C60 alkyl group; a substituted or unsubstituted C6 to C60 aryl group; or a C2 to C60 heteroaryl group substituted or unsubstituted and including O or S as a heteroatom.

In another embodiment, R6 to R9 are the same as or different from each other, and may be each independently hydrogen; a substituted or unsubstituted C1 to C40 alkyl group; a substituted or unsubstituted C6 to C40 aryl group; a C2 to C40 heteroaryl group substituted or unsubstituted and including O or S as a heteroatom.

In another embodiment, R6 to R9 may be hydrogen.

In one embodiment of the present application, Z11 may be -P(=O)RR'; -SiRR'R"; or a substituted or unsubstituted amine group.

In another embodiment, Z11 may be -SiRR'R".

In one embodiment of the present application, R, R' and R" are the same as or different from each other, and may be each independently a substituted or unsubstituted C1 to C60 alkyl group; a substituted or unsubstituted C6 to C60 aryl group; or a substituted or unsubstituted C2 to C60 heteroaryl group.

In another embodiment, R, R' and R" are the same as or different from each other, and may be each independently a substituted or unsubstituted C1 to C60 alkyl group; or a substituted or unsubstituted C6 to C60 aryl group.

In another embodiment, R, R' and R" are the same as or different from each other, and may be each independently a C1 to C60 alkyl group; or a C6 to C60 aryl group.

In another embodiment, R, R' and R" are the same as or different from each other, and may be each independently a methyl group; or a phenyl group.

In another embodiment, R, R' and R" may be a phenyl group.

In one embodiment of the present application, R21 and R22 are the same as or different from each other, and each independently a substituted or unsubstituted C1 to C60 alkyl group; a substituted or unsubstituted C6 to C60 aryl group; or a substituted or unsubstituted C2 to C60 heteroaryl group, or two or more groups adjacent to each other may bond to each other to form a substituted or unsubstituted C6 to C60 aromatic hydrocarbon ring or a substituted or unsubstituted C2 to C60 heteroring.

In another embodiment, R21 and R22 are the same as or different from each other, and each independently a substituted or unsubstituted C1 to C40 alkyl group; or a substituted or unsubstituted C6 to C40 aryl group, or two or more groups adjacent to each other may bond to each other to form a substituted or unsubstituted C6 to C40 aromatic hydrocarbon ring.

In another embodiment, R21 and R22 are the same as or different from each other, and each independently a substituted or unsubstituted C1 to C10 alkyl group; or a substituted or unsubstituted C6 to C20 aryl group, or two or more groups adjacent to each other may bond to each other to form a substituted or unsubstituted C6 to C30 aromatic hydrocarbon ring.

In another embodiment, R21 and R22 are the same as or different from each other, and each independently a C1 to C10 alkyl group; or a C6 to C20 aryl group, or two or more groups adjacent to each other may bond to each other to form a C6 to C30 aromatic hydrocarbon ring.

In another embodiment, R21 and R22 are the same as or different from each other, and each independently a methyl group; or a phenyl group, or two or more groups adjacent to each other may bond to each other to form a fluorene ring.

In one embodiment of the present application, R21 and R22 are the same as or different from each other, and each independently a substituted or unsubstituted C1 to C60 alkyl group; a substituted or unsubstituted C6 to C60 aryl group; or a substituted or unsubstituted C2 to C60 heteroaryl group, or R21 and R22 may bond to each other to form a substituted or unsubstituted C6 to C60 aromatic hydrocarbon ring or a substituted or unsubstituted C2 to C60 heteroring.

In another embodiment, R21 and R22 are the same as or different from each other, and each independently a substituted or unsubstituted C1 to C10 alkyl group; or a substituted or unsubstituted C6 to C20 aryl group, or R21 and R22 may bond to each other to form a substituted or unsubstituted C6 to C30 aromatic hydrocarbon ring.

In another embodiment, R21 and R22 are the same as or different from each other, and each independently a methyl group; or a phenyl group, or R21 and R22 may bond to each other to form a fluorene ring.

In the heterocyclic compound provided in one embodiment of the present application, Chemical Formula 1 is represented by any one of the following compounds.

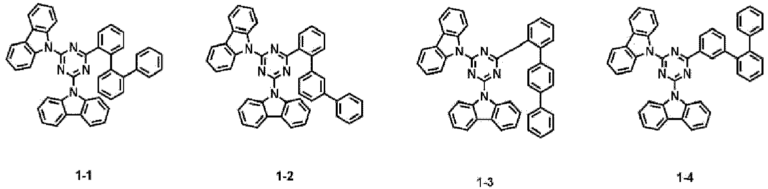

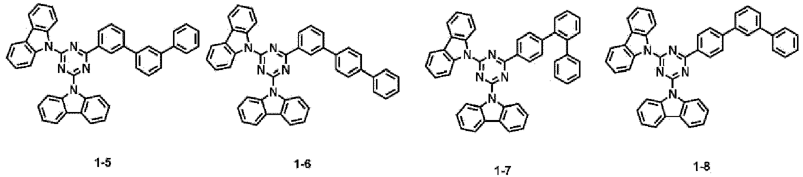

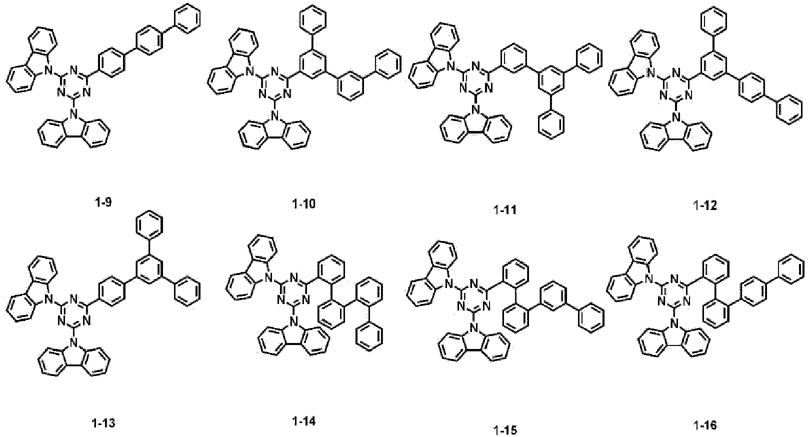

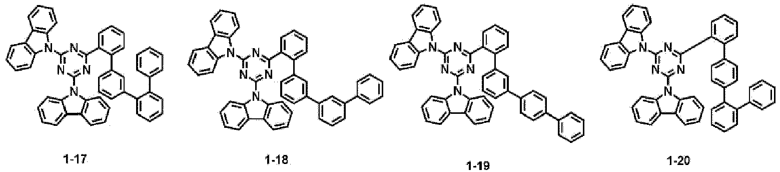

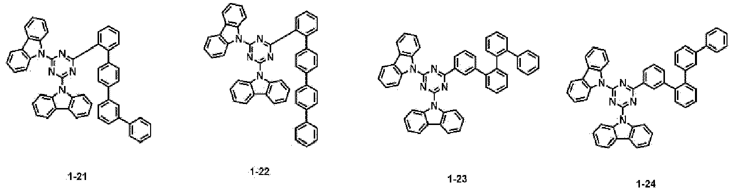

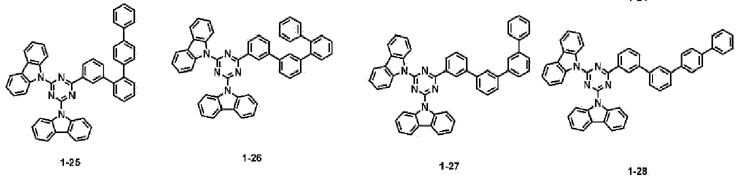

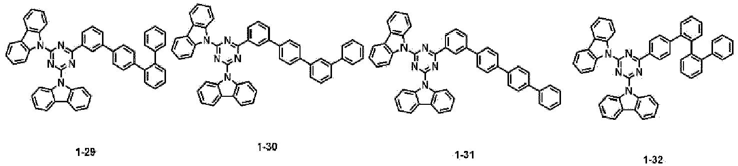

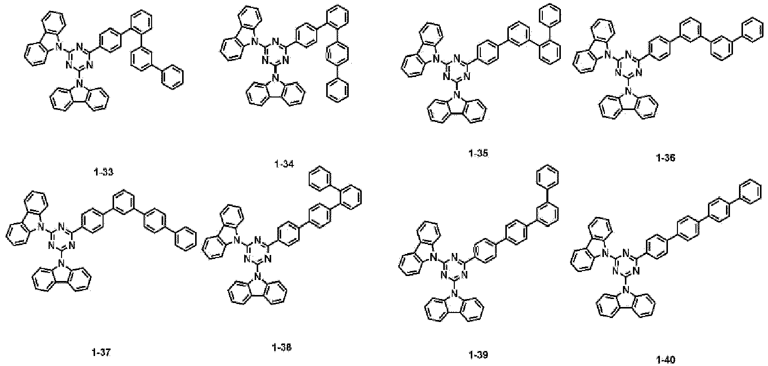

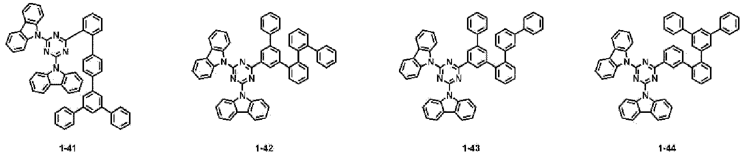

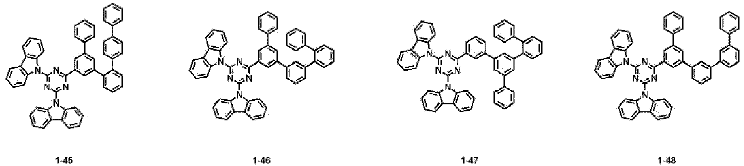

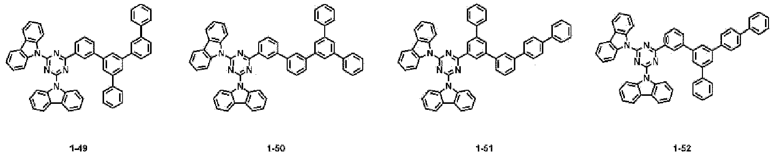

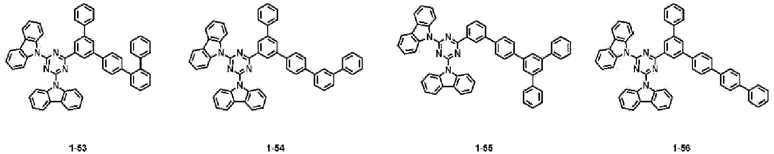

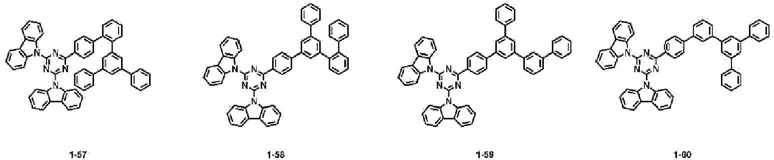

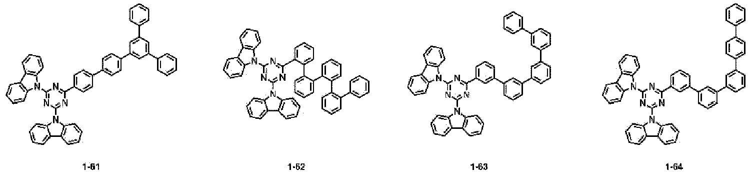

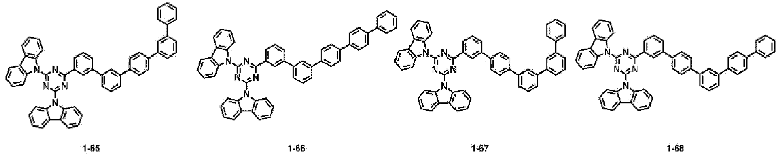

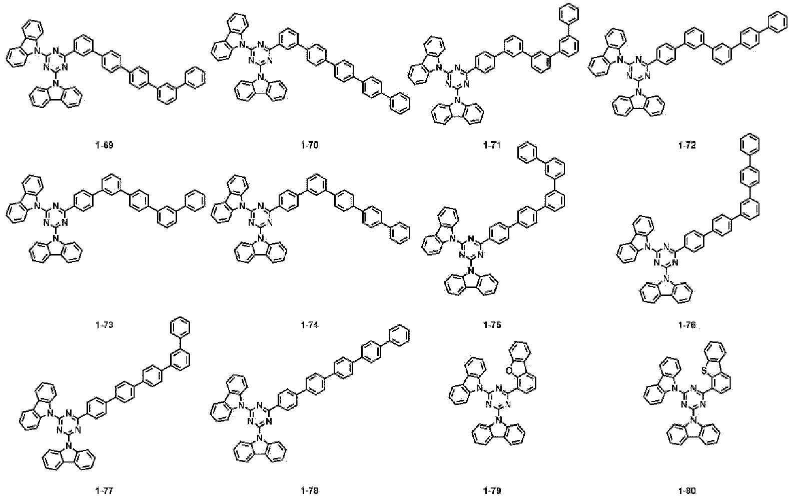

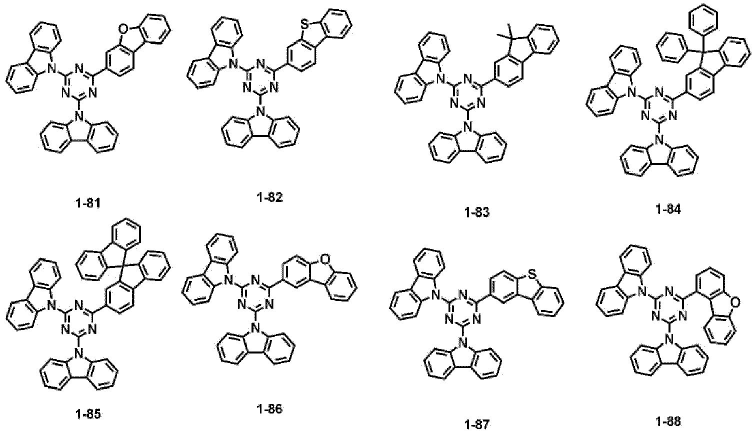

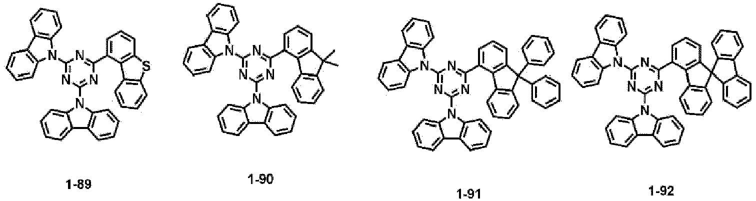

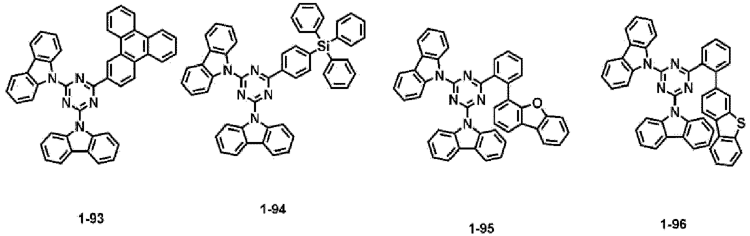

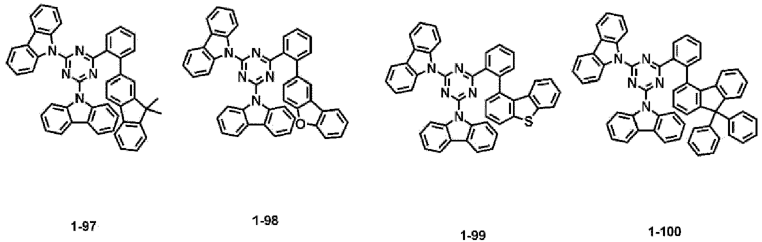

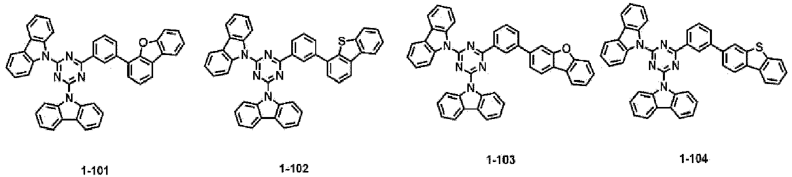

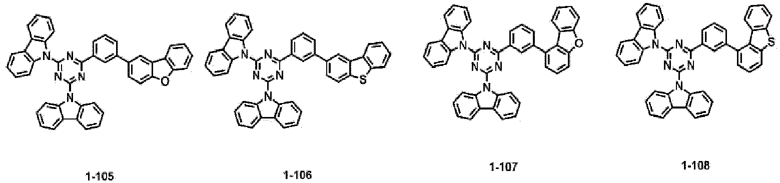

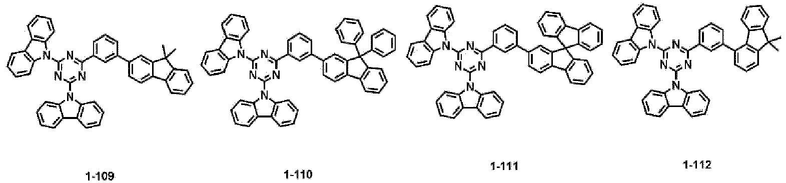

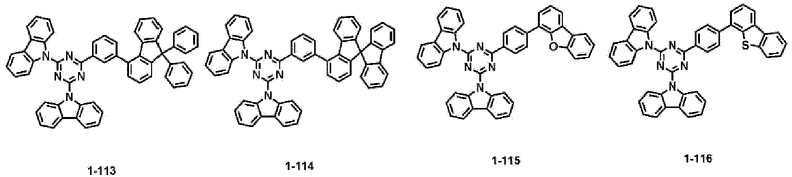

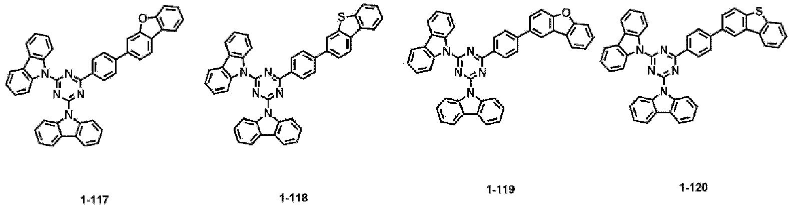

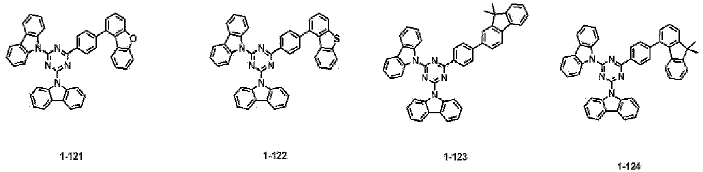

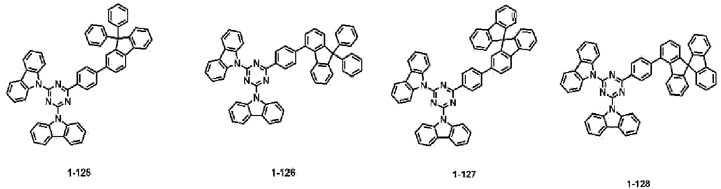

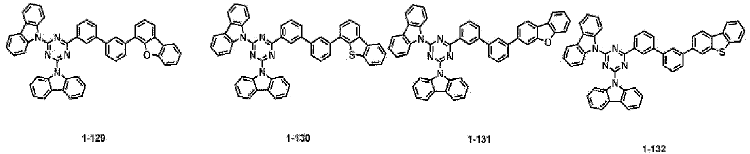

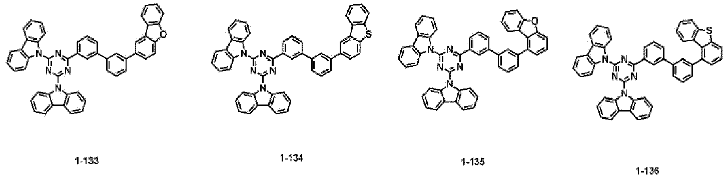

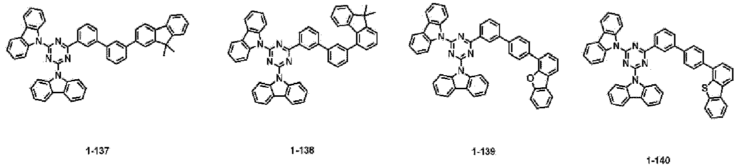

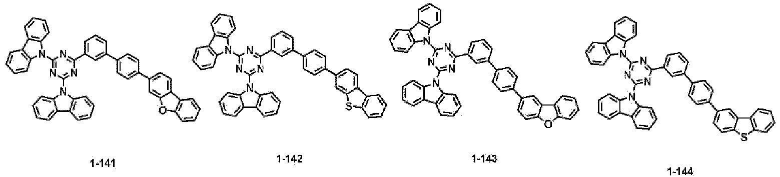

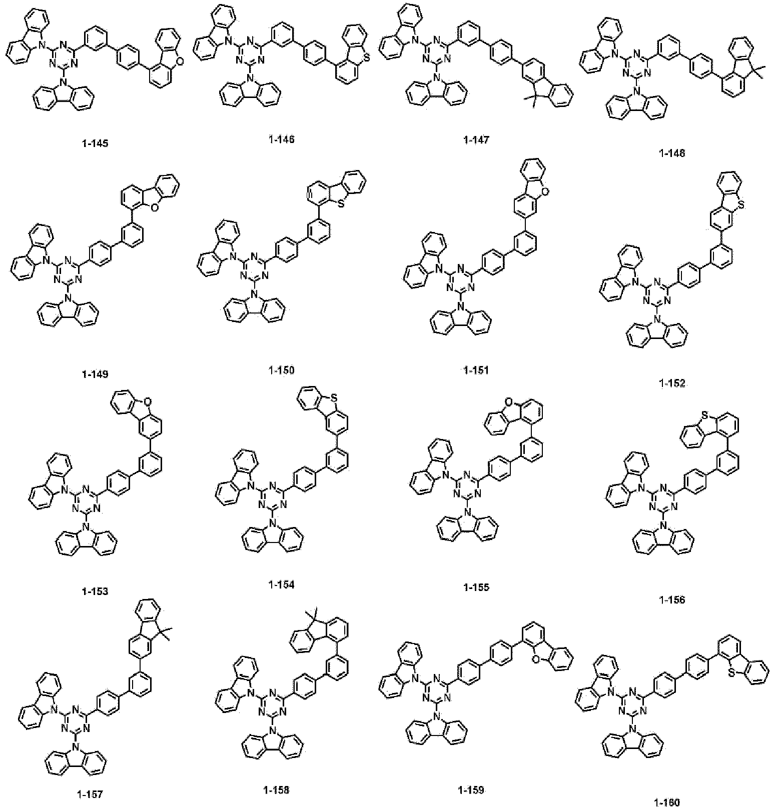

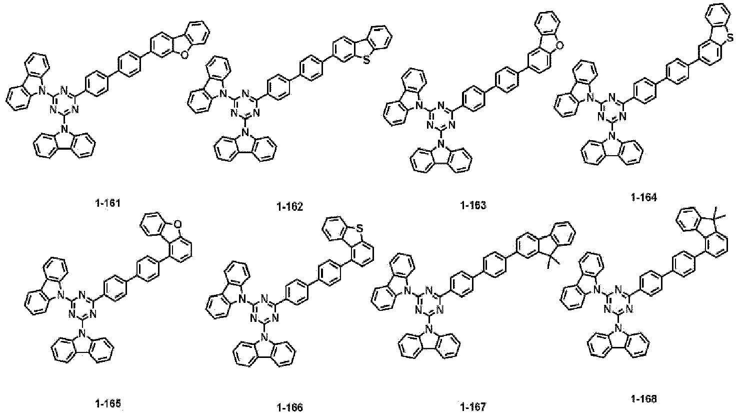

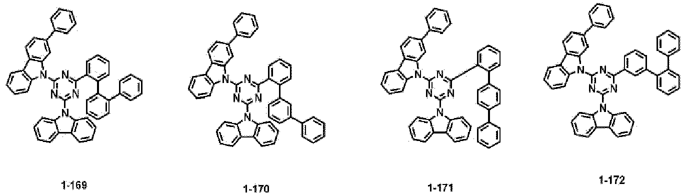

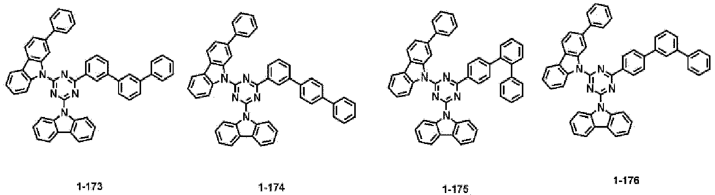

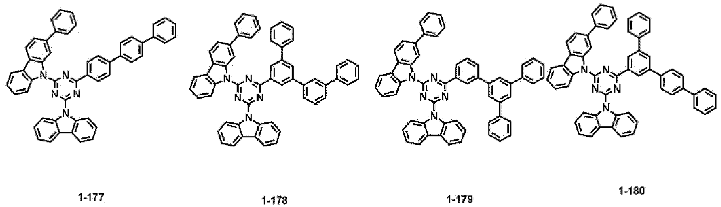

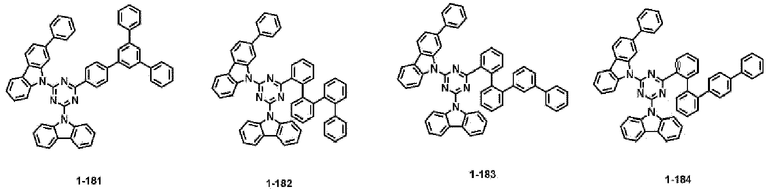

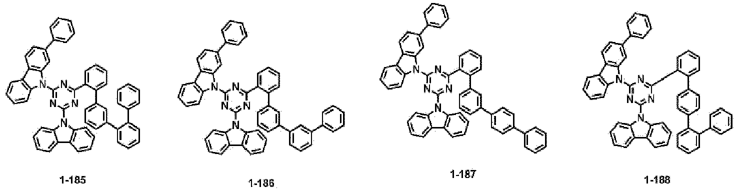

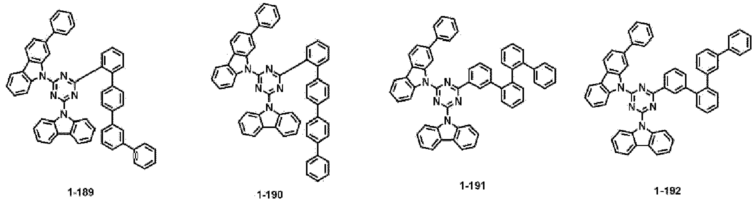

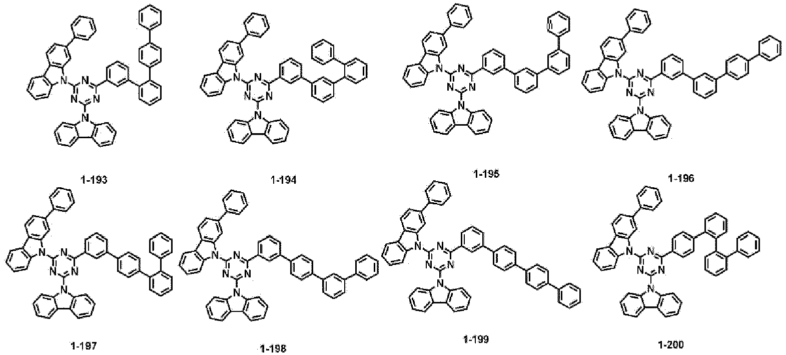

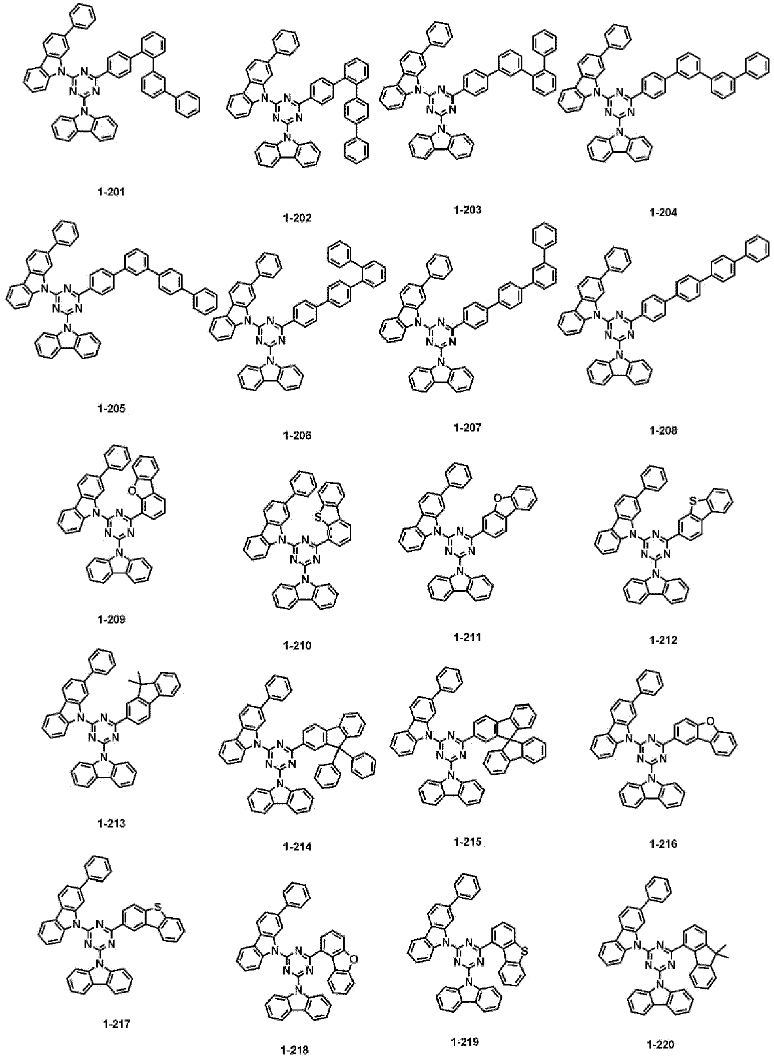

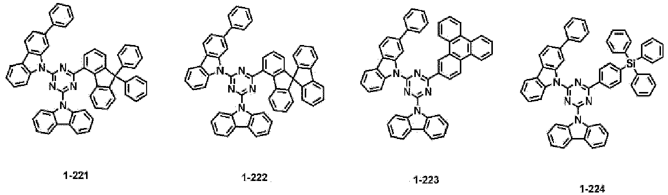

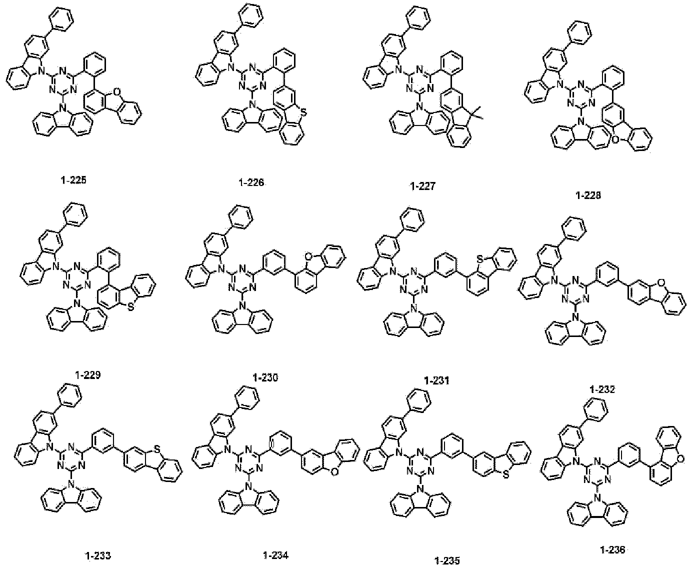

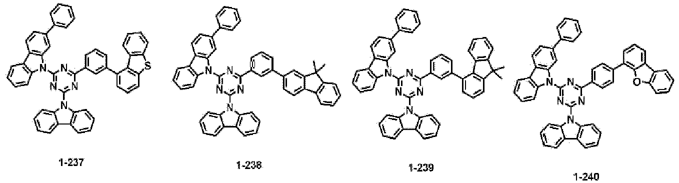

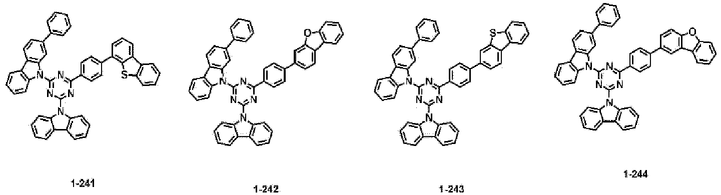

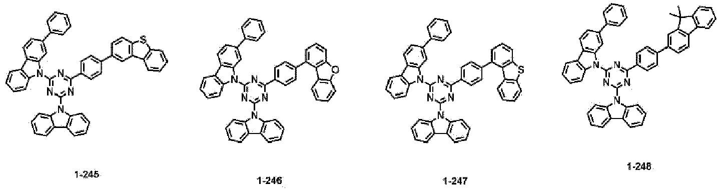

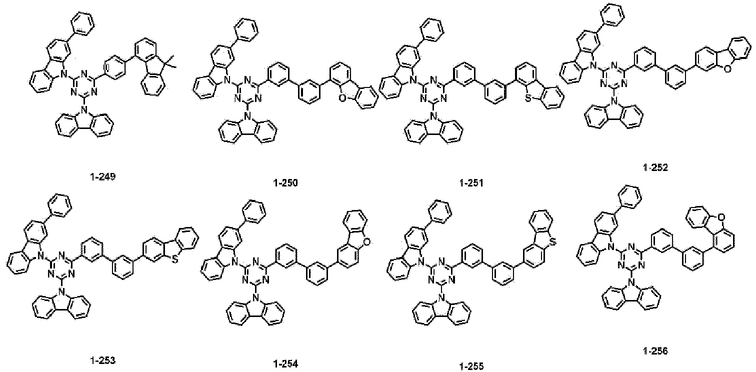

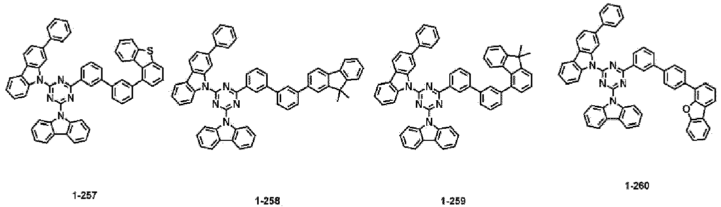

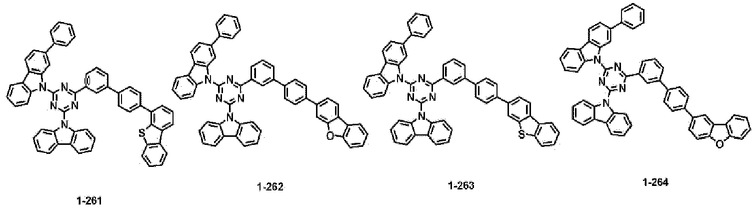

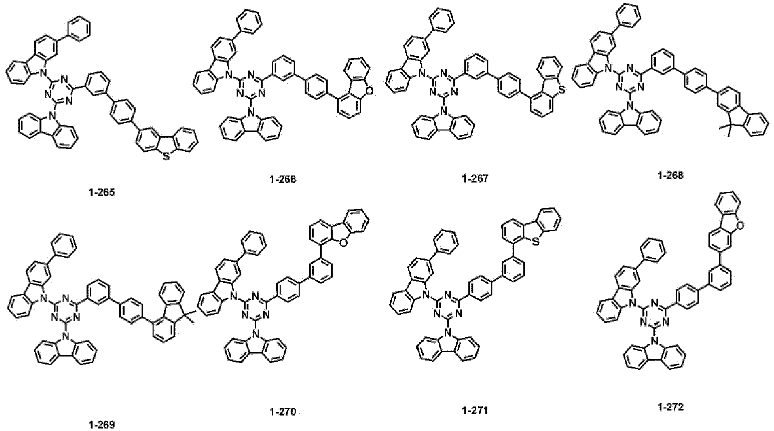

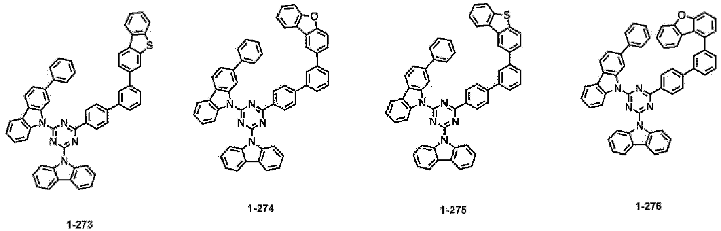

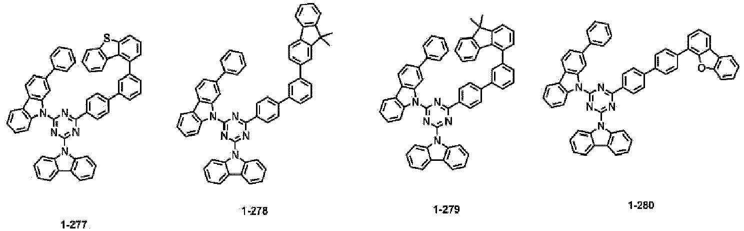

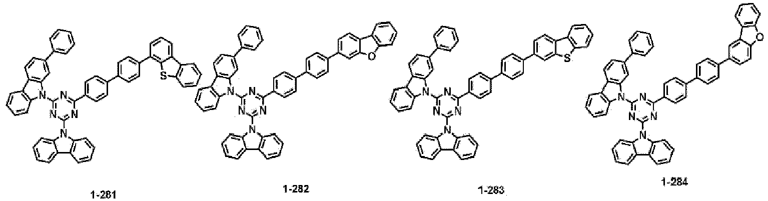

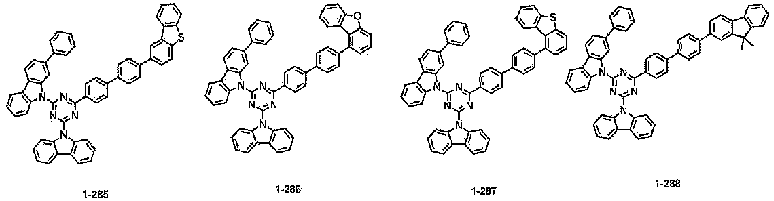

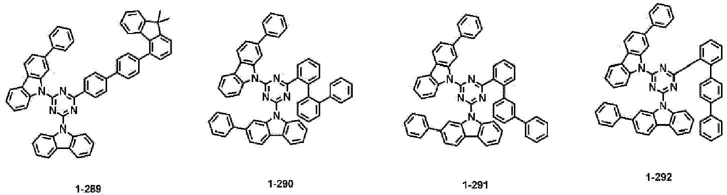

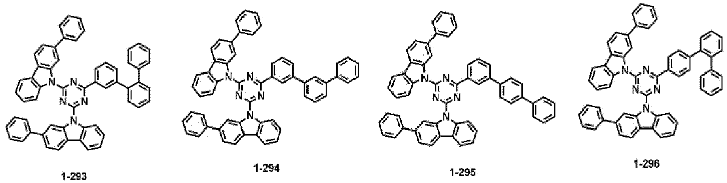

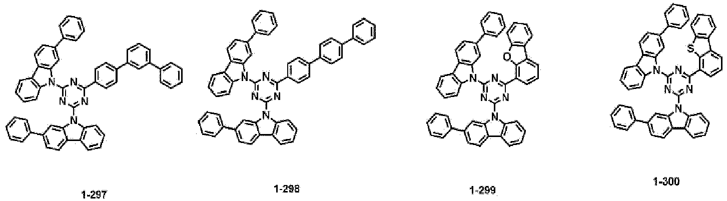

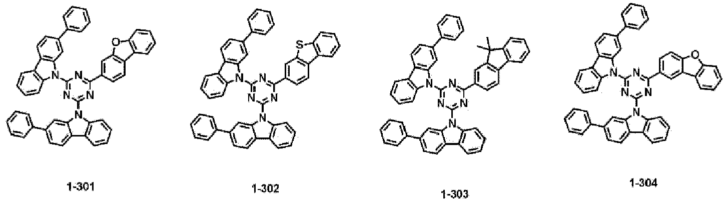

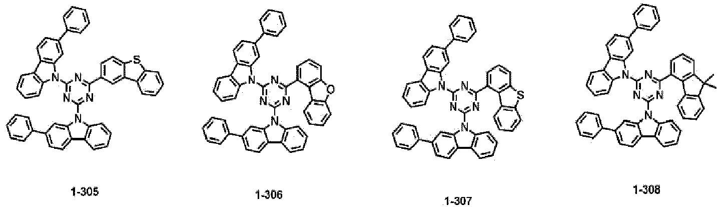

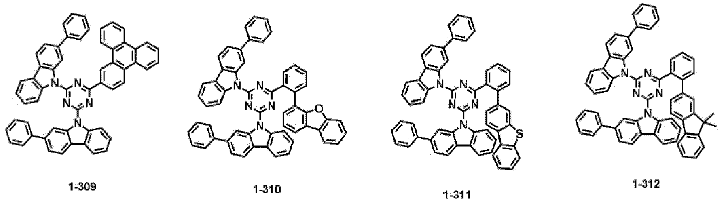

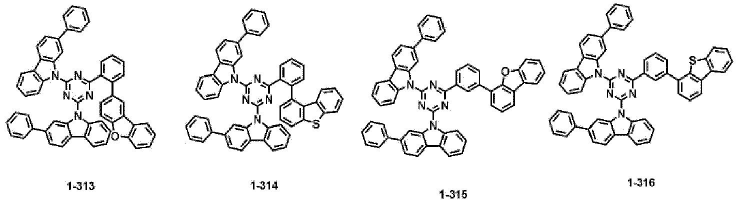

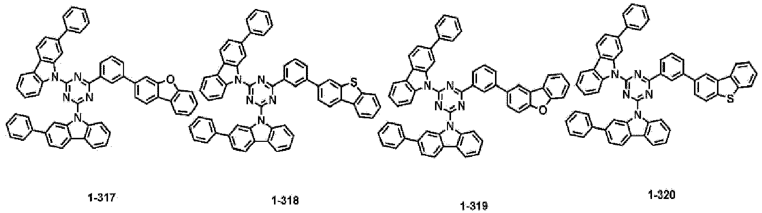

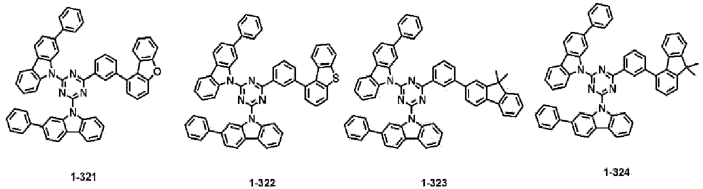

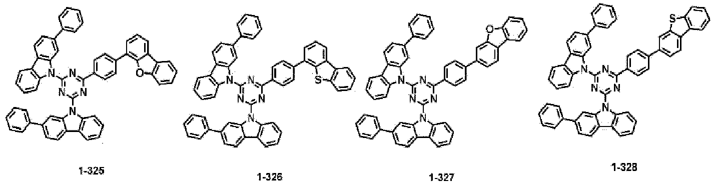

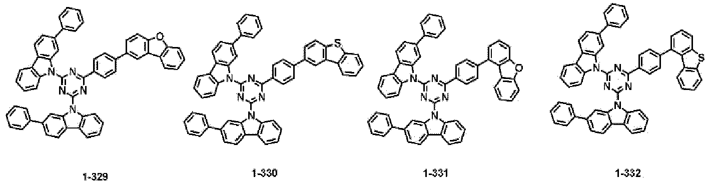

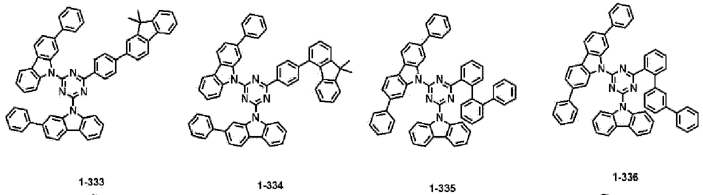

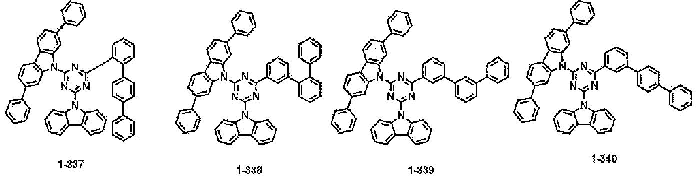

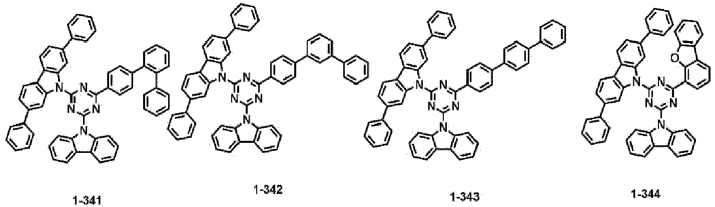

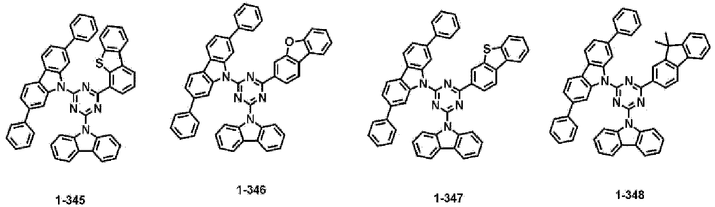

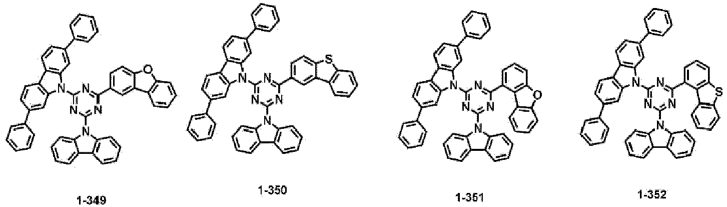

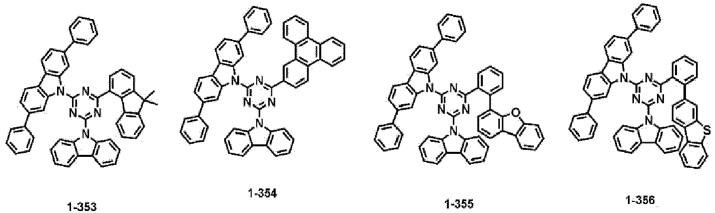

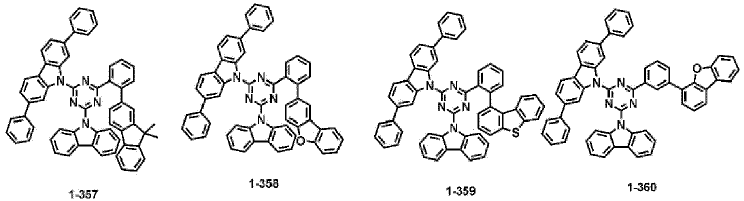

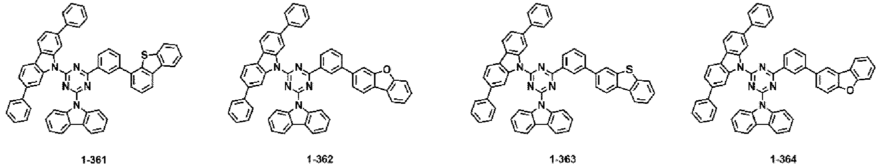

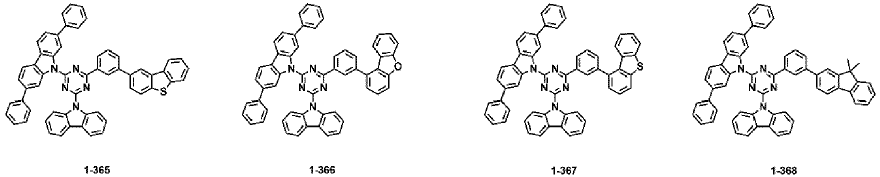

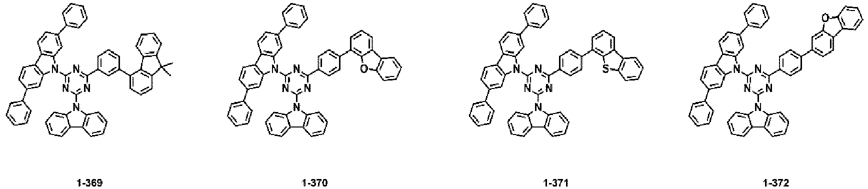

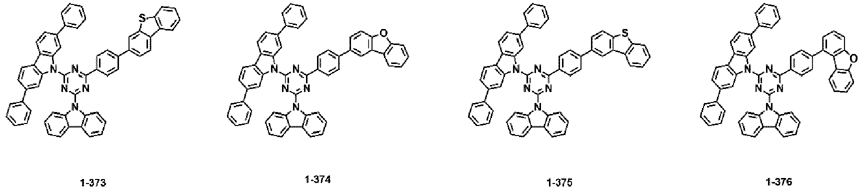

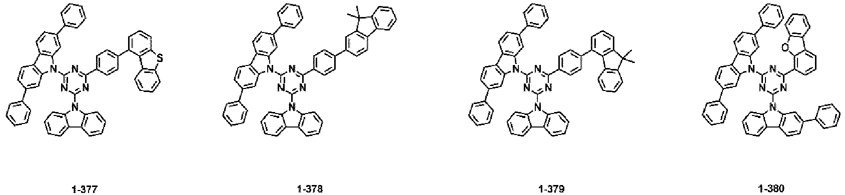

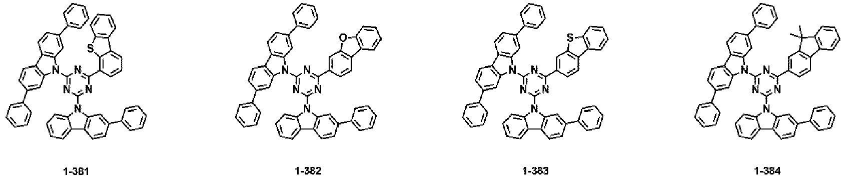

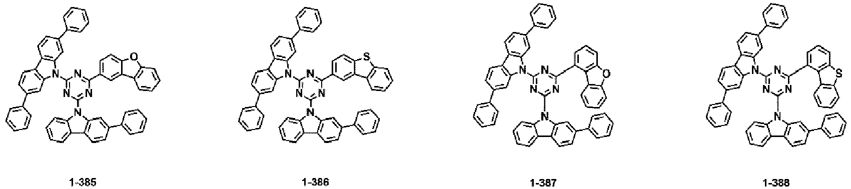

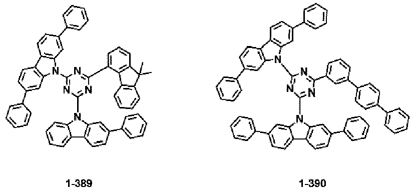

In addition, by introducing various substituents to the structure of Chemical Formula 1, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of Chemical Formula 1, the energy band gap may be finely controlled, and meanwhile, properties at interfaces between organic materials are enhanced, and material applications may become diverse.

Meanwhile, the compound has a high glass transition temperature (Tg), and has excellent thermal stability. Such an increase in the thermal stability becomes an important factor providing driving stability to a device.

In addition, one embodiment of the present application provides an organic light emitting device including a first electrode; a second electrode provided opposite to the first electrode; and one or more organic material layers provided between the first electrode and the second electrode, wherein one or more layers of the organic material layers include the heterocyclic compound represented by Chemical Formula 1.

In one embodiment of the present application, the first electrode may be an anode, and the second electrode may be a cathode.

In another embodiment, the first electrode may be a cathode, and the second electrode may be an anode.

In one embodiment of the present application, the organic light emitting device may be a blue organic light emitting device, and the heterocyclic compound represented by Chemical Formula 1 may be used as a material of the blue organic light emitting device.

In one embodiment of the present application, the organic light emitting device may be a green organic light emitting device, and the heterocyclic compound represented by Chemical Formula 1 may be used as a material of the green organic light emitting device.

In one embodiment of the present application, the organic light emitting device may be a red organic light emitting device, and the heterocyclic compound represented by Chemical Formula 1 may be used as a material of the red organic light emitting device.

In one embodiment of the present application, the organic light emitting device may be a blue organic light emitting device, and the heterocyclic compound represented by Chemical Formula 1 may be used as a light emitting layer material of the blue organic light emitting device.

In one embodiment of the present application, the organic light emitting device may be a green organic light emitting device, and the heterocyclic compound represented by Chemical Formula 1 may be used as a light emitting layer material of the green organic light emitting device.

In one embodiment of the present application, the organic light emitting device may be a red organic light emitting device, and the heterocyclic compound represented by Chemical Formula 1 may be used as a light emitting layer material of the red organic light emitting device.

Specific details on the heterocyclic compound represented by Chemical Formula 1 are the same as the descriptions provided above.

The organic light emitting device of the present disclosure may be manufactured using common organic light emitting device manufacturing methods and materials except that one or more organic material layers are formed using the heterocyclic compound described above.

The heterocyclic compound may be formed into an organic material layer through a solution coating method as well as a vacuum deposition method when manufacturing the organic light emitting device. Herein, the solution coating method means spin coating, dip coating, inkjet printing, screen printing, a spray method, roll coating and the like, but is not limited thereto.

The organic material layer of the organic light emitting device of the present disclosure may be formed in a single layer structure, or may also be formed in a multilayer structure in which two or more organic material layers are laminated. For example, the organic light emitting device according to one embodiment of the present disclosure may have a structure including a hole injection layer, a hole transfer layer, a light emitting layer, an electron transfer layer, an electron injection layer and the like as the organic material layer. However, the structure of the organic light emitting device is not limited thereto, and may include a smaller number of organic material layers.

In the organic light emitting device of the present disclosure, the organic material layer includes a light emitting layer, and the light emitting layer may include the heterocyclic compound.

In the organic light emitting device provided in one embodiment of the present application, the organic material layer including the heterocyclic compound represented by Chemical Formula 1 further includes a heterocyclic compound represented by the following Chemical Formula 2.

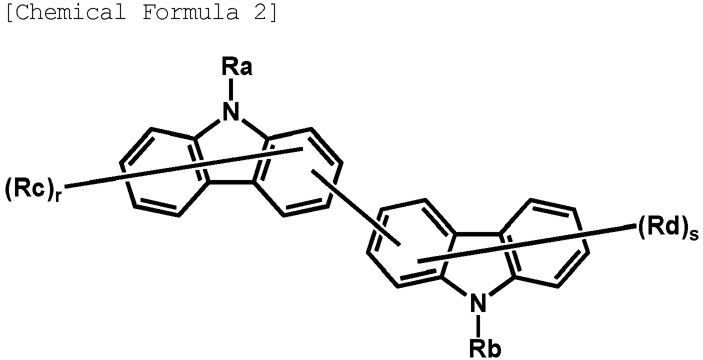

In Chemical Formula 2,
Rc and Rd are the same as or different from each other, and each independently selected from the group consisting of hydrogen; deuterium; a halogen group; -CN; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -Si(R10) (R11) (R12); - P(=O)(R10) (R11); and a substituted or unsubstituted amine group, or two or more groups adjacent to each other bond to each other to form a substituted or unsubstituted C6 to C60 aromatic hydrocarbon ring or a substituted or unsubstituted C2 to C60 heteroring,
R10, R11 and R12 are the same as or different from each other, and each independently hydrogen; deuterium; -CN; a substituted or unsubstituted C1 to C60 alkyl group; a substituted or unsubstituted C6 to C60 aryl group; or a substituted or unsubstituted C2 to C60 heteroaryl group,
Ra and Rb are the same as or different from each other, and each independently a substituted or unsubstituted C6 to C60 aryl group; or a substituted or unsubstituted C2 to C60 heteroaryl group,
r and s are each an integer of 0 to 7, and
when r and s are each 2 or greater, substituents in each parenthesis are the same as or different from each other.

Effects of more superior efficiency and lifetime are obtained when including the heterocyclic compound of Chemical Formula 1 and the heterocyclic compound of Chemical Formula 2 in the organic material layer of the organic light emitting device. Such results may lead to a forecast that an exciplex phenomenon occurs when including the two compounds at the same time.

The exciplex phenomenon is a phenomenon of releasing energy having sizes of a donor (p-host) HOMO level and an acceptor (n-host) LUMO level due to electron exchanges between two molecules. When the exciplex phenomenon occurs between two molecules, reverse intersystem crossing (RISC) occurs, and as a result, internal quantum efficiency of fluorescence may increase up to 100%. When a donor (p-host) having a favorable hole transfer ability and an acceptor (n-host) having a favorable electron transfer ability are used as a host of a light emitting layer, holes are injected to the p-host and electrons are injected to the n-host, and therefore, a driving voltage may be lowered, which resultantly helps with enhancement in the lifetime.

In one embodiment of the present application, Chemical Formula 2 may be represented by the following Chemical Formula 2-1 or Chemical Formula 2-2.

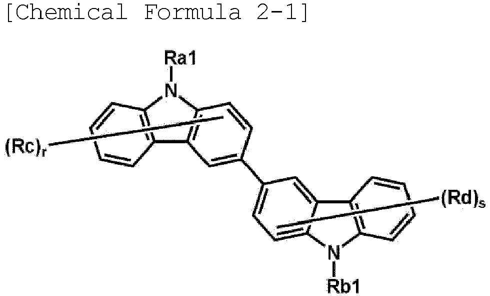

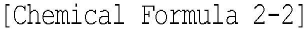

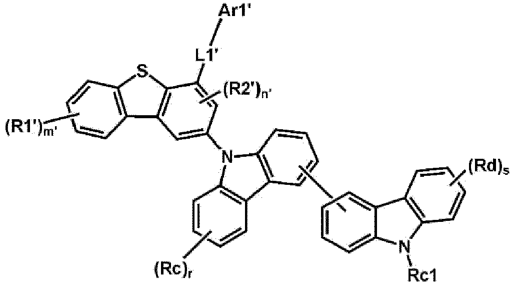

In Chemical Formulae 2-1 and 2-2,
Rc, Rd, r and s have the same definitions as in Chemical Formula 2,
Ra1, Rb1 and Rc1 are the same as or different from each other, and each independently a substituted or unsubstituted C6 to C60 aryl group,
R1' and R2' are the same as or different from each other, and each independently selected from the group consisting of hydrogen; a substituted or unsubstituted C1 to C60 alkyl group; a substituted or unsubstituted C6 to C60 aryl group; and a substituted or unsubstituted C2 to C60 heteroaryl group,
L1' is a direct bond; or a substituted or unsubstituted C6 to C60 arylene group,
Ar1' is a substituted or unsubstituted C6 to C60 aryl group; or a C2 to C60 heteroaryl group substituted or unsubstituted and including at least one of S and O,
m' is an integer of 0 to 4,
n' is an integer of 0 to 2, and
when m' is 2 or greater or n' is 2, substituents in each parenthesis are the same as or different from each other.

In one embodiment of the present application, Rc and Rd of Chemical Formula 2 may be hydrogen.

In one embodiment of the present application, Ra1 and Rb1 of Chemical Formula 2-1 are the same as or different from each other, and may be each independently a substituted or unsubstituted C6 to C60 aryl group.

In another embodiment, Ra1 and Rb1 of Chemical Formula 2-1 are the same as or different from each other, and may be each independently a substituted or unsubstituted C6 to C40 aryl group.

In another embodiment, Ra1 and Rb1 of Chemical Formula 2-1 are the same as or different from each other, and may be each independently a C6 to C40 aryl group unsubstituted or substituted with one or more substituents selected from the group consisting of a C1 to C40 alkyl group, a C6 to C40 aryl group, -CN and -Si(R104)(R105)(R106).

In another embodiment, Ra1 and Rb1 of Chemical Formula 2-1 are the same as or different from each other, and may be each independently a phenyl group unsubstituted or substituted with a phenyl group, -CN or -Si (R104) (R105) (R106); a biphenyl group unsubstituted or substituted with a phenyl group; a naphthyl group; a fluorenyl group unsubstituted or substituted with a methyl group or a phenyl group; a spirobifluorenyl group; or a triphenylenyl group.

In one embodiment of the present application, R104, R105 and R106 of Chemical Formula 2-1 may be a phenyl group.

In one embodiment of the present application, Rc1 of Chemical Formula 2-2 may be a substituted or unsubstituted C6 to C60 aryl group.

In another embodiment, Rc1 of Chemical Formula 2-2 may be a substituted or unsubstituted C6 to C40 aryl group.

In another embodiment, Rc1 of Chemical Formula 2-2 may be a C6 to C40 aryl group.

In another embodiment, Rc1 of Chemical Formula 2-2 may be a phenyl group.

In one embodiment of the present application, R1' and R2' of Chemical Formula 2-2 are the same as or different from each other, and may be each independently selected from the group consisting of hydrogen; a substituted or unsubstituted C1 to C60 alkyl group; a substituted or unsubstituted C6 to C60 aryl group; and a substituted or unsubstituted C2 to C60 heteroaryl group.

In another embodiment, R1' and R2' of Chemical Formula 2-2 are the same as or different from each other, and may be each independently selected from the group consisting of hydrogen; a substituted or unsubstituted C1 to C40 alkyl group; a substituted or unsubstituted C6 to C40 aryl group; and a substituted or unsubstituted C2 to C40 heteroaryl group.

In another embodiment, R1' and R2' of Chemical Formula 2-2 are the same as or different from each other, and may be each independently selected from the group consisting of hydrogen; a C1 to C40 alkyl group; a C6 to C40 aryl group; and a C2 to C40 heteroaryl group.

In another embodiment, R1' and R2' of Chemical Formula 2-2 may be hydrogen.

In one embodiment of the present application, L1' of Chemical Formula 2-2 may be a direct bond; or a substituted or unsubstituted C6 to C60 arylene group.

In another embodiment, L1' of Chemical Formula 2-2 may be a direct bond; or a substituted or unsubstituted C6 to C40 arylene group.

In another embodiment, L1' of Chemical Formula 2-2 may be a direct bond; or a substituted or unsubstituted C6 to C20 arylene group.

In another embodiment, L1' of Chemical Formula 2-2 may be a direct bond; or a C6 to C20 arylene group.

In another embodiment, L1' of Chemical Formula 2-2 may be a direct bond; or a phenylene group.

In one embodiment of the present application, Ar1' of Chemical Formula 2-2 may be a substituted or unsubstituted C6 to C60 aryl group; or a C2 to C60 heteroaryl group substituted or unsubstituted and including at least one of S and O.

In another embodiment, Ar1' of Chemical Formula 2-2 may be a substituted or unsubstituted C6 to C40 aryl group; or a C2 to C40 heteroaryl group substituted or unsubstituted and including at least one of S and O.

In another embodiment, Ar1' of Chemical Formula 2-2 may be a C6 to C40 aryl group unsubstituted or substituted with a C1 to C10 alkyl group; or a C2 to C40 heteroaryl group substituted or unsubstituted and including at least one of S and O.

In another embodiment, Ar1' of Chemical Formula 2-2 may be a phenyl group; a biphenyl group; a naphthyl group; a dimethylfluorenyl group; a dibenzothiophene group; or a dibenzofuran group.

In one embodiment of the present application, Chemical Formula 2 may be represented by any one of the following compounds.

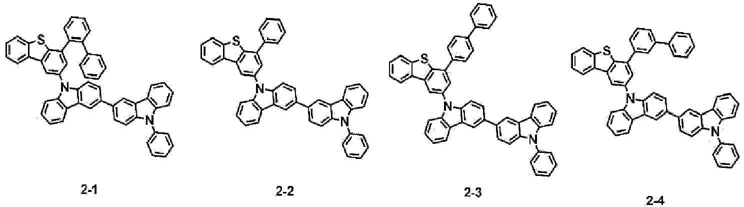

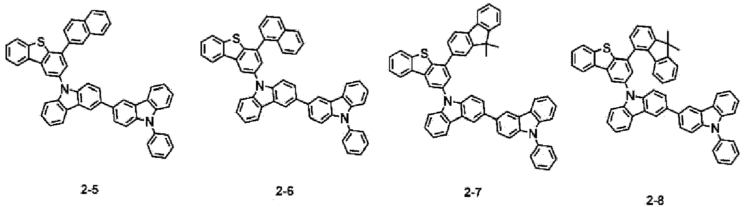

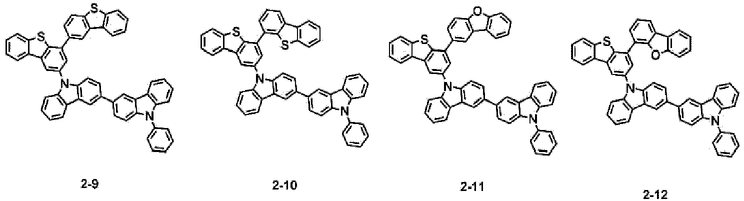

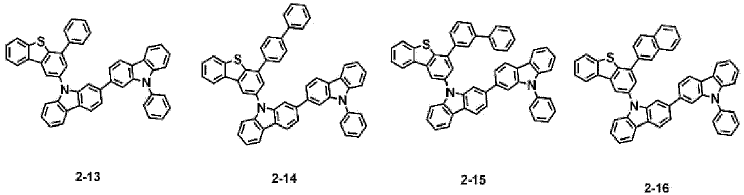

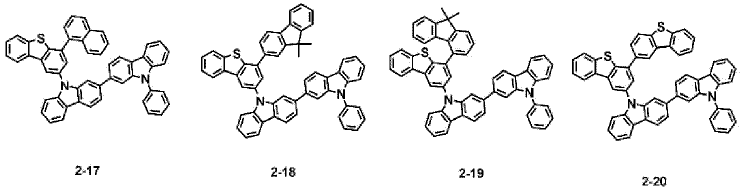

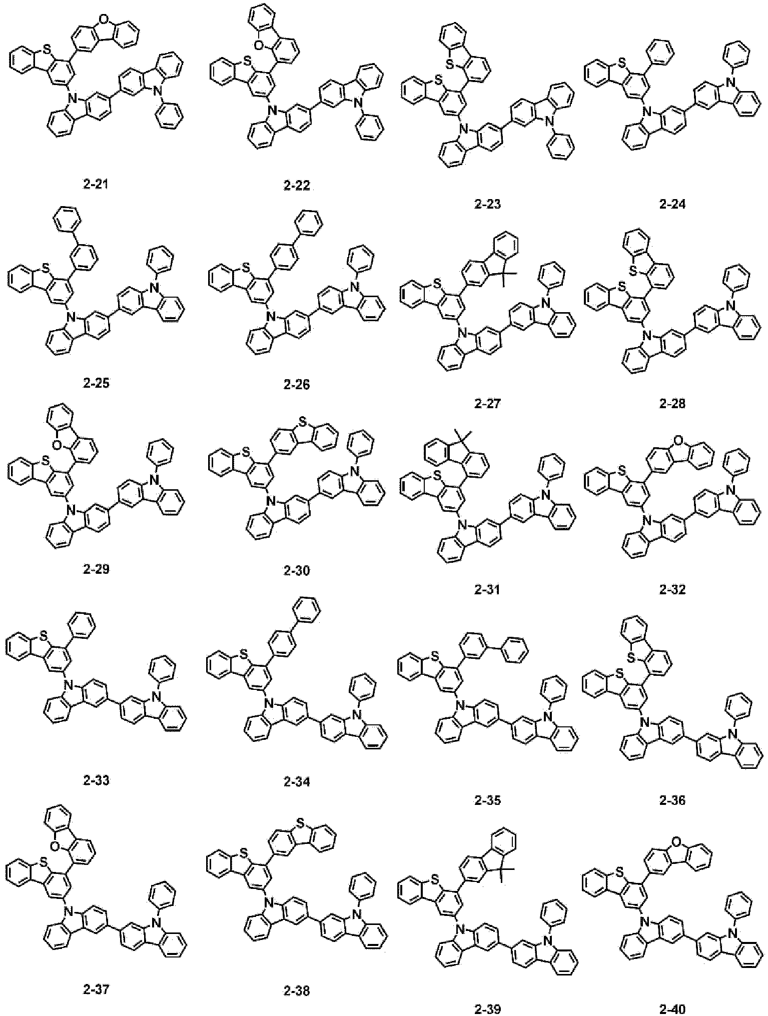

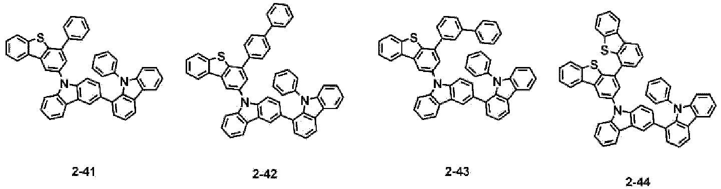

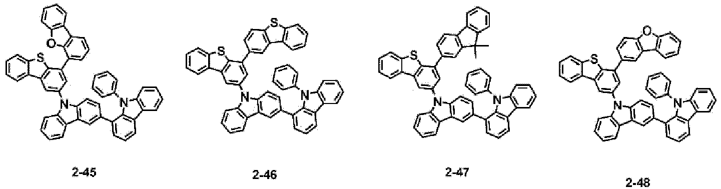

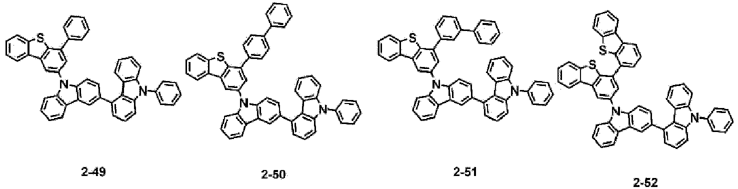

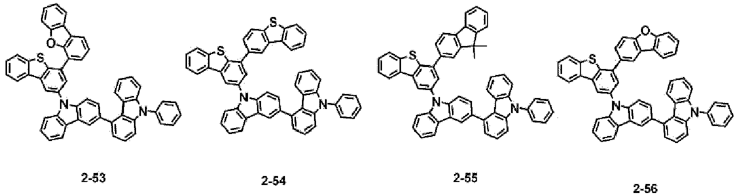

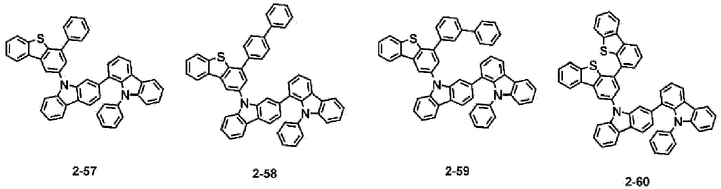

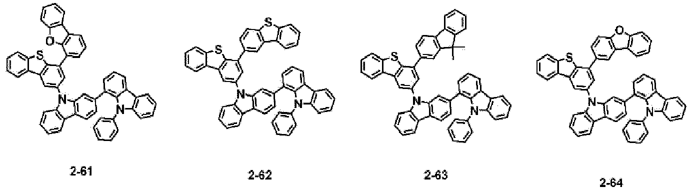

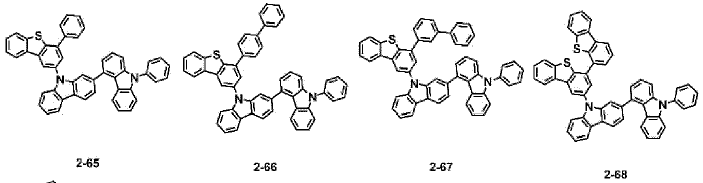

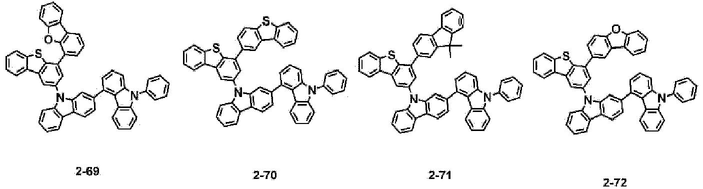

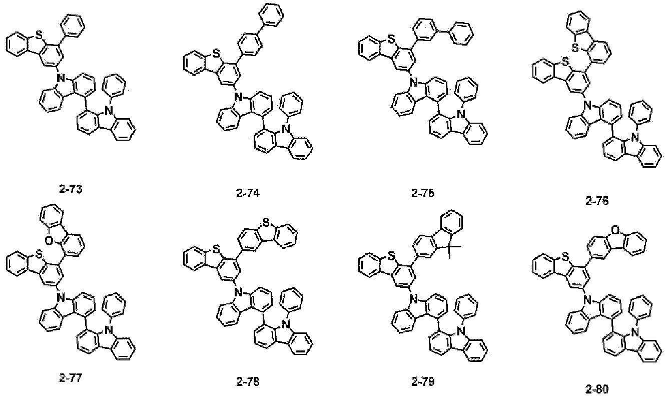

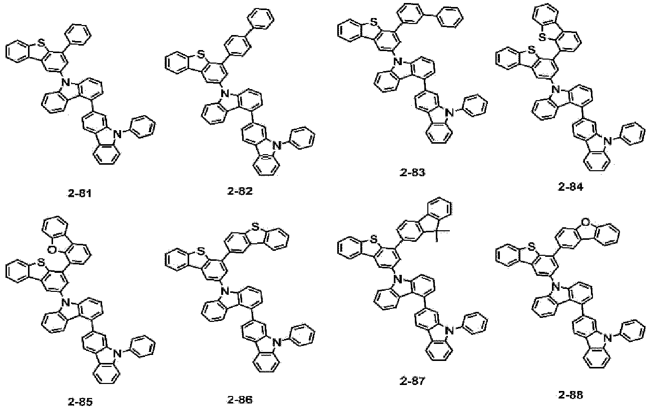

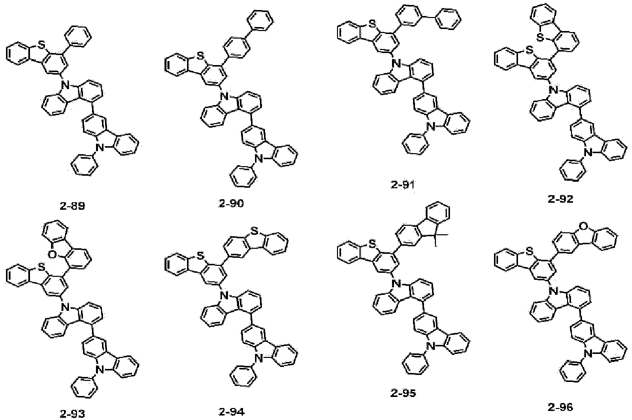

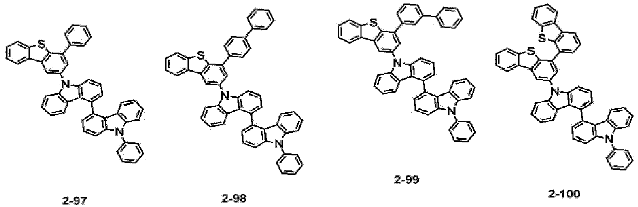

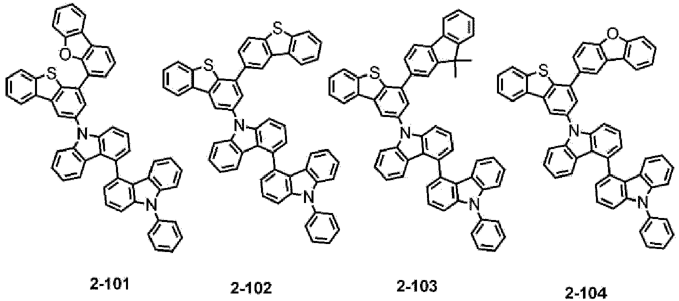

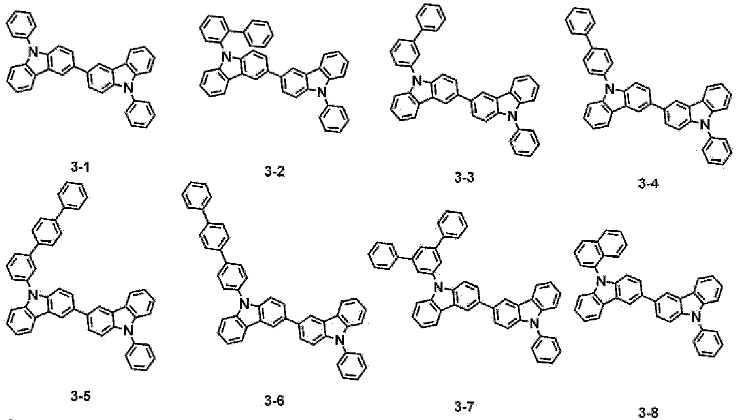

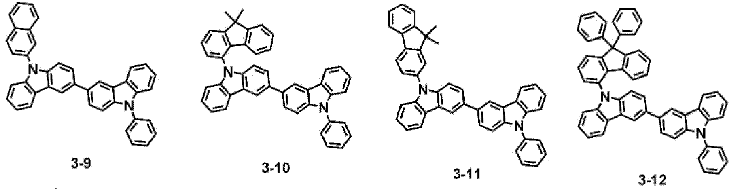

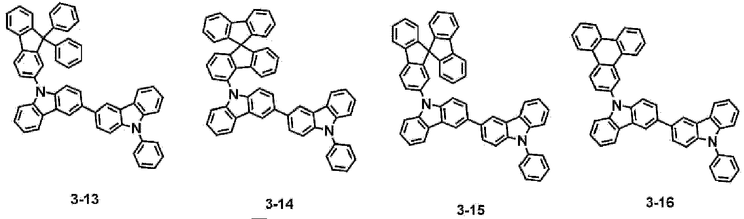

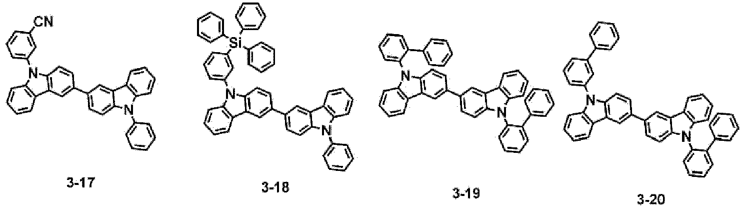

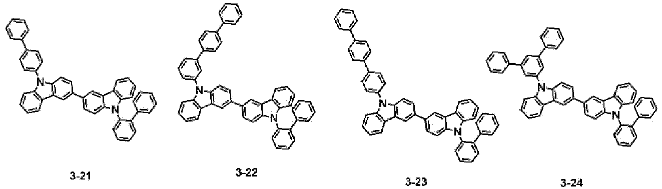

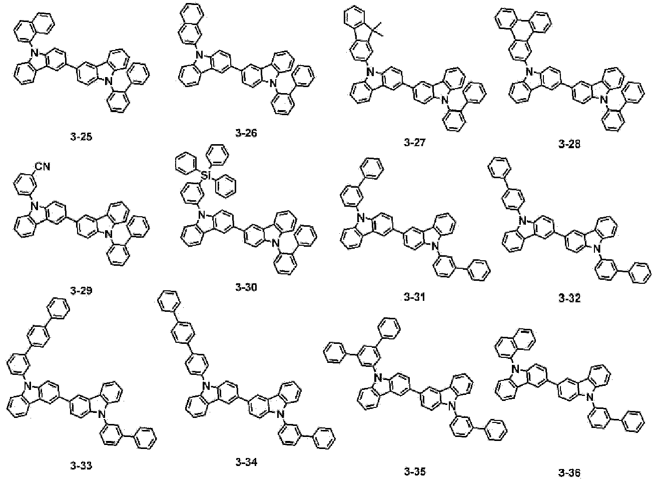

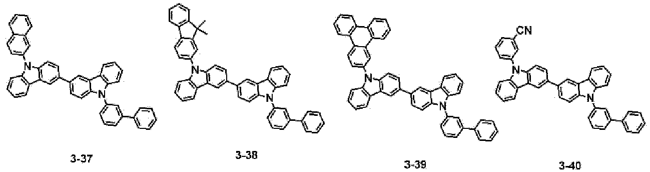

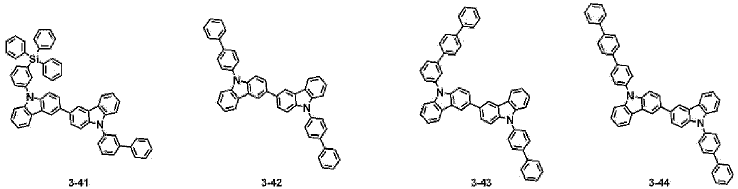

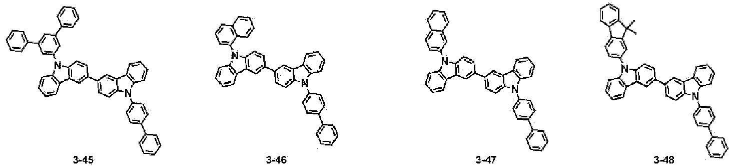

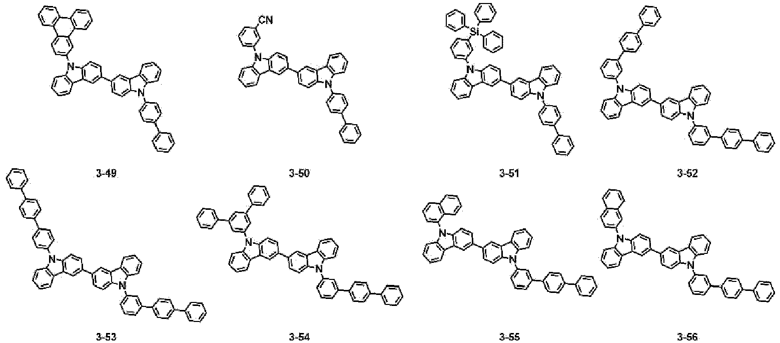

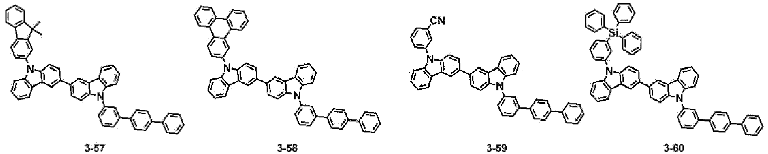

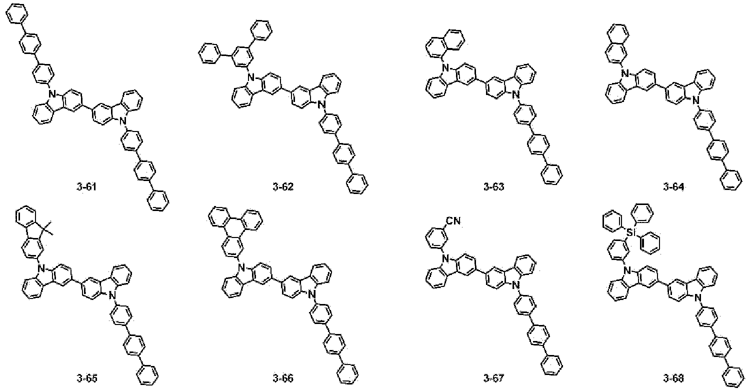

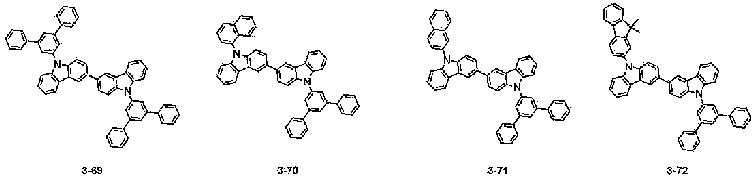

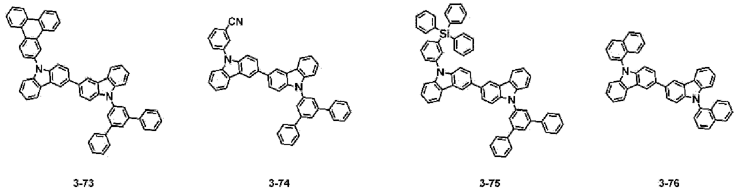

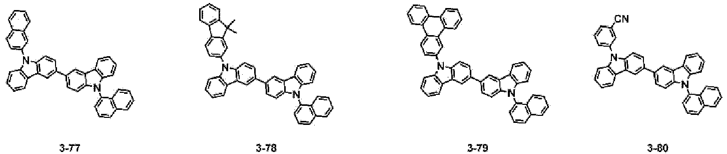

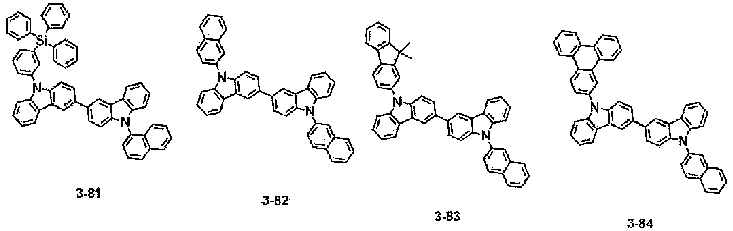

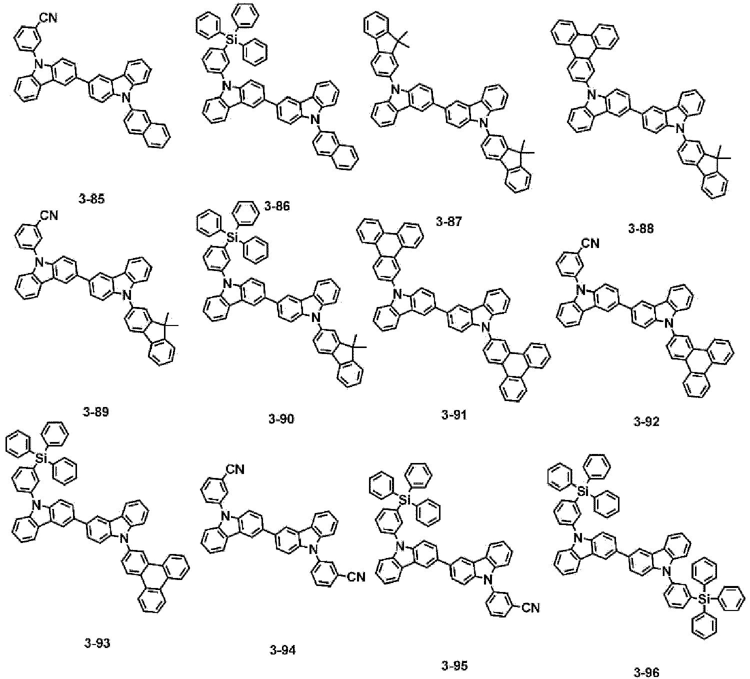

In addition, another embodiment of the present application provides a composition for an organic material layer of an organic light emitting device, the composition including the heterocyclic compound represented by Chemical Formula 1 and the heterocyclic compound represented by Chemical Formula 2.

Specific descriptions on the heterocyclic compound represented by Chemical Formula 1 and the heterocyclic compound represented by Chemical Formula 2 are the same as the descriptions provided above.

In the composition, the heterocyclic compound represented by Chemical Formula 1:the heterocyclic compound represented by Chemical Formula 2 may have a weight ratio of 1:10 to 10:1, 1:8 to 8:1, 1:5 to 5:1 or 1:2 to 2:1, however, the weight ratio is not limited thereto.

The composition may be used when forming an organic material of an organic light emitting device, and may be more preferably used when forming a host of a light emitting layer.

In one embodiment of the present application, the organic material layer includes the heterocyclic compound represented by Chemical Formula 1 and the heterocyclic compound represented by Chemical Formula 2, and a phosphorescent dopant may be used therewith.

In one embodiment of the present application, the organic material layer includes the heterocyclic compound represented by Chemical Formula 1 and the heterocyclic compound represented by Chemical Formula 2, and an iridium-based dopant may be used therewith.

As a material of the phosphorescent dopant, those known in the art may be used.

For example, phosphorescent dopant materials represented by LL'MX', LL'L"M, LMX'X", L₂MX' and L₃M may be used, however, the scope of the present disclosure is not limited to these examples.

Herein, L, L', L", X' and X" are a bidentate ligand different from each other, and M is a metal forming an octahedral complex.

M may include iridium, platinum, osmium and the like.

L is an anionic bidentate ligand coordinated to M as the iridium-based dopant by sp2 carbon and heteroatom, and X may function to trap electrons or holes. Nonlimiting examples of L may include 2-(1-naphthyl)benzoxazole, (2-phenylbenzoxazole), (2-phenylbenzothiazole), (2-phenylbenzothiazole), (7,8-benzoquinoline), (thiophene group pyrizine), phenylpyridine, benzothiophene group pyrizine, 3-methoxy-2-phenylpyridine, thiophene group pyrizine, tolylpyridine and the like. Nonlimiting examples of X' and X" may include acetylacetonate (acac), hexafluoroacetylacetonate, salicylidene, picolinate, 8-hydroxyquinolinate and the like.

More specific examples thereof are described below, however, the phosphorescent dopant is not limited to these examples.

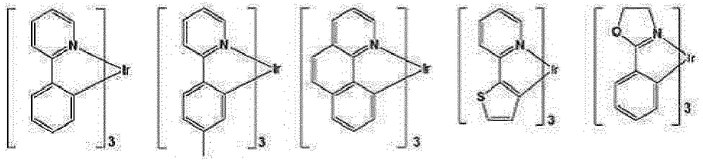

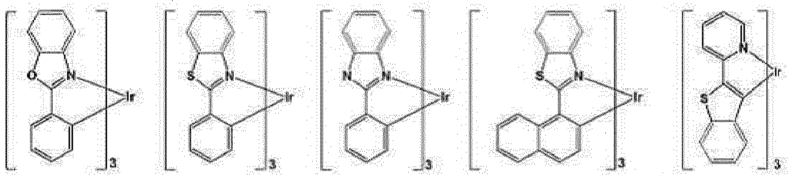

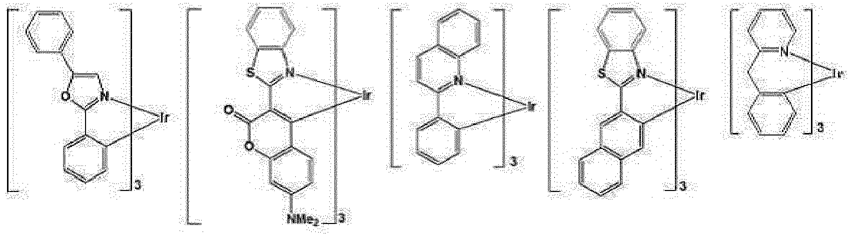

In one embodiment of the present application, as the iridium-based dopant, Ir(ppy)₃ may be used as a green phosphorescent dopant.

In one embodiment of the present application, a content of the dopant may be from 1% to 15%, preferably from 3% to 10% and more preferably from 5% to 10% based on the whole light emitting layer.

In the organic light emitting device of the present disclosure, the organic material layer includes an electron injection layer or an electron transfer layer, and the electron injection layer or the electron transfer layer may include the heterocyclic compound.

In another organic light emitting device, the organic material layer includes an electron blocking layer or a hole blocking layer, and the electron blocking layer or the hole blocking layer may include the heterocyclic compound.

In another organic light emitting device, the organic material layer includes an electron transfer layer, a light emitting layer or a hole blocking layer, and the electron transfer layer, the light emitting layer or the hole blocking layer may include the heterocyclic compound.

The organic light emitting device of the present disclosure may further include one, two or more layers selected from the group consisting of a light emitting layer, a hole injection layer, a hole transfer layer, an electron injection layer, an electron transfer layer, an electron blocking layer and a hole blocking layer.

FIG 1 to FIG. 3 illustrate a lamination order of electrodes and organic material layers of an organic light emitting device according to one embodiment of the present application. However, the scope of the present application is not limited to these diagrams, and structures of organic light emitting devices known in the art may also be used in the present application.

FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

FIG. 3 illustrates a case of the organic material layer being a multilayer. The organic light emitting device according to FIG. 3 includes a hole injection layer (301), a hole transfer layer (302), a light emitting layer (303), a hole blocking layer (304), an electron transfer layer (305) and an electron injection layer (306). However, the scope of the present application is not limited to such a lamination structure, and as necessary, layers other than the light emitting layer may not be included, and other necessary functional layers may be further added.

One embodiment of the present application provides a method for manufacturing an organic light emitting device, the method including preparing a substrate; forming a first electrode on the substrate; forming one or more organic material layers on the first electrode; and forming a second electrode on the organic material layer, wherein the forming of organic material layers includes forming one or more organic material layers using the composition for an organic material layer according to one embodiment of the present application.

In the method for manufacturing an organic light emitting device provided in one embodiment of the present application, the forming of organic material layers is forming the heterocyclic compound of Chemical Formula 1 and the heterocyclic compound of Chemical Formula 2 using a thermal vacuum deposition method after pre-mixing.

The pre-mixing means first mixing the heterocyclic compound of Chemical Formula 1 and the heterocyclic compound of Chemical Formula 2 in one source of supply before depositing on the organic material layer.

The premixed material may be referred to as the composition for an organic material layer according to one embodiment of the present application.

The organic material layer including Chemical Formula 1 may further include other materials as necessary.

The organic material layer including both Chemical Formula 1 and Chemical Formula 2 may further include other materials as necessary.

In the organic light emitting device according to one embodiment of the present application, materials other than the compound of Chemical Formula 1 or Chemical Formula 2 are illustrated below, however, these are for illustrative purposes only and not for limiting the scope of the present application, and may be replaced by materials known in the art.

As the anode material, materials having relatively large work function may be used, and transparent conductive oxides, metals, conductive polymers or the like may be used. Specific examples of the anode material include metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDOT), polypyrrole and polyaniline, and the like, but are not limited thereto.

As the cathode material, materials having relatively small work function may be used, and metals, metal oxides, conductive polymers or the like may be used. Specific examples of the cathode material include metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tri[phenyl(m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], polyaniline/dodecylbenzene sulfonic acid, poly(3,4-ethylenedioxythiophene)/poly(4-styrenesulfonate), polyaniline/camphor sulfonic acid or polyaniline/poly(4-styrene-sulfonate) that are conductive polymers having solubility, and the like, may be used.

As the hole transfer material, pyrazoline derivatives, arylamine-based derivatives, stilbene derivatives, triphenyldiamine derivatives and the like may be used, and low molecular or high molecular materials may also be used.

As the electron transfer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

As examples of the electron injection material, LiF is typically used in the art, however, the present application is not limited thereto.

As the light emitting material, red, green or blue light emitting materials may be used, and as necessary, two or more light emitting materials may be mixed and used. Herein, two or more light emitting materials may be used by being deposited as individual sources of supply or by being premixed and deposited as one source of supply. In addition, fluorescent materials may also be used as the light emitting material, however, phosphorescent materials may also be used. As the light emitting material, materials emitting light by bonding electrons and holes injected from an anode and a cathode, respectively, may be used alone, however, materials having a host material and a dopant material involving in light emission together may also be used.

When mixing light emitting material hosts, same series hosts may be mixed, or different series hosts may be mixed. For example, any two or more types of materials among n-type host materials or p-type host materials may be selected and used as a host material of a light emitting layer.

The organic light emitting device according to one embodiment of the present application may be a top-emission type, a bottom-emission type or a dual-emission type depending on the materials used.

The heterocyclic compound according to one embodiment of the present application may also be used in an organic electronic device including an organic solar cell, an organic photo conductor, an organic transistor and the like under a similar principle used in the organic light emitting device.

Hereinafter, the present specification will be described in more detail with reference to examples, however, these are for illustrative purposes only, and the scope of the present application is not limited thereto.

### <Preparation Example>

### <Preparation Example 1> Preparation of Compound 1-1

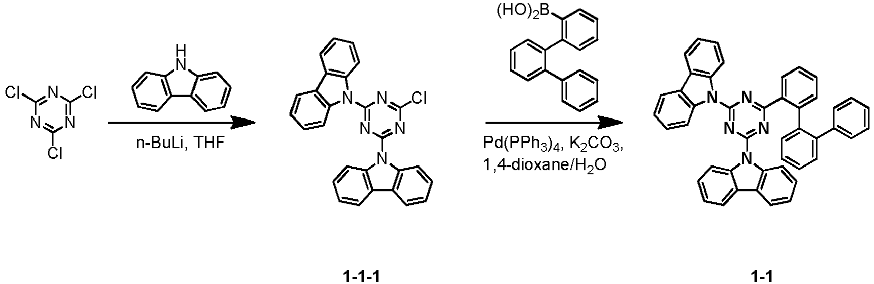

### 1) Preparation of Intermediate 1-1-1

After dissolving 9H-carbazole (10.0 g, 59.8 mM) in THF, the flask was nitrogen substituted at -78°C. 2.5 M n-BuLi (28.7 mL, 71.8 mM) was slowly introduced thereto at -78°C, and the result was stirred for 30 minutes. 2,4,6-Trichloro-1,3,5-triazine (5.5 g, 29.9 mM) was introduced thereto, and the result was stirred for 2 hours at room temperature. After the reaction was completed, the result was extracted by introducing distilled water and DCM thereto at room temperature, and after drying the organic layer with MgSO₄, the solvent was removed using a rotary evaporator. The reaction material was recrystallized with methanol to obtain Intermediate 1-1-1 (10.9 g, 82%).

### 2) Preparation of Compound 1-1

After dissolving Intermediate 1-1-1 (10 g, 22.42 mM), [1,1':2',1"-terphenyl]-2-ylboronic acid (6.8 g, 24.67 mM), Pd(PPh₃)₄ (1.3 g, 1.1 mM) and K₂CO₃ (6.2 g, 44.84 mM) in 1,4-dioxane/H₂O (250 mL/50 mL), the mixture was refluxed for 24 hours. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:Hex=1:3), and recrystallized with methanol to obtain target Compound 1-1 (12.3 g, 86%).

Target compounds were synthesized in the same manner as in Preparation Example 1 except that Compound A of the following Table 1 was used instead of [1,1':2',1"-terphenyl]-2-ylboronic acid.

**[Table 1]**

| Compound No. | Compound A | Target Compound |
|---|---|---|
| 1-5 | 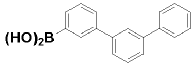 | 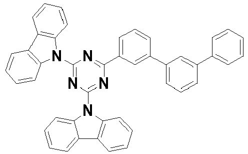 |
| 1-9 | 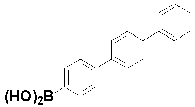 | 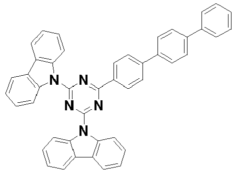 |
| 1-40 | 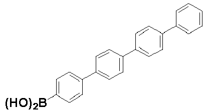 | 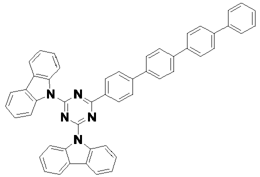 |
| 1-78 | 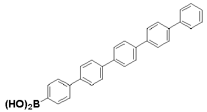 | 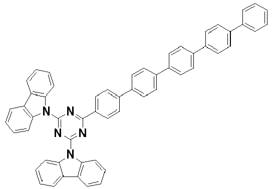 |
| 1-79 | 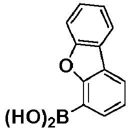 | 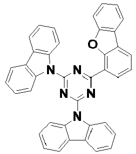 |
| 1-80 | 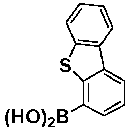 | 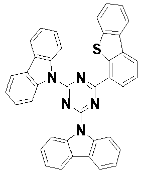 |
| 1-81 | 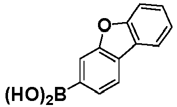 | 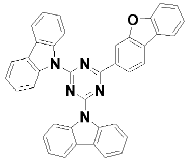 |
| 1-88 | 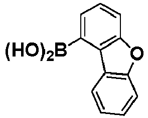 | 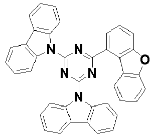 |
| 1-115 | 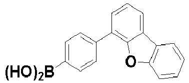 | 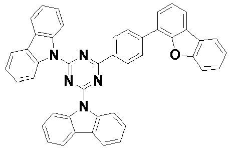 |
| 1-117 | 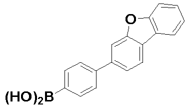 | 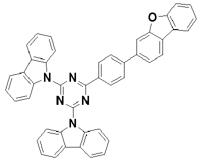 |
| 1-123 | 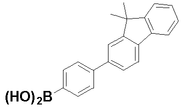 | 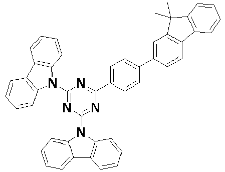 |

### <Preparation Example 2> Preparation of Compound 1-177

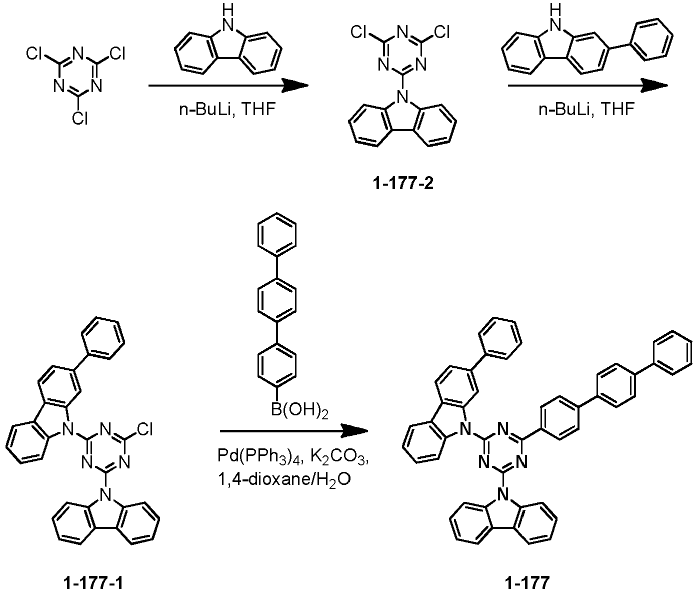

### 1) Preparation of Intermediate 1-177-2

After dissolving 9H-carbazole (10.0 g, 59.8 mM) in THF, the flask was nitrogen substituted at -78°C. 2.5 M n-BuLi (28.7 mL, 71.8 mM) was slowly introduced thereto at -78°C, and the result was stirred for 30 minutes. 2,4,6-Trichloro-1,3,5-triazine (10.9 g, 59.8 mM) was introduced thereto, and the result was stirred for 2 hours at room temperature. After the reaction was completed, the result was extracted by introducing distilled water and DCM thereto at room temperature, and after drying the organic layer with MgSO₄, the solvent was removed using a rotary evaporator. The reaction material was recrystallized with methanol to obtain Intermediate 1-177-2 (15.6 g, 83%).

### 2) Preparation of Intermediate 1-177-1

After dissolving 2-phenyl-9H-carbazole (14.5 g, 59.8 mM) in THF, the flask was nitrogen substituted at -78°C. 2.5 M n-BuLi (28.7 mL, 71.8 mM) was slowly introduced thereto at -78°C, and the result was stirred for 30 minutes. Intermediate 1-177-2 (18.8 g, 59.8 mM) was introduced thereto, and the result was stirred for 2 hours at room temperature. After the reaction was completed, the result was extracted by introducing distilled water and DCM thereto at room temperature, and after drying the organic layer with MgSO₄, the solvent was removed using a rotary evaporator. The reaction material was recrystallized with methanol to obtain Intermediate 1-177-1 (25.6 g, 82%).

### 2) Preparation of Compound 1-177

After dissolving Intermediate 1-177-1 (11.7 g, 22.42 mM), [1,1':2',1"-terphenyl]-4-ylboronic acid (6.8 g, 24.67 mM), Pd(PPh₃)₄ (1.3 g, 1.1 mM) and K₂CO₃ (6.2 g, 44.84 mM) in 1,4-dioxane/H₂O (250 mL/50 mL), the mixture was refluxed for 24 hours. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:Hex=1:3), and recrystallized with methanol to obtain target Compound 1-177 (13.6 g, 85%).

Target compounds were synthesized in the same manner as in Preparation Example 2 except that Compound B of the following Table 2 was used instead of [1,1':2',1"-terphenyl]-4-ylboronic acid.

**[Table 2]**

| Compound No. | Compound B | Target Compound |
|---|---|---|
| 1-208 | 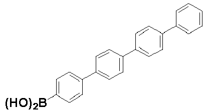 | 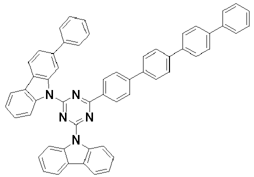 |
| 1-211 | 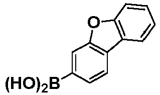 | 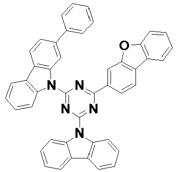 |

Target compounds were synthesized in the same manner as in Preparation Example 1 except that Compound C of the following Table 3 was used instead of 9H-carbazole, and Compound D of the following Table 3 was used instead of [1,1':2',1"-terphenyl]-2-ylboronic acid.

**[Table 3]**

| Compound No. | Compound C | Compound D | Target Compound |
|---|---|---|---|
| 1-298 | 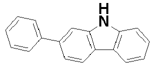 | 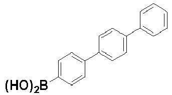 | 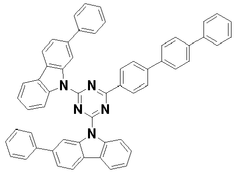 |
| 1-301 | 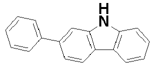 | 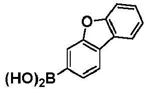 | 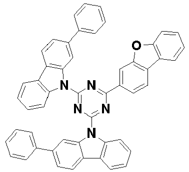 |

### <Preparation Example 3> Preparation of Compound 2-2

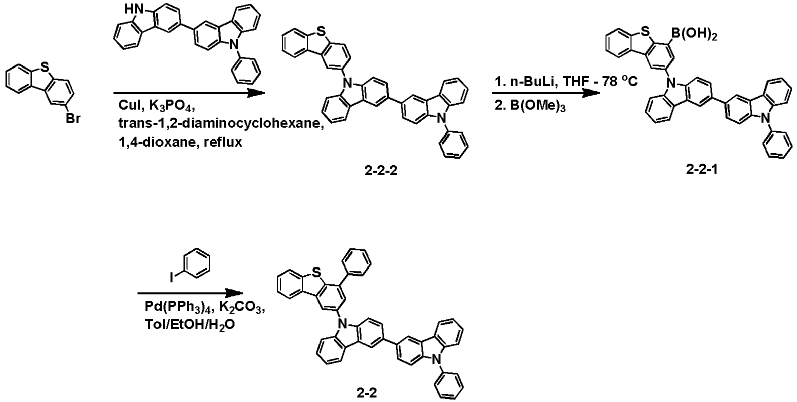

### 1) Preparation of Intermediate 2-2-2

After dissolving 2-bromodibenzo[b,d]thiophene (4.2 g, 15.8 mM), 9-phenyl-9H,9'H-3,3'-bicarbazole (6.5 g, 15.8 mM), CuI (3.0 g, 15.8 mM), trans-1,2-diaminocyclohexane (1.9 mL, 15.8 mM) and K₃PO₄ (3.3 g, 31.6 mM) in 1,4-oxane (100 mL), the mixture was refluxed for 24 hours. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:Hex=1:3), and recrystallized with methanol to obtain Intermediate 2-2-2 (7.9 g, 85%).

### 2) Preparation of Intermediate 2-2-1

To a mixture solution of Intermediate 2-2-2 (8.4 g, 14.3 mmol) and THF (100 mL), 2.5 M n-BuLi (7.4 mL, 18.6 mmol) was added dropwise at -78°C, and the result was stirred for 1 hour at room temperature. Trimethyl borate (4.8 mL, 42.9 mmol) was added dropwise to the reaction mixture, and the result was stirred for 2 hours at room temperature. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:MeOH=100:3), and recrystallized with DCM to obtain Intermediate 2-2-1 (3.9 g, 70%).

### 3) Preparation of Compound 2-2

After dissolving Intermediate 2-2-1 (6.7 g, 10.5 mM), iodobenzene (2.1 g, 10.5 mM), Pd(PPh₃)₄ (606 mg, 0.52 mM) and K₂CO₃ (2.9 g, 21.0 mM) in toluene/EtOH/H₂O (100 mL/20 mL/20 mL), the mixture was refluxed for 12 hours. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:Hex=1:3), and recrystallized with methanol to obtain target Compound 2-2 (4.9 g, 70%).

### <Preparation Example 4> Synthesis of Compound 2-3

Target Compound 2-3 (83%) was obtained in the same manner as in Preparation of Compound 2-2 of Preparation Example 3 except that 4-iodo-1,1'-biphenyl was used instead of iodobenzene.

### <Preparation Example 5> Synthesis of Compound 2-12

Target Compound 2-12 (80%) was obtained in the same manner as in Preparation of Compound 2-2 of Preparation Example 3 except that 4-iododibenzo[b,d]furan was used instead of iodobenzene.

### <Preparation Example 6> Synthesis of Compound 3-3

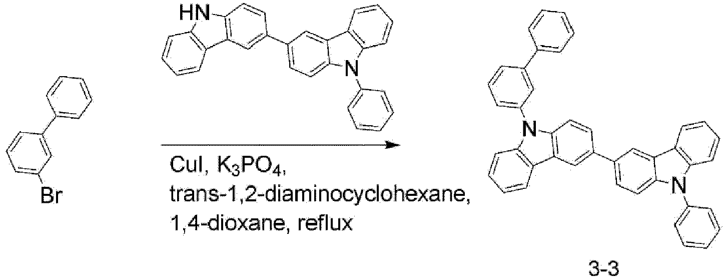

After dissolving 3-bromo-1,1'-biphenyl (3.7 g, 15.8 mM), 9-phenyl-9H,9'H-3,3'-bicarbazole (6.5 g, 15.8 mM), CuI (3.0 g, 15.8 mM), trans-1,2-diaminocyclohexane (1.9 mL, 15.8 mM) and K₃PO₄ (3.3 g, 31.6 mM) in 1,4-dioxane (100 mL), the mixture was refluxed for 24 hours. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:Hex=1:3), and recrystallized with methanol to obtain target Compound 3-3 (7.5 g, 85%).

Target compounds were synthesized in the same manner as in Preparation Example 6 except that Compound E of the following Table 4 was used instead of 3-bromo-1,1'-biphenyl, and Compound F of the following Table 4 was used instead of 9-phenyl-9H,9'H-3,3'-bicarbazole.

**[Table 4]**

| Compound No. | Compound E | Compound F | Target Compound |
|---|---|---|---|
| 3-4 | 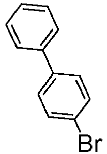 | 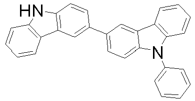 | 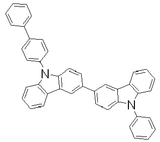 |
| 3-7 | 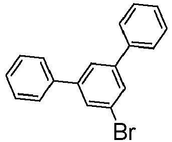 | | 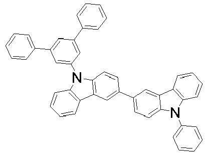 |
| 3-9 | 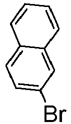 | | 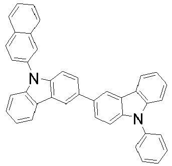 |
| 3-31 | 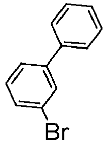 | 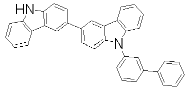 | 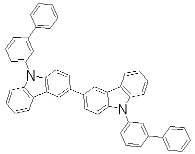 |
| 3-32 | 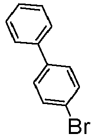 | | 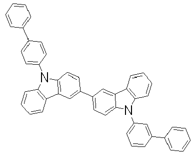 |
| 3-42 | 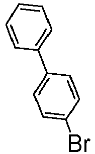 | 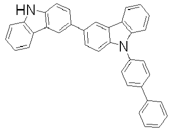 | 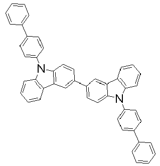 |

The rest of the compounds other than the compounds described in Table 1 to Table 4 were also prepared in the same manner as in the preparation examples described above.

The following Table 5 and Table 6 show 1H NMR data and FD-MS data of the synthesized compounds, and through the following data, syntheses of the target compounds were identified.

**[Table 5]**

| Compound No. | ¹H NMR (CDCl₃, 200 Mz) |
|---|---|
| 1-1 | δ=8.55 (2H, d), 8.19 (2H, d), 7.96-7.94 (6H, m), 7.79 (2H, d), 7.60-7.35 (13H, m), 7.20-7.16 (4H, t) |
| 1-5 | δ=8.55 (2H, d), 8.38 (1H, d), 8.19 (2H, d), 7.94 (4H, m), 7.75-7.73 (4H, |
| | m), 7.61-7.35 (12H, m), 7.20-7.16 (4H, m) |
| 1-9 | δ=8.55 (2H, d), 8.19 (2H, d), 7.96-7.94 (4H, m), 7.75 (2H, m), 7.58-7.35 (9H, m), 7.25-7.16 (10H, m) |
| 1-40 | δ=8.55 (2H, d), 8.19 (2H, d), 7.96-7.94 (4H, m), 7.75 (2H, m), 7.58-7.35 (9H, m), 7.25-7.16 (14H, m) |
| 1-78 | δ=8.55 (2H, d), 8.19 (2H, d), 7.96-7.94 (4H, m), 7.75 (2H, m), 7.58-7.35 (9H, m), 7.25-7.16 (18H, m) |
| 1-79 | δ=8.55 (2H, d), 8.19 (2H, d), 8.08 (1H, d), 7.98-7.88 (4H, m), 7.58-7.50 (6H, m), 7.39-7.31 (4H, m), 7.20-7.16 (4H, m) |
| 1-80 | δ=8.55 (3H, d), 8.45 (1H, d), 8.19 (2H, d), 7.94-7.92 (4H, m), 7.70 (1H, t), 7.58-7.49 (6H, m), 7.35 (2H, t), 7.20-7.16 (4H, m) |
| 1-81 | δ=8.55 (2H, d), 8.19 (2H, d), 8.03-7.94 (4H, m), 7.82-7.76 (2H, m), 7.58-7.16 (13H, m) |
| 1-88 | δ=8.55 (2H, d), 8.19 (2H, d), 7.98-7.94 (3H, m), 7.82 (1H, d), 7.69 (1H, d), 7.58-7.50 (6H, m), 7.39-7.31 (4H, m), 7.20-7.16 (2H, m) |
| 1-93 | δ=9.60 (1H, d), 9.27 (1H, s), 8.55 (2H, d), 8.33-8.30 (2H, m), 8.19-8.15 (3H, m), 7.94 (2H, d), 7.70-7.50 (10H, m), 7.35 (2H, t), 7.20-7.16 (4H, m) |
| 1-115 | δ=8.55 (2H, d), 8.19 (2H, d), 8.08-7.94 (7H, m), 7.58-7.50 (6H, m), 7.39-7.16 (10H, m) |
| 1-117 | δ=8.55 (2H, d), 8.19 (2H, d), 8.03-7.94 (6H, m), 7.82-7.76 (2H, m), 7.58-7.50 (5H, m), 7.39-7.16 (10H, m) |
| 1-123 | δ=8.55 (2H, d), 8.19 (2H, d), 8.09 (1H, d), 7.96-7.89 (6H, m), 7.78 (1H, d), 7.58-7.50 (5H, m), 7.38-7.16 (10H, m), 1.69 (6H, s) |
| 1-177 | δ=8.55 (2H, d), 8.31 (1H, d), 8.19 (1H, d), 7.96-7.91 (5H, m), 7.75-7.74 (5H, m), 7.58-7.35 (10H, m), 7.25-7.16 (9H, m) |
| 1-208 | δ=8.55 (2H, d), 8.31 (1H, d), 8.19 (1H, d), 7.96-7.91 (5H, m), 7.75-7.74 (5H, m), 7.58(1H, d), 7.50-7.35 (9H, m), 7.25-7.16 (13H, m) |
| 1-211 | δ=8.55 (2H, d), 8.31 (1H, d), 8.19 (1H, d), 8.03-7.91 (5H, m), 7.82-7.74 (5H, m), 7.58-7.31 (10H, m), 7.20-7.16 (3H, m) |
| 1-298 | δ=8.62 (1H, d), 8.55 (1H, d), 8.31 (1H, d), 8.22-8.19 (2H, m), 7.96-7.91 (4H, m), 7.75-7.74 (8H, m), 7.58-7.35 (12H, m), 7.25-7.16 (8H, m) |
| 1-301 | δ=8.62 (1H, d), 8.55 (1H, d), 8.31 (1H, d), 8.22-8.19 (2H, m), 8.03-7.91 (4H, m), 7.82-7.74 (8H, m), 7.58-7.31 (12H, m), 7.20-7.16 (2H, m) |
| 2-2 | δ=8.55 (1H, d), 8.45(1H, d), 8.30(1H, d), 8.19(1H, d), 8.13(1H, d), 8.00~7.89(6H, m), 7.77(2H, m), 7.62~7.35(15H, m), 7.20~7.16(2H, m) |
| 2-3 | δ=8.55 (1H, d), 8.45(1H, d), 8.30(1H, d), 8.19(1H, d), 8.13(1H, d), 8.00~7.89(6H, m), 7.77-7.75(4H, m), 7.62~7.35(13H, m), 7.25~7.16(6H, m) |
| 2-12 | δ=8.55 (1H, d), 8.45(1H, d), 8.30(1H, d), 8.19-7.89 (11H, m), 7.77(2H, m), 7.62~7.31(14H, m), 7.20~7.16(2H, m) |
| 3-3 | δ=8.55 (1H, d), 8.30 (1H, d), 8.21-8.13 (3H, m), 7.99-7.89 (4H, m), 7.77-7.35 (17H, m), 7.20-7.16 (2H, m) |
| 3-4 | δ=8.55 (1H, d), 8.30(1H, d), 8.19-8.13(2H, m), 7.99-7.89(8H, m), 7.77-7.75 (3H, m), 7.62-7.35 (11H, m), 7.20-7.16 (2H, m) |
| 3-7 | δ=8.55 (1H, d), 8.31-8.30 (3H, d), 8.19-8.13 (2H, m), 7.99-7.89 (5H, m), 7.77-7.75 (5H, m), 7.62-7.35 (14H, m), 7.20-7.16 (2H, m) |
| 3-9 | δ=8.55 (1H, d), 8.30 (1H, d), 8.19-8.13 (2H, m), 8.03-7.77 (9H, m), 7.62-7.50 (9H, m), 7.36-7.35 (2H, m), 7.20-7.16 (2H, m) |
| 3-31 | δ=8.55 (1H, d), 8.30 (1H, d), 8.21-8.13 (4H, m), 7.99-7.89 (4H, m), 7.77-7.35 (20H, m), 7.20-7.16 (2H, m) |
| 3-32 | δ=8.55 (1H, d), 8.30 (1H, d), 8.21-8.13 (3H, m), 7.99-7.89 (8H, m), 7.77-7.35 (17H, m), 7.20-7.16 (2H, m) |
| 3-42 | δ=8.55 (1H, d), 8.30 (1H, d), 8.19 (1H, d), 8.13 (1H, d), 7.99-7.89 (12H, m), 7.77-7.75 (5H, m), 7.58 (1H, d), 7.50-7.35 (8H, m), 7.20-7.16 (2H, m) |

**[Table 6]**

| Compou nd | FD-MS | Compou nd | FD-MS |
|---|---|---|---|
| 1-1 | m/z=639.24 (C₄₅H₂₉N₅=639.76) | 1-5 | m/z=639.24 (C₄₅H₂₉N₅=639.76) |
| 1-9 | m/z=639.24 (C₄₅H₂₉N₅=639.76) | 1-40 | m/z=715.27 (C₅₁H₃₃N₅=715.86) |
| 1-78 | m/z=791.30 (C₅₇H₃₇N₅=791.30) | 1-79 | m/z=577.19 (C₃₉H₂₃N₅O=577.65) |
| 1-80 | m/z=593.17 (C₃₉H₂₃N₅S=593.71) | 1-81 | m/z=577.19 (C₃₉H₂₃N₅O=577.65) |
| 1-88 | m/z=577.19 (C₃₉H₂₃N₅O=577.65) | 1-115 | m/z=653.22 (C₄₅H₂₇N₅O=653.75) |
| 1-117 | m/z=653.22 (C₄₅H₂₇N₅O=653.75) | 1-123 | m/z=679.27 (C₄₈H₃₃N₅=679. 83) |
| 1-177 | m/z=715.27 (C₅₁H₃₃N₅=715.86) | 1-208 | m/z=791.30 (C₅₇H₃₇N₅=791.30) |
| 1-211 | m/z=653.22 (C₄₅H₂₇N₅O=653.75) | 1-298 | m/z=791.30 (C₅₇H₃₇N₅=791.30) |
| 1-301 | m/z=729.25 (C₅₁H₃₁N₅O=729.84) | 2-2 | m/z=666.21 (C₄₈H₃₀N₂S=666.84) |
| 2-3 | m/z=742.24 (C₅₄H₃₄N₂S=742.94) | 2-12 | m/z=756.22 (C₅₄H₃₂N₂OS=756.92) |
| 3-3 | m/z=560.23 (C₄₂H₂₈N₂=560.70) | 3-4 | m/z=560.23 (C₄₂H₂₈N₂=560.70) |
| 3-7 | m/z=636.26 (C₄₈H₃₂N₂=636.80) | 3-9 | m/z=534.21 (C₄₀H₂₆N₂=534. 66) |
| 3-31 | m/z=636.26 (C₄₈H₃₂N₂=636.80) | 3-32 | m/z=636.26 (C₄₈H₃₂N₂=636.80) |
| 3-42 | m/z=636.26 (C₄₈H₃₂N₂=636.80) | | |

### <Experimental Example 1>

### - Manufacture of Organic Light Emitting Device

A glass substrate on which indium tin oxide (ITO) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and ultraviolet ozone (UVO) treatment was conducted for 5 minutes using UV in an ultraviolet (UV) cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4',4"-tris[2-naphthyl(phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. As the light emitting layer, a compound of Chemical Formula 1 described in the following Table 6 was deposited to 400 Å, and a green phosphorescent dopant [Ir(ppy)₃] was doped and deposited by 7% with respect to the deposited thickness of the light emitting layer. After that, BCP (bathocuproine) was deposited to 60 Å as a hole blocking layer, and Alq₃ was deposited to 200 Å thereon as an electron transfer layer. Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic electroluminescent device was manufactured.

Meanwhile, all the organic compounds required to manufacture the OLED were vacuum sublimation purified under 10⁻⁸ torr to 10⁻⁶ torr for each material to be used in the OLED manufacture.

For each of the organic electroluminescent devices manufactured as above, electroluminescent (EL) properties were measured using M7000 manufactured by McScience Inc., and with the measurement results, T₉₀ was measured when standard luminance was 6,000 cd/m² through a lifetime measurement system (M6000) manufactured by McScience Inc..

Results of measuring driving voltage, light emission efficiency, color (EL color) and lifetime of the organic light emitting devices manufactured according to the present disclosure are as shown in the following Table 7.

**[Table 7]**

| | Light Emitting Layer Compound | Driving Voltage (V) | Efficiency (cd/A) | Color EL Color | Lifetime (T₉₀) |
|---|---|---|---|---|---|
| Example 1 | 1-1 | 4.38 | 76.4 | | 230 |
| Example 2 | 1-5 | 4.66 | 71.2 | | 251 |
| Example 3 | 1-9 | 4.66 | 71.1 | | 352 |
| Example 4 | 1-40 | 4.66 | 71.2 | | 360 |
| Example 5 | 1-78 | 4.67 | 71.2 | | 356 |
| Example 6 | 1-79 | 4.67 | 71.2 | | 277 |
| Example 7 | 1-80 | 4.66 | 71.1 | | 271 |
| Example 8 | 1-81 | 4.66 | 71.1 | | 347 |
| Example 9 | 1-88 | 4.32 | 71.5 | | 289 |
| Example 10 | 1-93 | 4.42 | 73.2 | | 277 |
| Example 11 | 1-115 | 4.31 | 79.2 | | 261 |
| Example 12 | 1-117 | 4.69 | 66.2 | | 271 |
| Example 13 | 1-123 | 4.36 | 78.9 | | 256 |
| Example 14 | 1-177 | 4.45 | 72.8 | | 370 |
| Example 15 | 1-208 | 4.35 | 79.2 | Green | 382 |
| Example 16 | 1-211 | 4.31 | 79.2 | | 365 |
| Example 17 | 1-298 | 4.33 | 74.2 | | 405 |
| Example 18 | 1-301 | 4.67 | 71.2 | | 390 |
| Comparative Example 1 | Ref. 1 | 5.66 | 45.8 | | 68 |
| Comparative Example 2 | Ref. 2 | 5.03 | 48.7 | | 39 |
| Comparative Example 3 | Ref. 3 | 5.47 | 41.2 | | 37 |
| Comparative Example 4 | Ref. 4 | 4.42 | 75.7 | | 137 |
| Comparative Example 5 | Ref. 5 | 5.33 | 49.2 | | 93 |
| Comparative Example 6 | Ref. 6 | 5.37 | 43.5 | | 52 |
| Comparative Example 7 | Ref. 7 | 5.82 | 42.8 | | 28 |
| Comparative Example 8 | Ref. 8 | 4.45 | 72.8 | | 98 |
| Comparative Example 9 | Ref. 9 | 5.14 | 48.9 | | 57 |
| Comparative Example 10 | Ref. 10 | 5.34 | 48.9 | | 36 |
| Comparative Example 11 | Ref. 11 | 4.41 | 75.8 | | 149 |
| Comparative Example 12 | Ref. 12 | 4.33 | 75.2 | | 146 |
| Comparative Example 13 | Ref. 13 | 5.28 | 46.2 | | 39 |
| Comparative Example 14 | Ref. 14 | 5.12 | 45.6 | | 54 |
| Comparative Example 15 | Ref. 15 | 4.36 | 78.9 | | 139 |
| Comparative Example 16 | Ref. 16 | 4.13 | 79.2 | | 142 |
| Comparative Example 17 | Ref. 17 | 5.64 | 43.9 | | 41 |
| Comparative Example 18 | Ref. 18 | 5.51 | 43.3 | | 41 |
| Comparative Example 19 | Ref. 19 | 4.67 | 71.2 | | 101 |

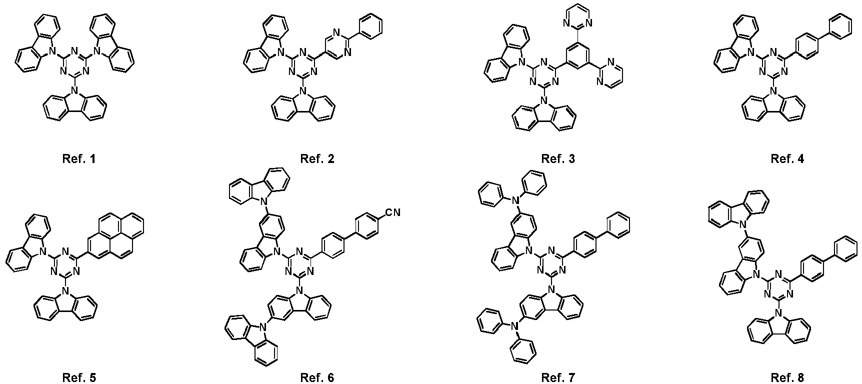

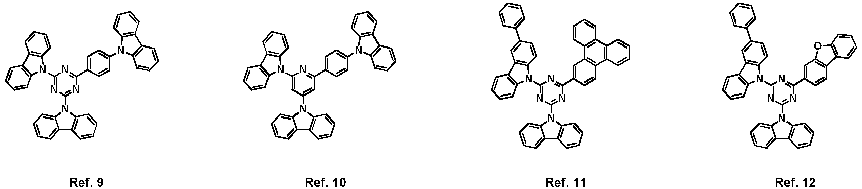

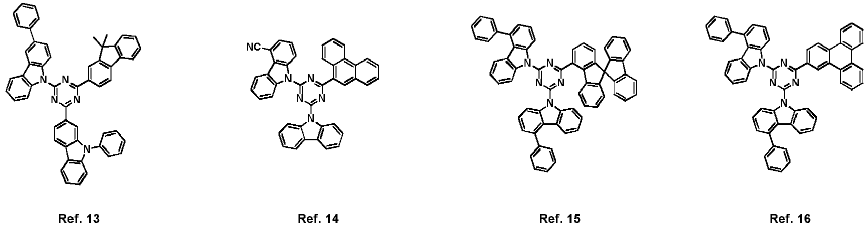

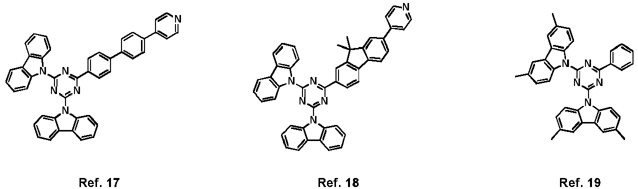

As seen from the results from Table 7, the organic electroluminescent device using the organic electroluminescent device light emitting layer material of the present disclosure had lower driving voltage, enhanced light emission efficiency and significantly improved lifetime compared to Comparative Examples 1 to 18.

Specifically, in the following Table 8, the LUMO orbital of Compound 1-298 (Example 17) was delocalized to the triazine and the terphenyl group substituent. However, it was identified that, when a triazine group did not have a sufficiently long aryl substituent as in the compounds of Ref. 4, 7, 8, 11, 14, 16 and 19, the LUMO orbital of the triazine was localized to the short aryl substituent failing to effectively stabilize electrons and thereby reducing a lifetime.

**[Table 8]**

| Compound | LUMO |
|---|---|
| 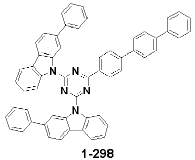 | 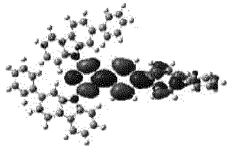 |
| 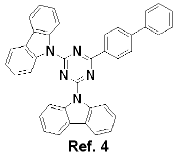 | 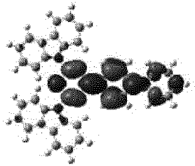 |
| 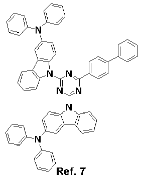 | 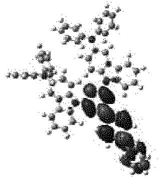 |
| 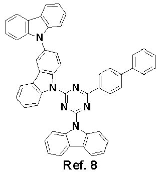 | 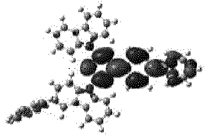 |
| 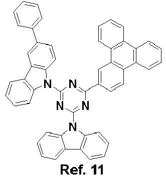 | 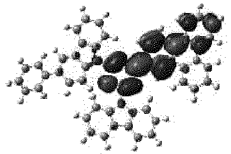 |
| 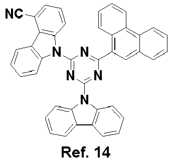 | 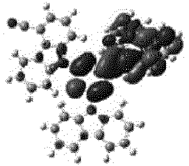 |
| 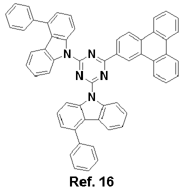 | 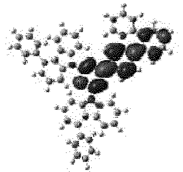 |
| 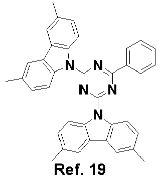 | 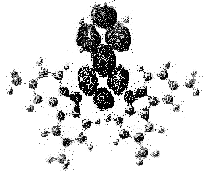 |

In the following Table 9, it was identified that, when a triazine group was all substituted with carbazole as in the compounds of Ref. 1, 9 and 10, the LUMO orbital of the triazine was delocalized to the carbazole. It was identified that the triazine having electron-withdrawing properties effectively stabilized electrons, however, the carbazole having electron-pushing properties failed to effectively stabilize electrons and thereby reduced a lifetime.

**[Table 9]**

| Compound | LUMO |
|---|---|
| 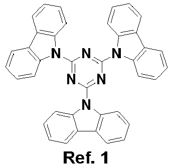 | 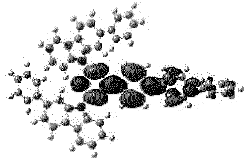 |
| 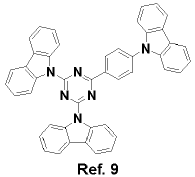 | 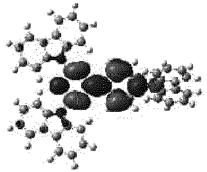 |
| 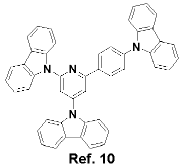 | 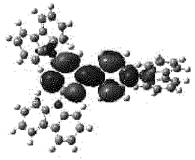 |

It was identified that, when a triazine group was substituted with a pyrimidine group, a cyano group or a pyridine group as in the compounds of Ref. 2, 3, 6, 17 and 18, electron mobility excessively increased and a balance between holes and electrons was broken in the light emitting layer, and as a result, the lifetime decreased.

When Nos. 3 and 4 positions of carbazole were substituted with a phenyl group as in the compounds of Ref. 11, 12, 13, 15 and 16, the sublimation temperature increased by having a larger molecular weight compared to unsubstituted carbazole, and a relatively planar structure was formed by having a smaller structural distortion compared to carbazole having a phenyl group substituted at a No. 2 position and the sublimation temperature increased. It was identified that the organic compound was deteriorated and the lifetime decreased when exposed for a long period of time at a high sublimation temperature.

It was identified that, when carbazole was substituted with amine or carbazole as in the compounds of Ref. 6, 7 and 8, hole mobility excessively increased and a balance between holes and electrons was broken in the light emitting layer, and as a result, the lifetime decreased.

In the compound of Ref. 10, pyridine bonds instead of triazine. It was identified that this decreased electron mobility and a balance between holes and electrons was broken in the light emitting layer, and as a result, the lifetime decreased.

In the following Table 10, the compound of Ref. 5 has a pyrene group directly bonding to triazine. It was identified that the pyrene substituted with a substituent absorbed light energy (approximately 2.4 eV) emitting from the green dopant again due to a low T₁ level, and the device had decreased efficiency and lifetime. Pyrene having a low T₁ level is mainly used as a dopant of a fluorescent OLED device instead of the phosphorescent OLED device, and has a principle of driving a device by preventing T₁ energy transfer and aiming S₁ energy transfer.

**[Table 10]**

| Compound | T₁ Level | S₁ Level |
|---|---|---|
| 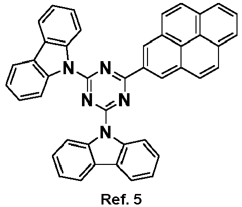 | 2.12 | 3.11 |
| 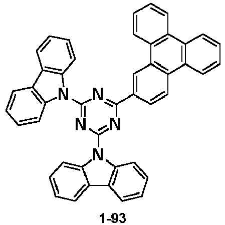 | 2.68 | 3.35 |

### <Experimental Example 2>

### - Manufacture of Organic Light Emitting Device

A glass substrate on which indium tin oxide (ITO) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and ultraviolet ozone (UVO) treatment was conducted for 5 minutes using UV in an ultraviolet (UV) cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4',4"-tris[2-naphthyl(phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. As the light emitting layer, one type of the compound of Chemical Formula 1 and one type of the compound of Chemical Formula 2 as described in the following Table 11 were pre-mixed in a weight ratio described in the following Table 11 and deposited to 400 Å in one source of supply as a host, and a green phosphorescent dopant [Ir(ppy)₃] was doped and deposited by an amount of 7% with respect to the deposited thickness of the light emitting layer. After that, BCP (bathocuproine) was deposited to 60 Å as a hole blocking layer, and Alq₃ was deposited to 200 Å thereon as an electron transfer layer. Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic electroluminescent device was manufactured.

Meanwhile, all the organic compounds required to manufacture the OLED were vacuum sublimation purified under 10⁻⁸ torr to 10⁻⁶ torr for each material to be used in the OLED manufacture.

For each of the organic electroluminescent devices manufactured as above, electroluminescent (EL) properties were measured using M7000 manufactured by McScience Inc., and with the measurement results, T₉₀ was measured when standard luminance was 6,000 cd/m² through a lifetime measurement system (M6000) manufactured by McScience Inc..

Results of measuring driving voltage, light emission efficiency, color coordinate (CIE) and lifetime of the organic light emitting devices manufactured according to the present disclosure are as shown in the following Table 11.

**[Table 11]**

| | Light Emitting Layer Compound | Ratio | Driving Voltage (V) | Efficien cy (cd/A) | Color EL Color | Lifetime (T₉₀) |
|---|---|---|---|---|---|---|
| Example 19 | 1-40: 2-2 | 1:8 | 4.73 | 54.2 | Green | 338 |
| Example 20 | | 1:5 | 4.71 | 57.2 | | 444 |
| Example 21 | | 1:2 | 4.33 | 75.2 | | 569 |
| Example 22 | | 1:1 | 4.48 | 70.2 | | 543 |
| Example 23 | | 2:1 | 4.69 | 69.2 | | 518 |
| Example 24 | | 5:1 | 4.32 | 68.3 | | 433 |
| Example 25 | | 8:1 | 4.21 | 67.0 | | 325 |
| Example 26 | 1-78: 2-3 | 1:2 | 4.38 | 76.4 | | 539 |
| Example 27 | | 1:1 | 4.45 | 72.8 | | 522 |
| Example 28 | | 2:1 | 4.66 | 71.1 | | 500 |
| Example 29 | 1-177: 2-12 | 1:2 | 4.31 | 79.2 | | 586 |
| Example 30 | | 1:1 | 4.42 | 75.7 | | 558 |
| Example 31 | | 2:1 | 4.66 | 71.1 | | 536 |
| Example 32 | 1-208: 3-4 | 1:2 | 4.35 | 79.2 | | 599 |
| Example 33 | | 1:1 | 4.41 | 75.8 | | 591 |
| Example 34 | | 2:1 | 4.67 | 71.2 | | 540 |
| Example 35 | 1-211: 3-7 | 1:2 | 4.33 | 74.2 | | 562 |
| Example 36 | | 1:1 | 4.42 | 72.2 | | 553 |
| Example 37 | | 2:1 | 4.66 | 71.2 | | 528 |
| Example 38 | 1-298: 3-31 | 1:2 | 4.33 | 75.2 | | 679 |
| Example 39 | | 1:1 | 4.48 | 70.2 | | 546 |
| Example 40 | | 2:1 | 4.69 | 69.2 | | 525 |
| Example 41 | 1-301: 3-32 | 1:2 | 4.33 | 75.2 | | 620 |
| Example 42 | | 1:1 | 4.48 | 70.2 | | 582 |
| Example 43 | | 2:1 | 4.69 | 69.2 | | 543 |

Based on the results of Table 11, effects of more superior efficiency and lifetime were obtained when including the compound of Chemical Formula 1 (n type) and the compound of Chemical Formula 2 at the same time. Such a result may lead to a forecast that an exciplex phenomenon occurs when including the two compounds at the same time.

The exciplex phenomenon is a phenomenon of releasing energy having sizes of a donor (p-host) HOMO level and an acceptor (n-host) LUMO level due to electron exchanges between two molecules. When the exciplex phenomenon occurs between two molecules, reverse intersystem crossing (RISC) occurs, and as a result, internal quantum efficiency of fluorescence may increase up to 100%. When a donor (p-host) having a favorable hole transfer ability and an acceptor (n-host) having a favorable electron transfer ability are used as a host of a light emitting layer, holes are injected to the p-host and electrons are injected to the n-host, and therefore, a driving voltage may be lowered, which resultantly helps with enhancement in the lifetime. In the disclosure of the present application, it was identified that the compound of Chemical Formula 2 performed a donor role and the compound of Chemical Formula 1 performed an acceptor role, and superior device properties were obtained when they were used together as a host of the light emitting layer.

## Claims

1. A heterocyclic compound represented by the following Chemical Formula 1: 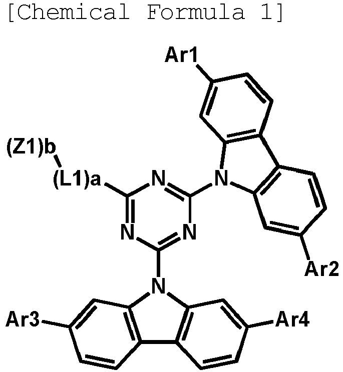 wherein, in Chemical Formula 1,
Ar1 to Ar4 are the same as or different from each other, and each independently hydrogen; deuterium; a substituted or unsubstituted C1 to C60 alkyl group; a substituted or unsubstituted C6 to C60 aryl group; or a substituted or unsubstituted C2 to C60 heteroaryl group;
L1 is a direct bond; a substituted or unsubstituted C6 to C60 arylene group; or a substituted or unsubstituted C2 to C60 heteroarylene group;
Z1 is selected from the group consisting of halogen; -CN; a substituted or unsubstituted C1 to C60 alkyl group; a substituted or unsubstituted C10 to C60 aryl group; a C2 to C60 heteroaryl group substituted or unsubstituted and including O or S as a heteroatom; -P(=O)RR'; -SiRR'R"; and a substituted or unsubstituted amine group;
when Z1 is a dicyclic or tetracyclic C15 to C60 aryl group substituted or unsubstituted and having the number of protons of 9 or less, L1 is a substituted or unsubstituted C6 to C60 arylene group; or a substituted or unsubstituted C2 to C60 heteroarylene group;
R, R' and R" are the same as or different from each other, and each independently a substituted or unsubstituted C1 to C60 alkyl group; a substituted or unsubstituted C6 to C60 aryl group; or a substituted or unsubstituted C2 to C60 heteroaryl group;
a is an integer of 1 to 4;
b is an integer of 1 to 6; and
when a and b are each 2 or greater, substituents in each parenthesis are the same as or different from each other.

2. The heterocyclic compound of Claim 1, wherein Chemical Formula 1 is represented by any one of the following Chemical Formula 3 to Chemical Formula 8: 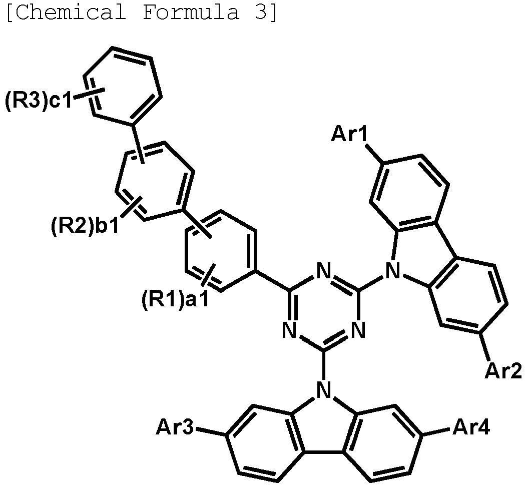 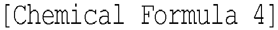 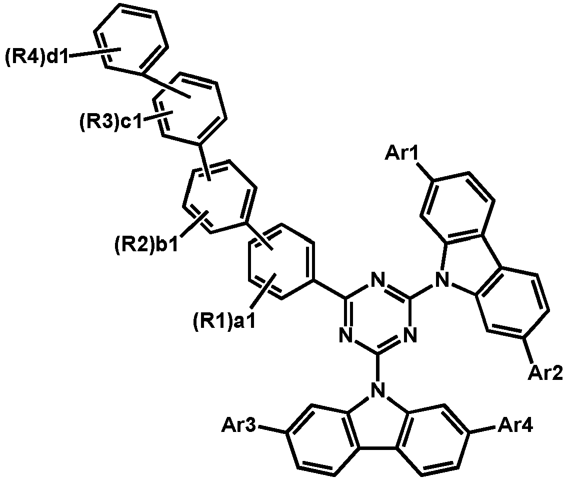 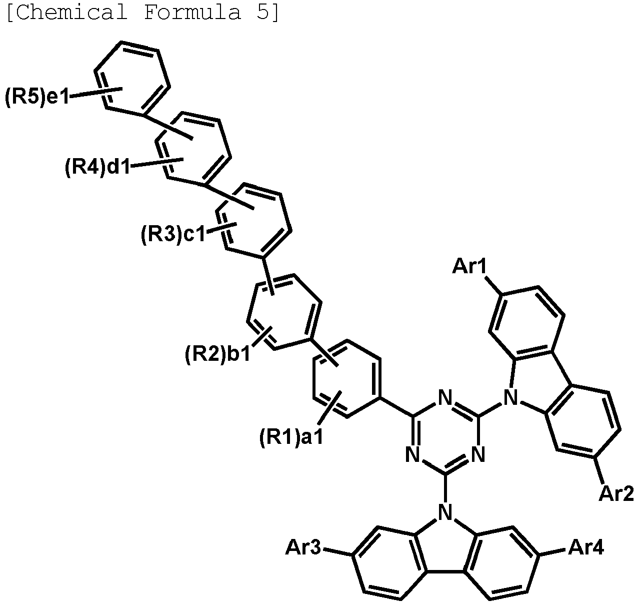 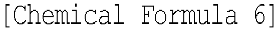 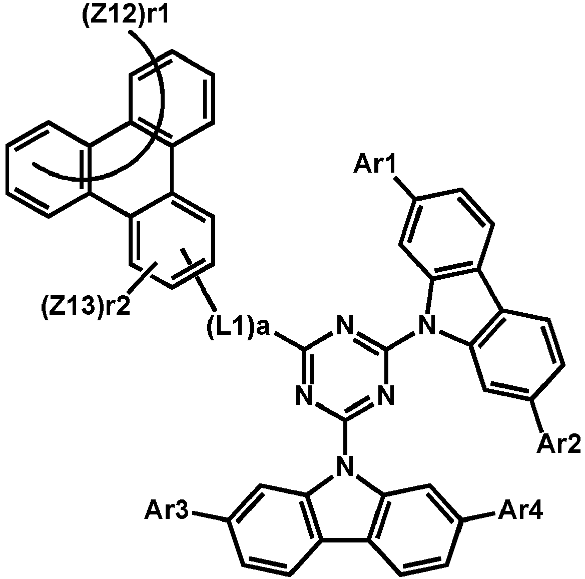 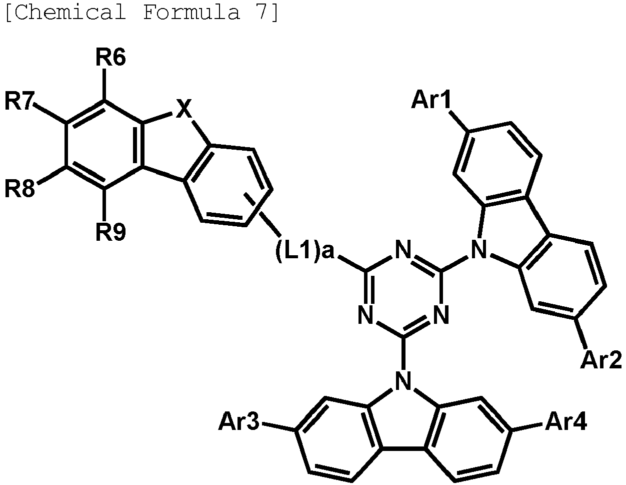 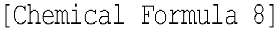 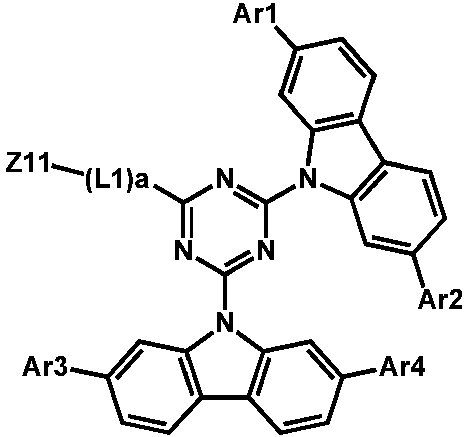 in Chemical Formulae 3 to 8,
Ar1 to Ar4, L1 and a have the same definitions as in Chemical Formula 1;
X is O; S; or C(R21)(R22);
R1 to R5 are the same as or different from each other, and each independently hydrogen; a substituted or unsubstituted C1 to C60 alkyl group; a substituted or unsubstituted C6 to C60 aryl group; or a substituted or unsubstituted C2 to C60 heteroaryl group;
R6 to R9 are the same as or different from each other, and each independently hydrogen; deuterium; a halogen group; - CN; a substituted or unsubstituted C1 to C60 alkyl group; a substituted or unsubstituted C6 to C60 aryl group; or a C2 to C60 heteroaryl group substituted or unsubstituted and including O or S as a heteroatom;
Z11 is -P(=O)RR'; -SiRR'R"; or a substituted or unsubstituted amine group;
Z12 and Z13 are the same as or different from each other, and each independently a substituted or unsubstituted C1 to C60 alkyl group; a substituted or unsubstituted C6 to C60 aryl group; or a C2 to C60 heteroaryl group substituted or unsubstituted and including O or S as a heteroatom;
R, R' and R" have the same definitions as in Chemical Formula 1;
R21 and R22 are the same as or different from each other, and each independently a substituted or unsubstituted C1 to C60 alkyl group; a substituted or unsubstituted C6 to C60 aryl group; or a substituted or unsubstituted C2 to C60 heteroaryl group, or two or more groups adjacent to each other bond to each other to form a substituted or unsubstituted C6 to C60 aromatic hydrocarbon ring or a substituted or unsubstituted C2 to C60 heteroring;
a1 to d1 are each an integer of 1 to 4;
e1 is an integer of 1 to 5;
when a1 to e1 are each 2 or greater, substituents in each parenthesis are the same as or different from each other;
r1 and r2 are each an integer of 0 or 1; and
r1+r2<2.

3. The heterocyclic compound of Claim 1, wherein the "substituted or unsubstituted" means being substituted with one or more substituents selected from the group consisting of deuterium; halogen; a cyano group; C1 to C60 linear or branched alkyl; C2 to C60 linear or branched alkenyl; C2 to C60 linear or branched alkynyl; C3 to C60 monocyclic or polycyclic cycloalkyl; C2 to C60 monocyclic or polycyclic heterocycloalkyl; C6 to C60 monocyclic or polycyclic aryl; C2 to C60 monocyclic or polycyclic heteroaryl; -SiRR'R"; - P(=O)RR'; C1 to C20 alkylamine; C6 to C60 monocyclic or polycyclic arylamine; and C2 to C60 monocyclic or polycyclic heteroarylamine, or being unsubstituted, or being substituted with a substituent linking two or more substituents selected from among the substituents illustrated above, or being unsubstituted; and
R, R' and R" have the same definitions as in Chemical Formula 1.

4. The heterocyclic compound of Claim 1, wherein Chemical Formula 1 is represented by any one of the following Chemical Formula 9 to Chemical Formula 14: 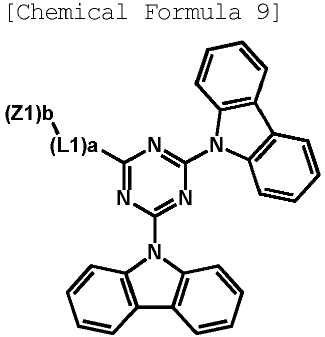 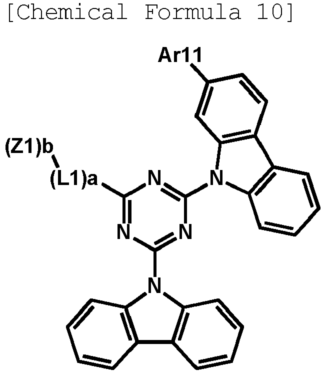 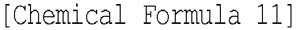 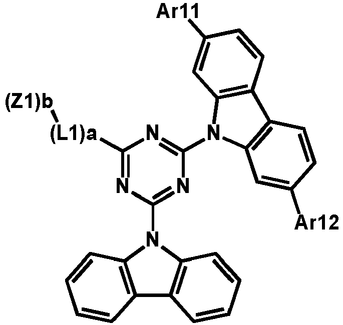 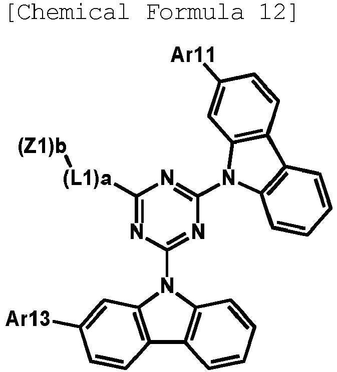 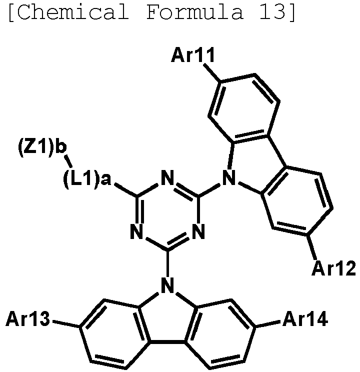 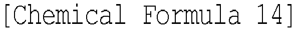 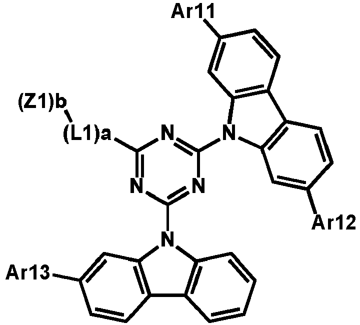 in Chemical Formulae 9 to 14,
L1, Z1, a and b have the same definitions as in Chemical Formula 1; and
Ar11 to Ar14 are the same as or different from each other, and each independently deuterium; a substituted or unsubstituted C6 to C60 aryl group; or a substituted or unsubstituted C2 to C60 heteroaryl group.

5. The heterocyclic compound of Claim 1, wherein Chemical Formula 1 is represented by any one of the following compounds:

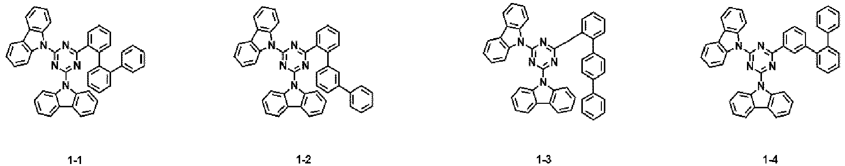

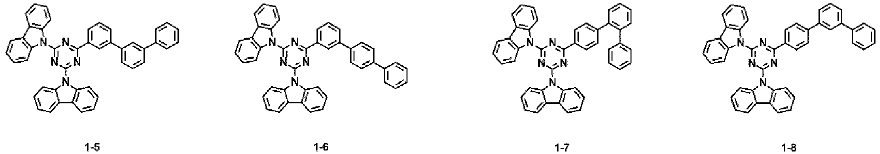

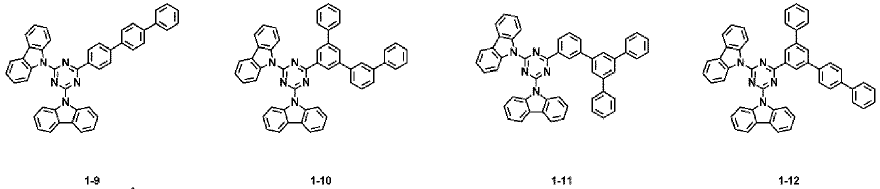

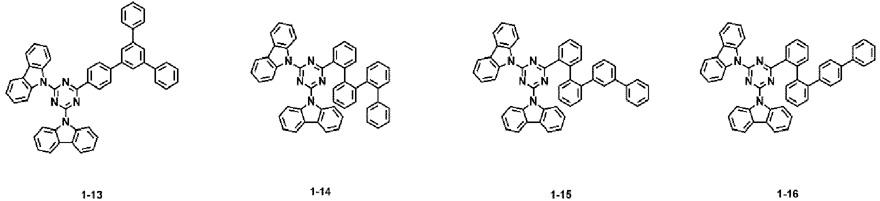

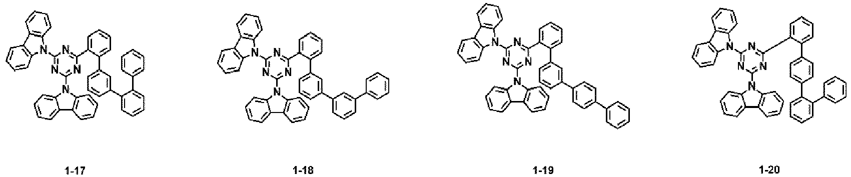

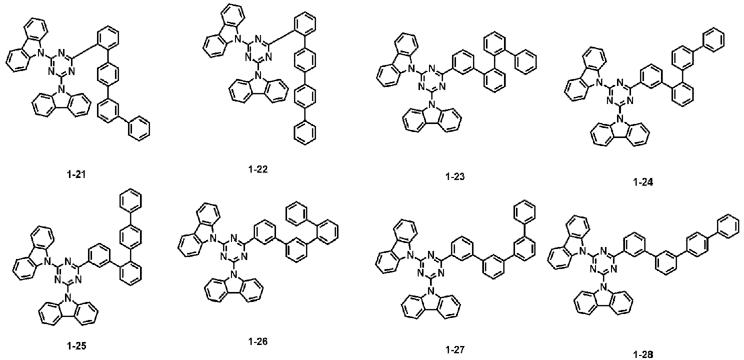

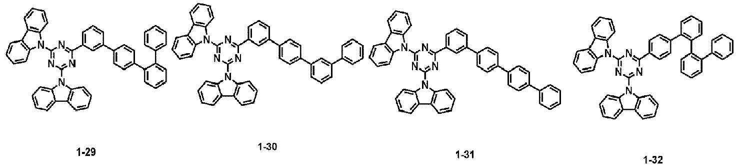

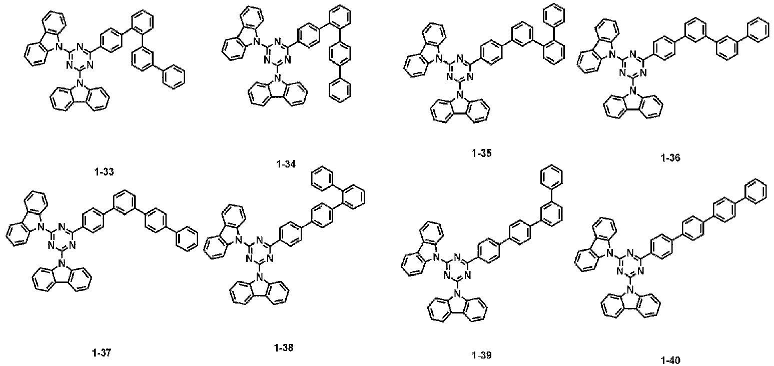

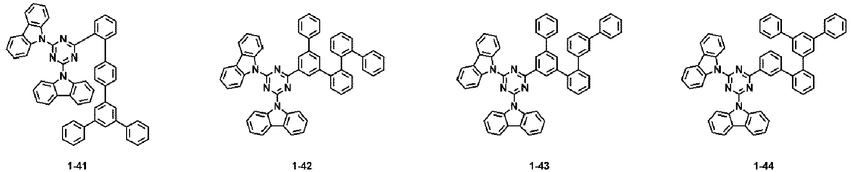

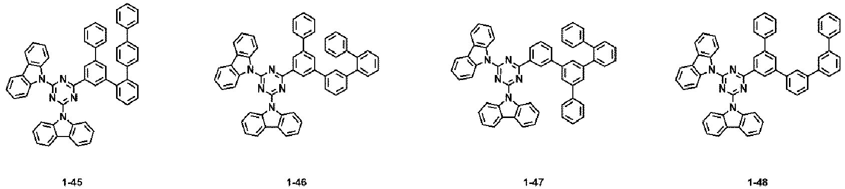

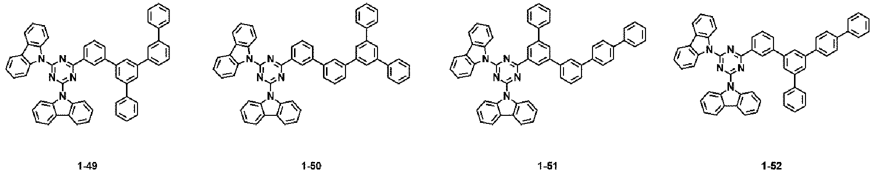

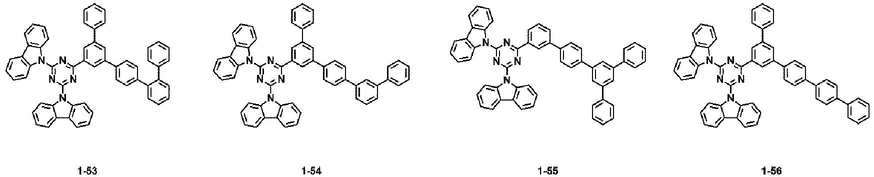

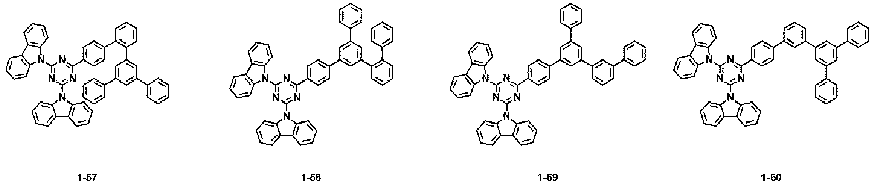

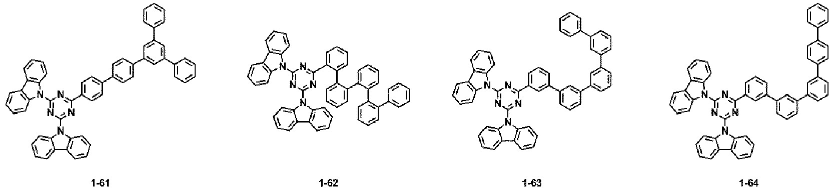

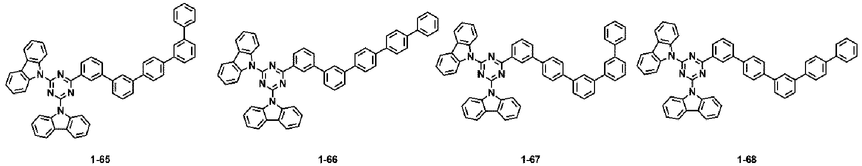

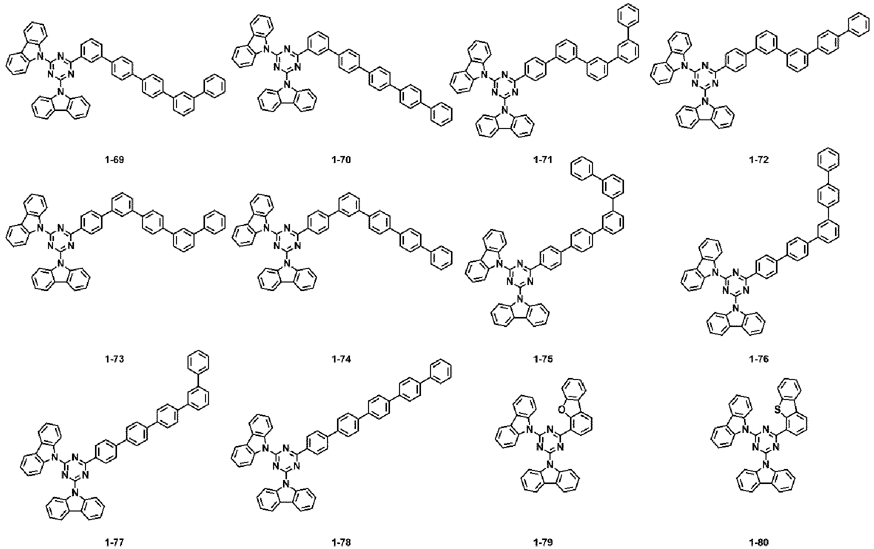

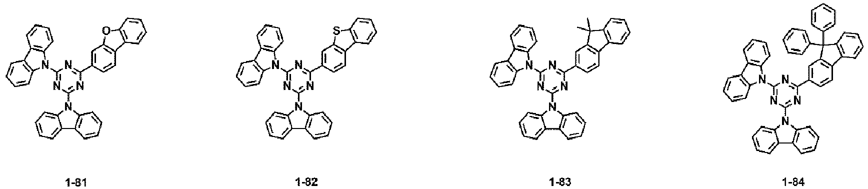

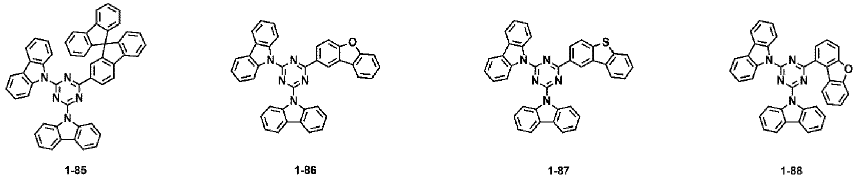

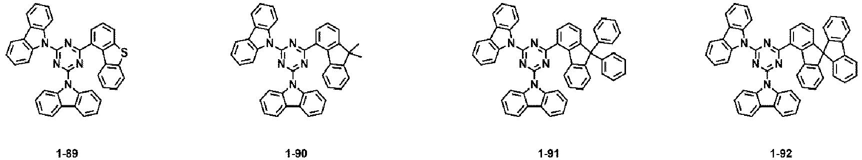

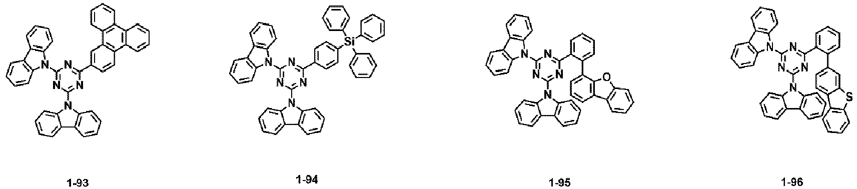

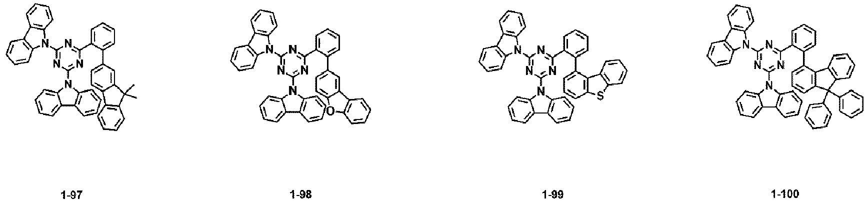

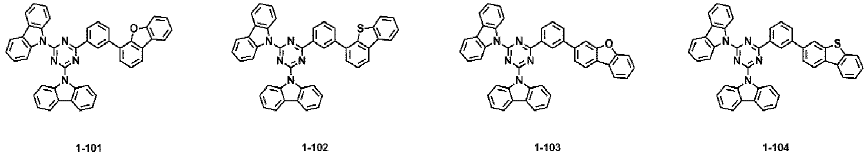

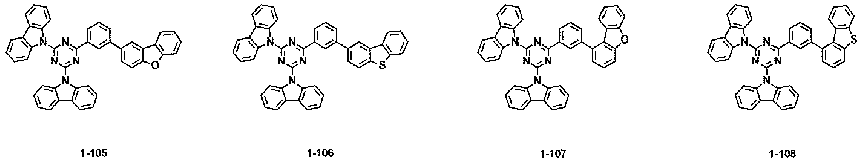

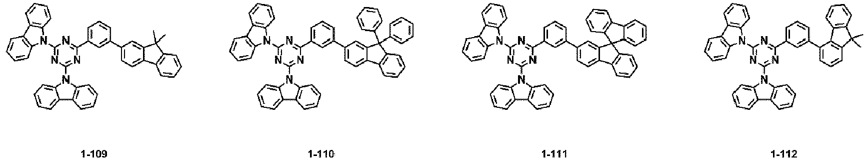

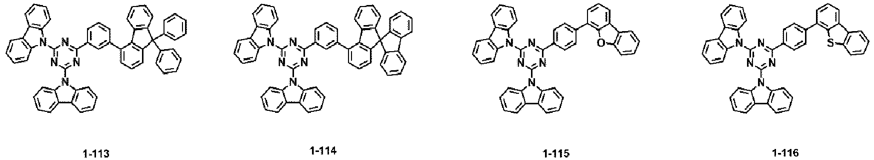

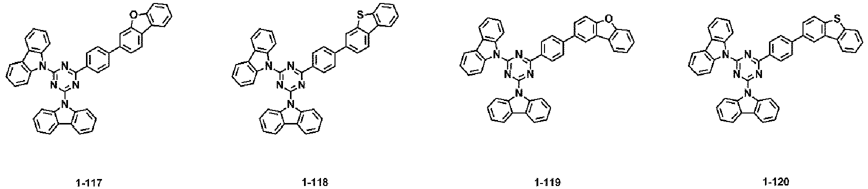

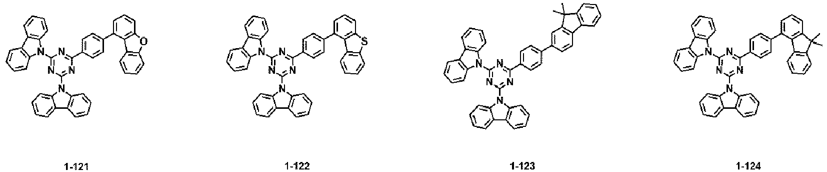

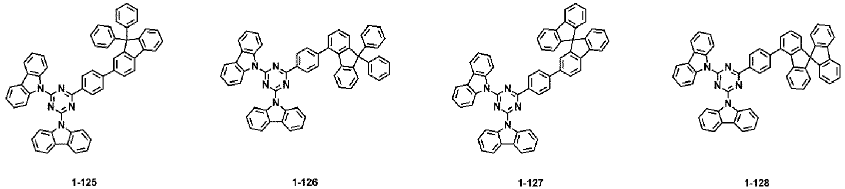

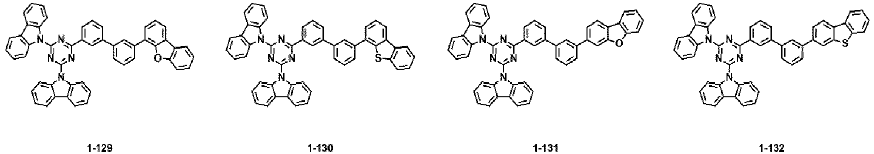

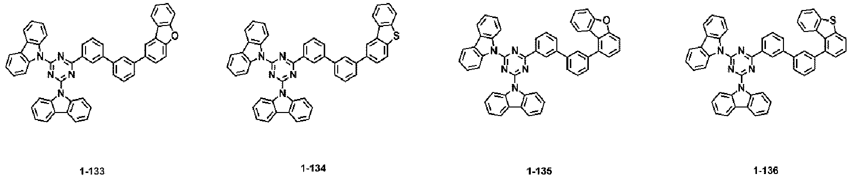

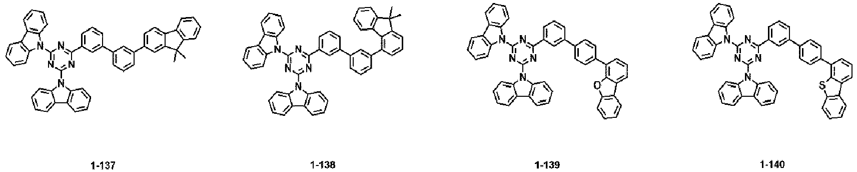

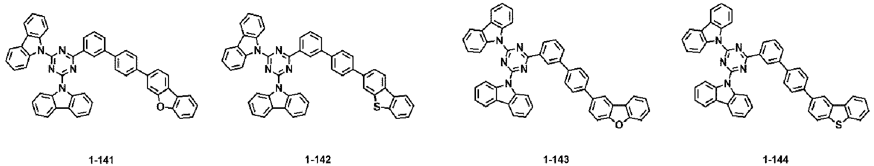

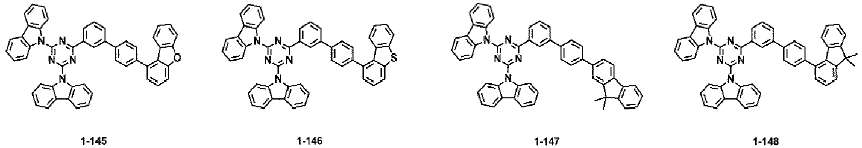

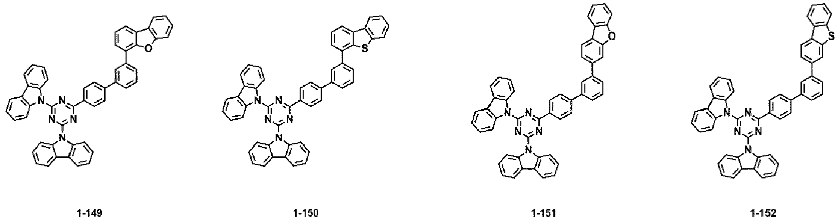

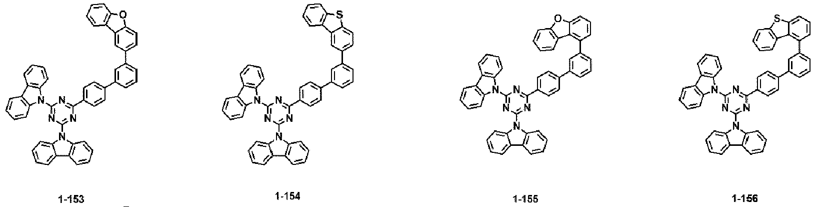

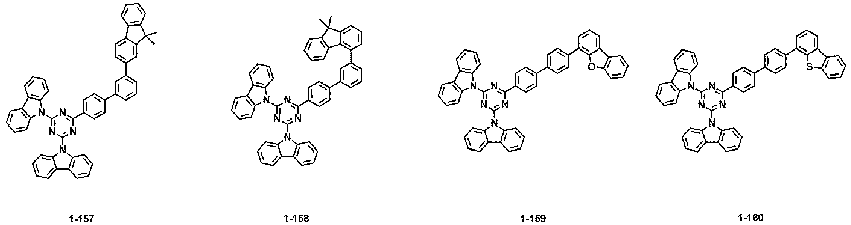

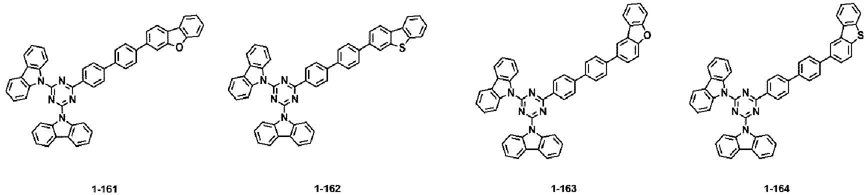

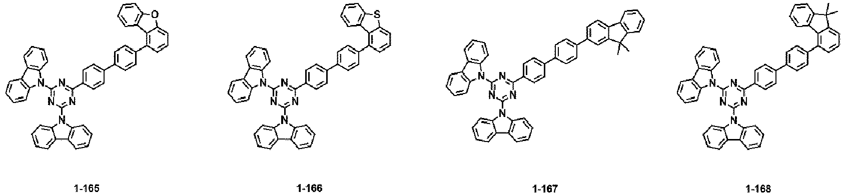

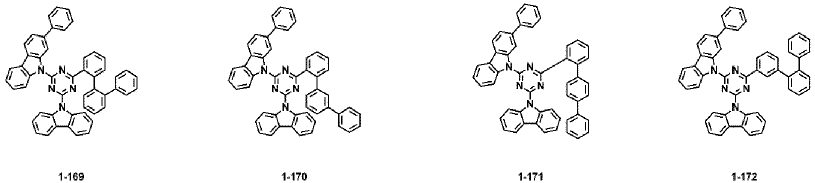

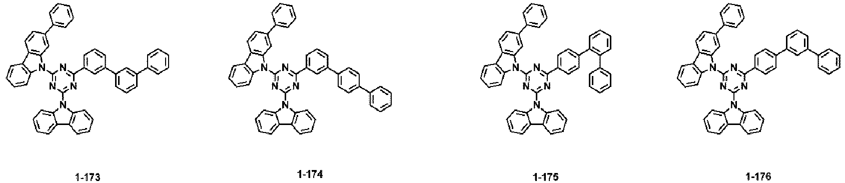

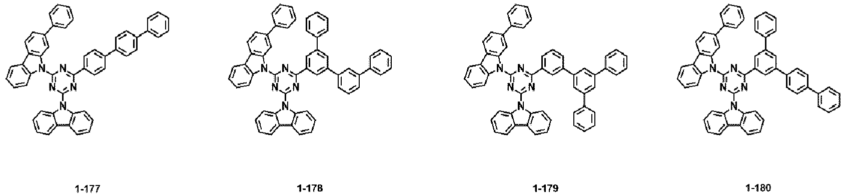

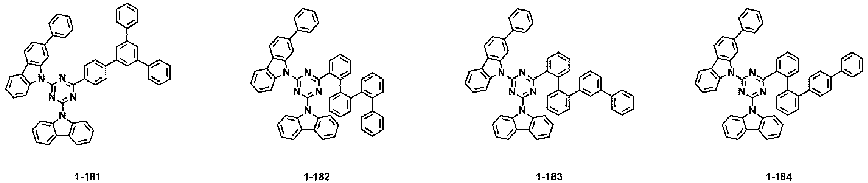

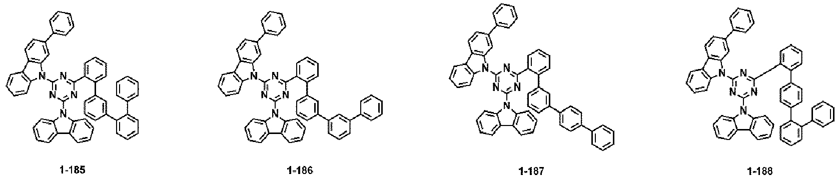

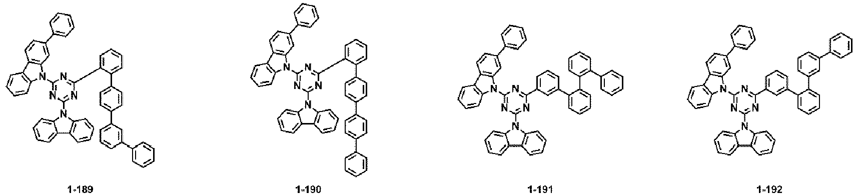

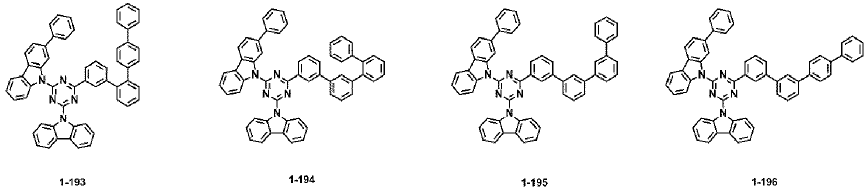

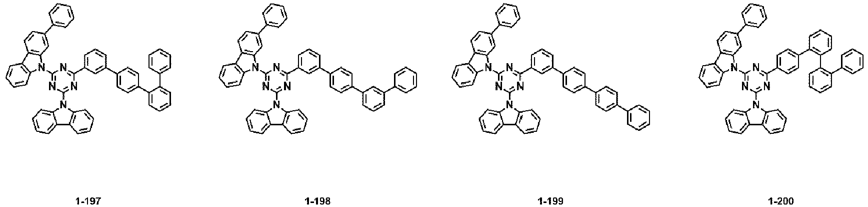

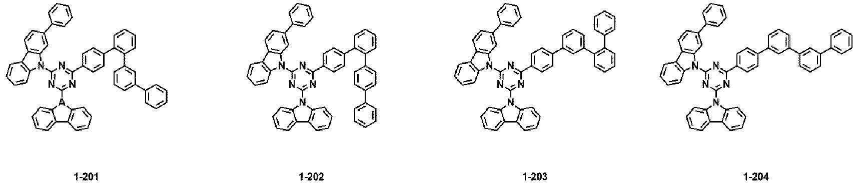

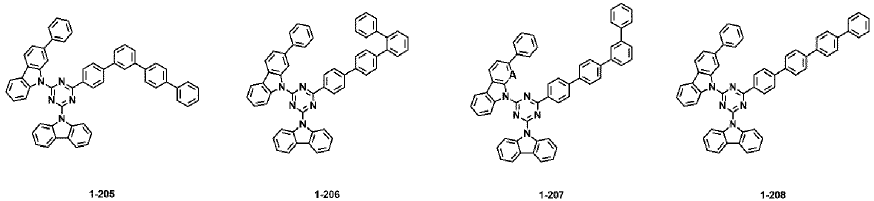

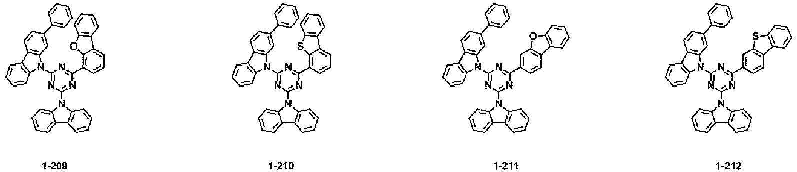

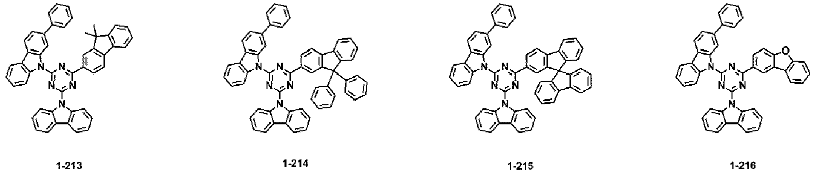

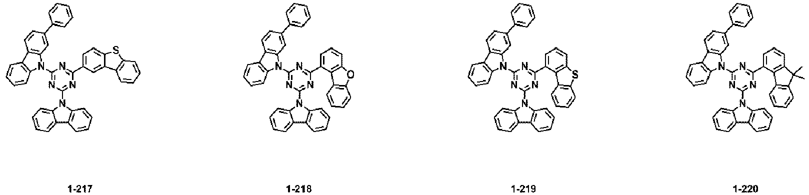

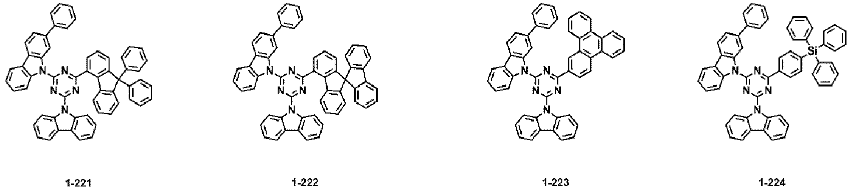

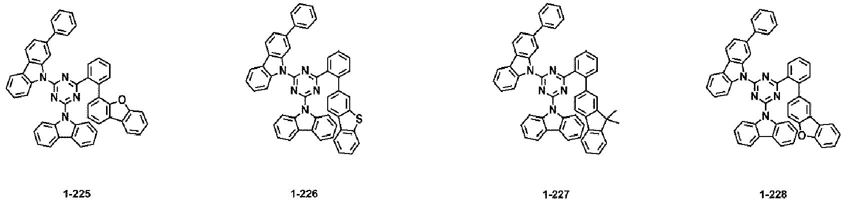

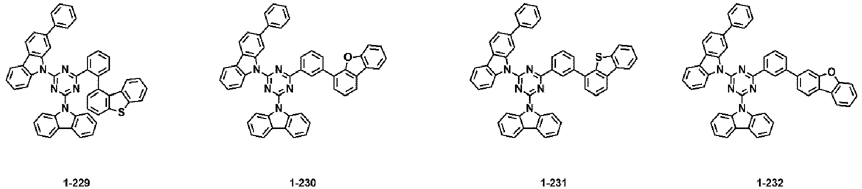

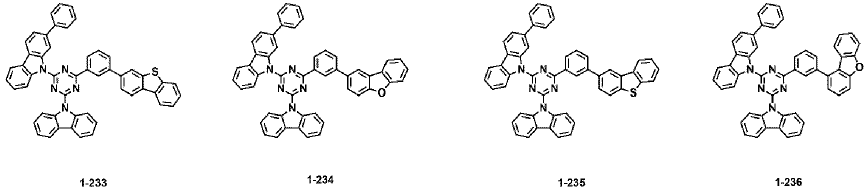

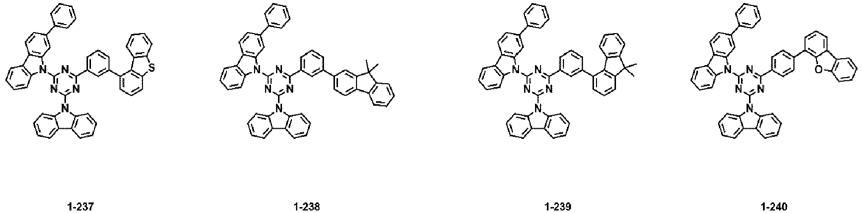

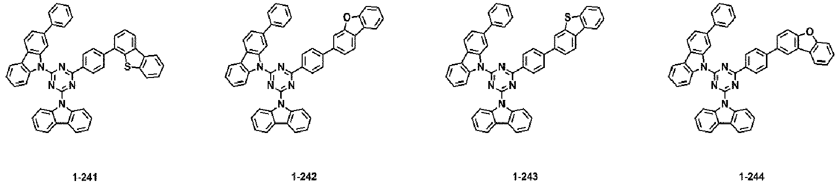

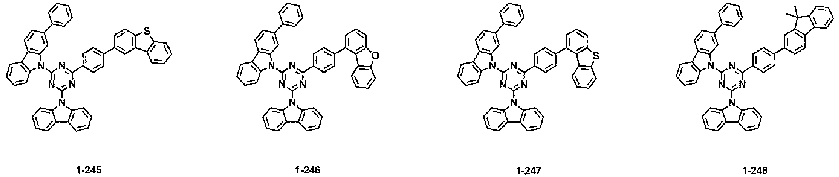

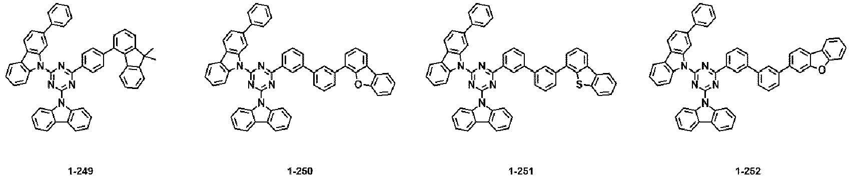

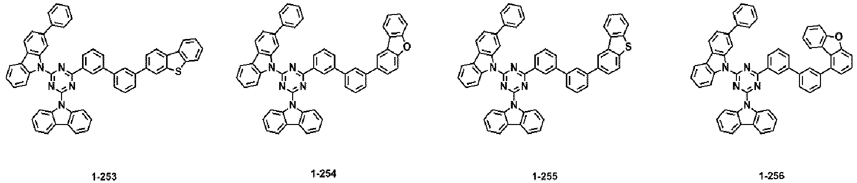

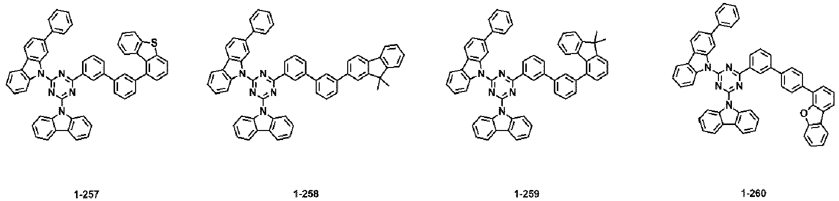

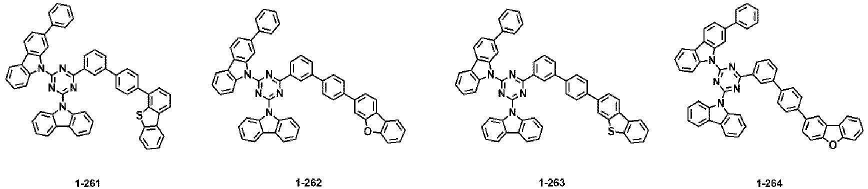

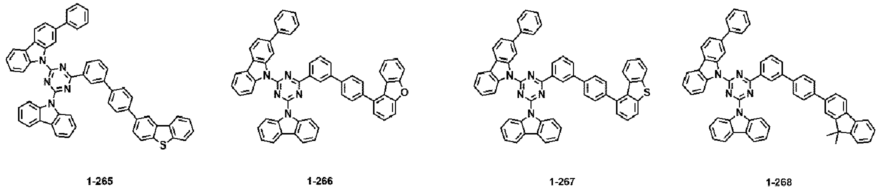

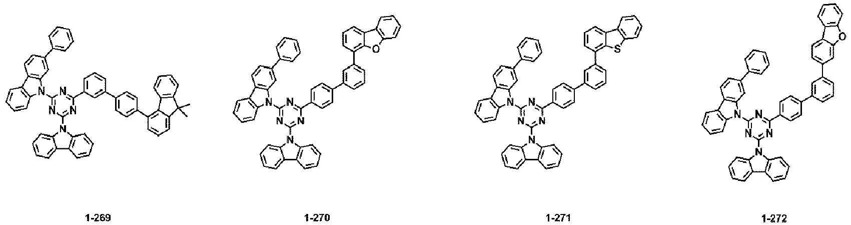

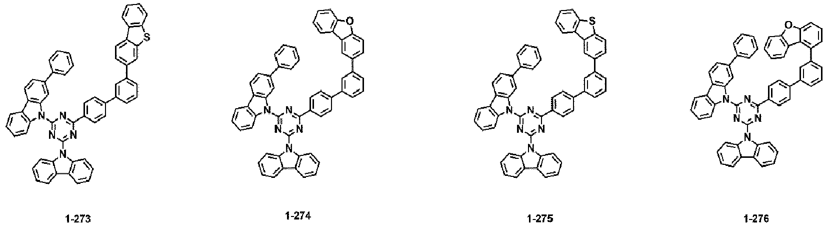

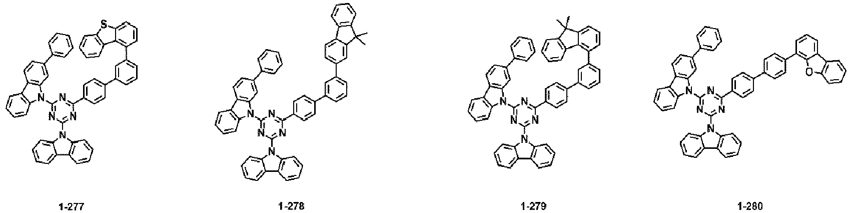

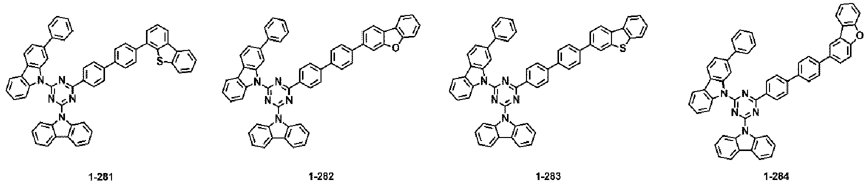

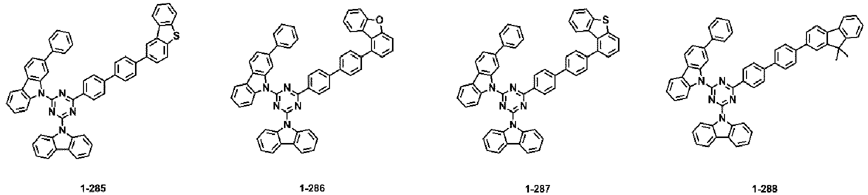

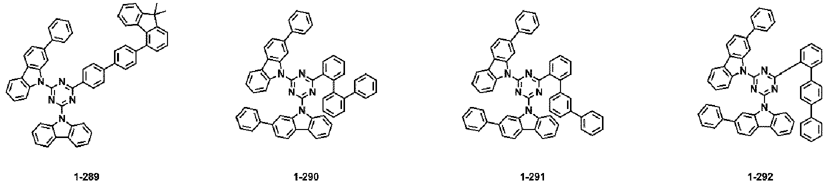

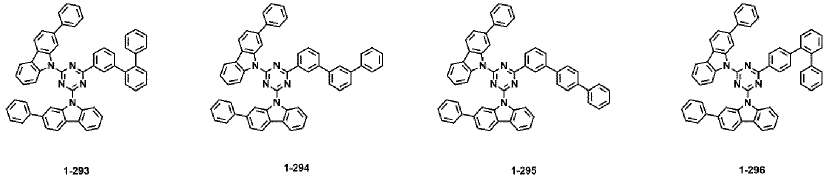

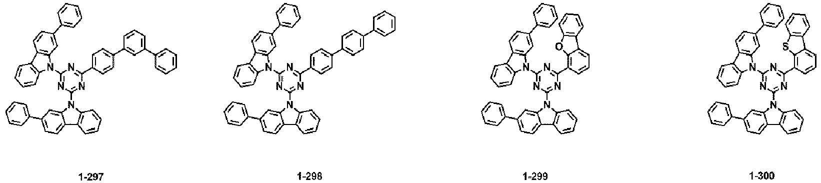

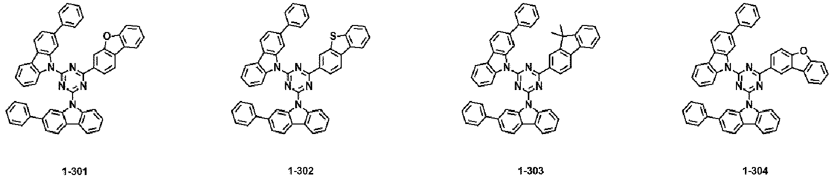

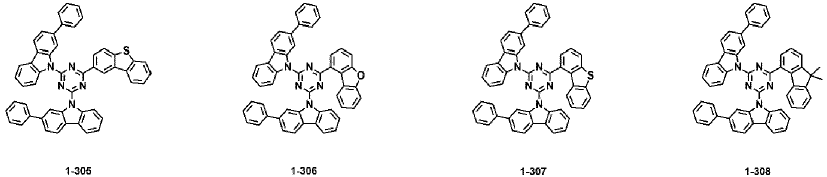

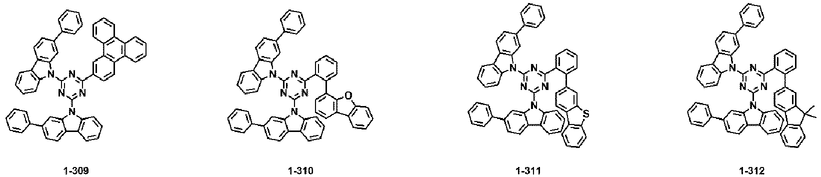

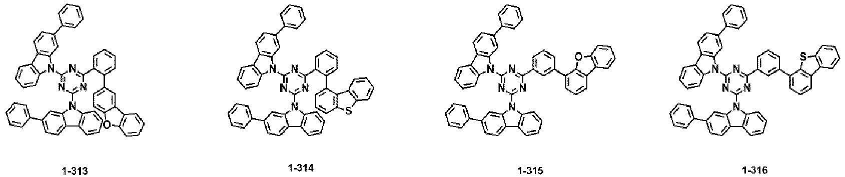

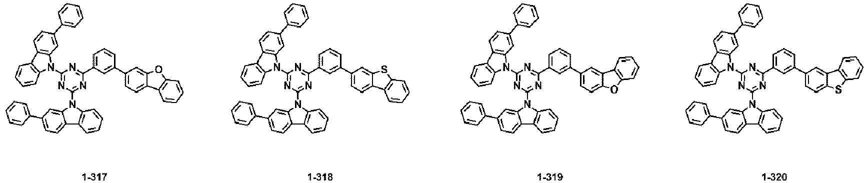

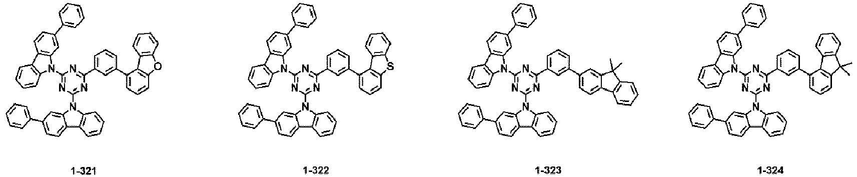

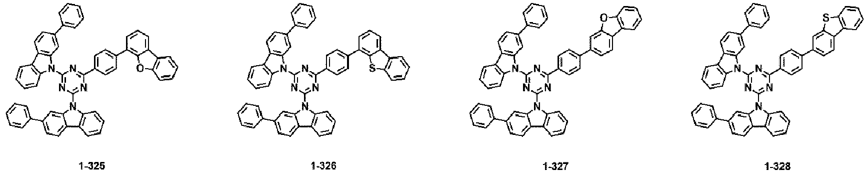

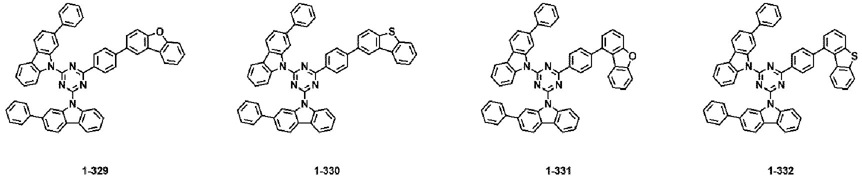

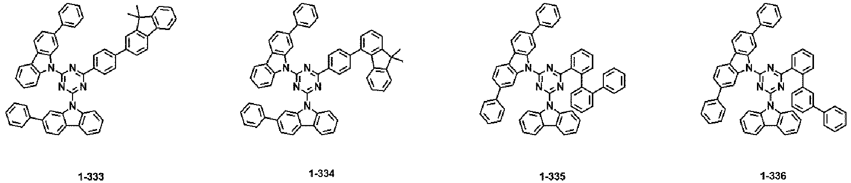

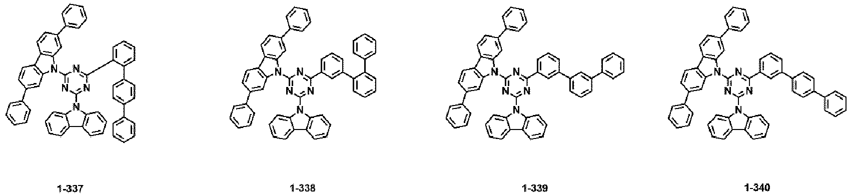

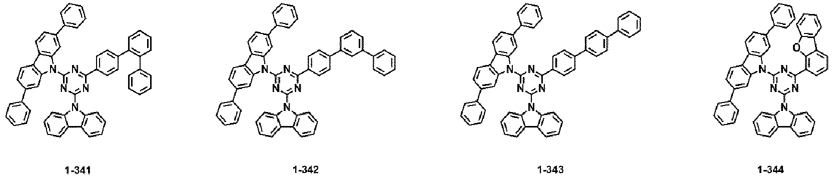

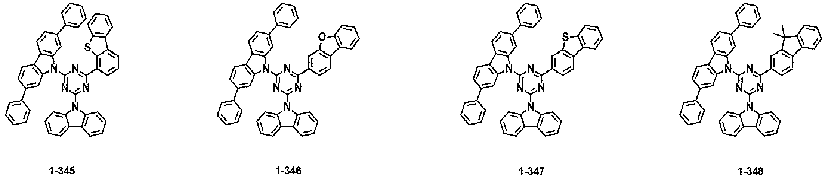

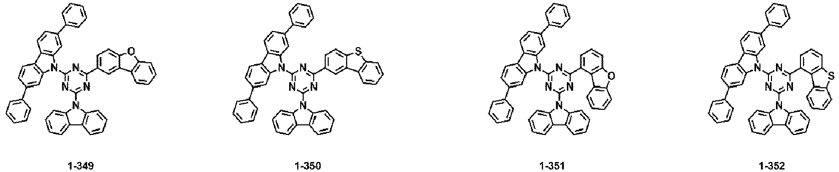

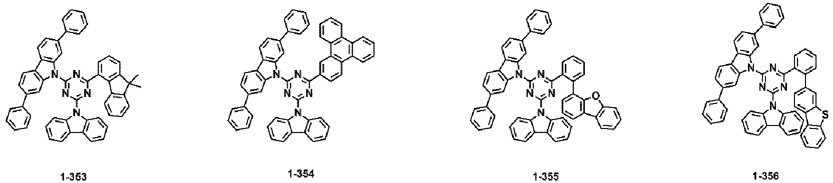

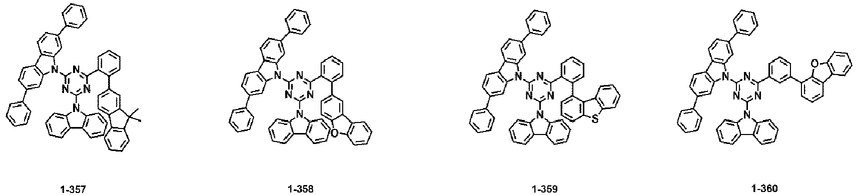

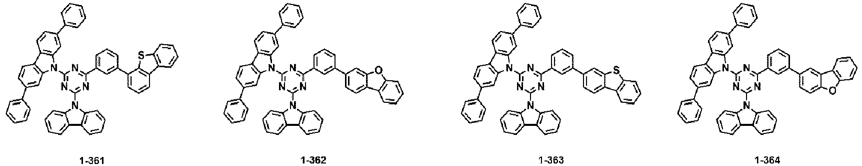

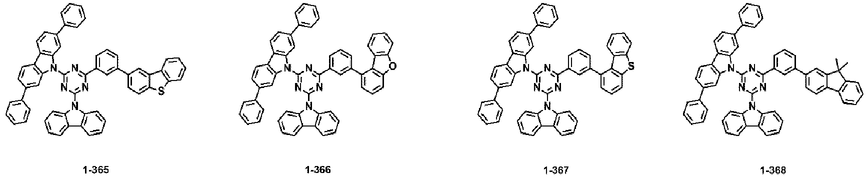

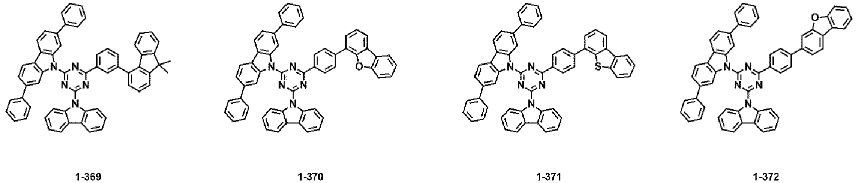

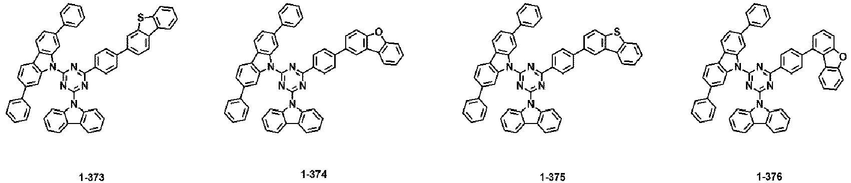

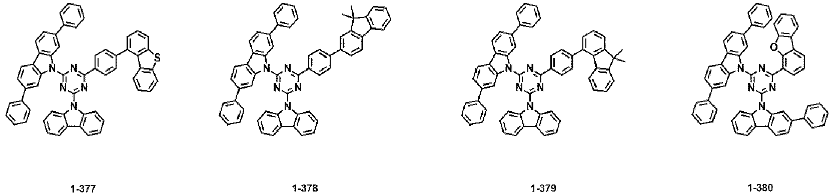

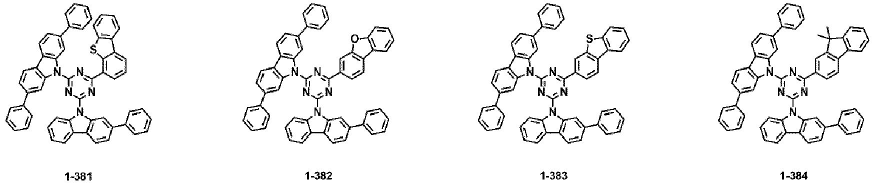

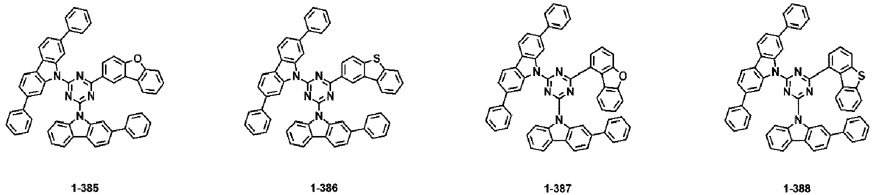

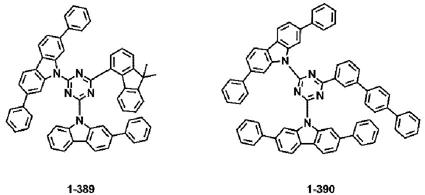

6. An organic light emitting device comprising:
a first electrode;
a second electrode provided opposite to the first electrode; and
one or more organic material layers provided between the first electrode and the second electrode,
wherein one or more layers of the organic material layers include the heterocyclic compound of any one of Claims 1 to 5.

7. The organic light emitting device of Claim 6, wherein the organic material layer including the heterocyclic compound further includes a heterocyclic compound represented by the following Chemical Formula 2: 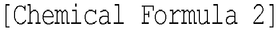 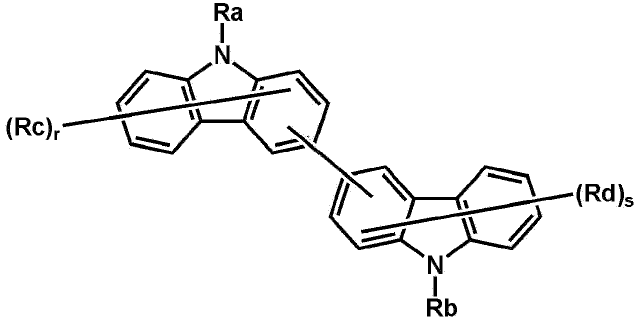 in Chemical Formula 2,
Rc and Rd are the same as or different from each other, and each independently selected from the group consisting of hydrogen; deuterium; a halogen group; -CN; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -Si(R10) (R11) (R12); - P(=O) (R10) (R11); and a substituted or unsubstituted amine group, or two or more groups adjacent to each other bond to each other to form a substituted or unsubstituted C6 to C60 aromatic hydrocarbon ring or a substituted or unsubstituted C2 to C60 heteroring;
R10, R11 and R12 are the same as or different from each other, and each independently hydrogen; deuterium; -CN; a substituted or unsubstituted C1 to C60 alkyl group; a substituted or unsubstituted C6 to C60 aryl group; or a substituted or unsubstituted C2 to C60 heteroaryl group;
Ra and Rb are the same as or different from each other, and each independently a substituted or unsubstituted C6 to C60 aryl group; or a substituted or unsubstituted C2 to C60 heteroaryl group;
r and s are each an integer of 0 to 7; and
when r and s are each 2 or greater, substituents in each parenthesis are the same as or different from each other.

8. The organic light emitting device of Claim 7, wherein Chemical Formula 2 is represented by the following Chemical Formula 2-1 or Chemical Formula 2-2: 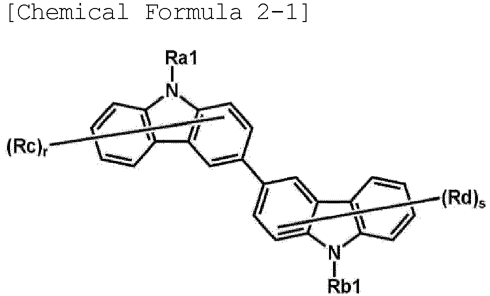 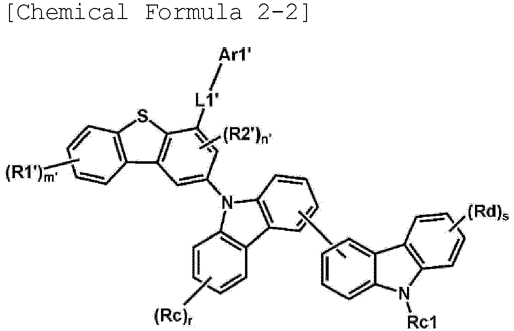 in Chemical Formulae 2-1 and 2-2,
Rc, Rd, r and s have the same definitions as in Chemical Formula 2;
Ra1, Rb1 and Rc1 are the same as or different from each other, and each independently a substituted or unsubstituted C6 to C60 aryl group;
R1' and R2' are the same as or different from each other, and each independently selected from the group consisting of hydrogen; a substituted or unsubstituted C1 to C60 alkyl group; a substituted or unsubstituted C6 to C60 aryl group; and a substituted or unsubstituted C2 to C60 heteroaryl group;
L1' is a direct bond; or a substituted or unsubstituted C6 to C60 arylene group;
Ar1' is a substituted or unsubstituted C6 to C60 aryl group; or a C2 to C60 heteroaryl group substituted or unsubstituted and including at least one of S and O;
m' is an integer of 0 to 4;
n' is an integer of 0 to 2; and
when m' is 2 or greater or n' is 2, substituents in each parenthesis are the same as or different from each other.

9. The organic light emitting device of Claim 7, wherein the heterocyclic compound represented by Chemical Formula 2 is any one selected from among the following compounds:

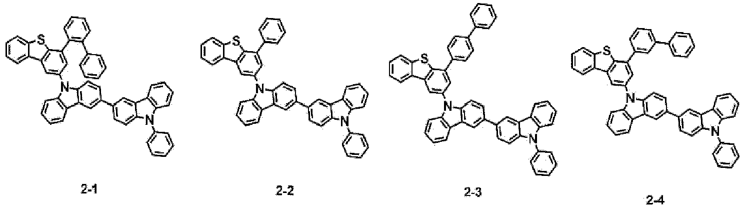

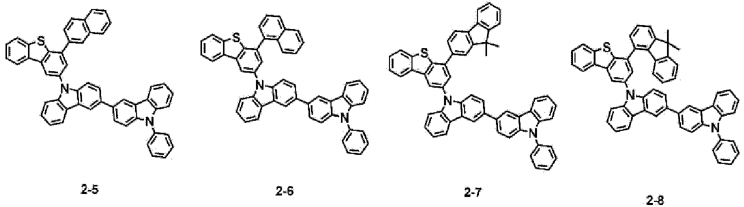

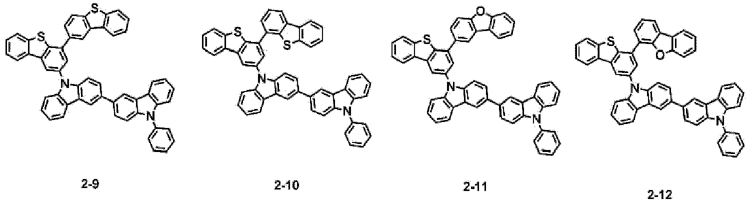

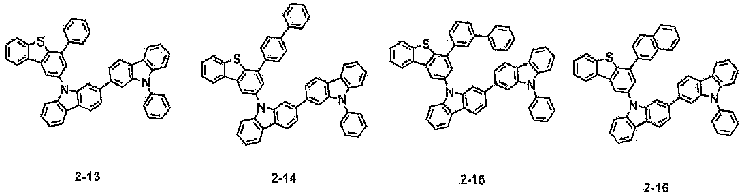

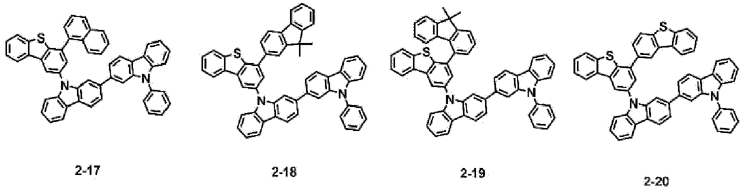

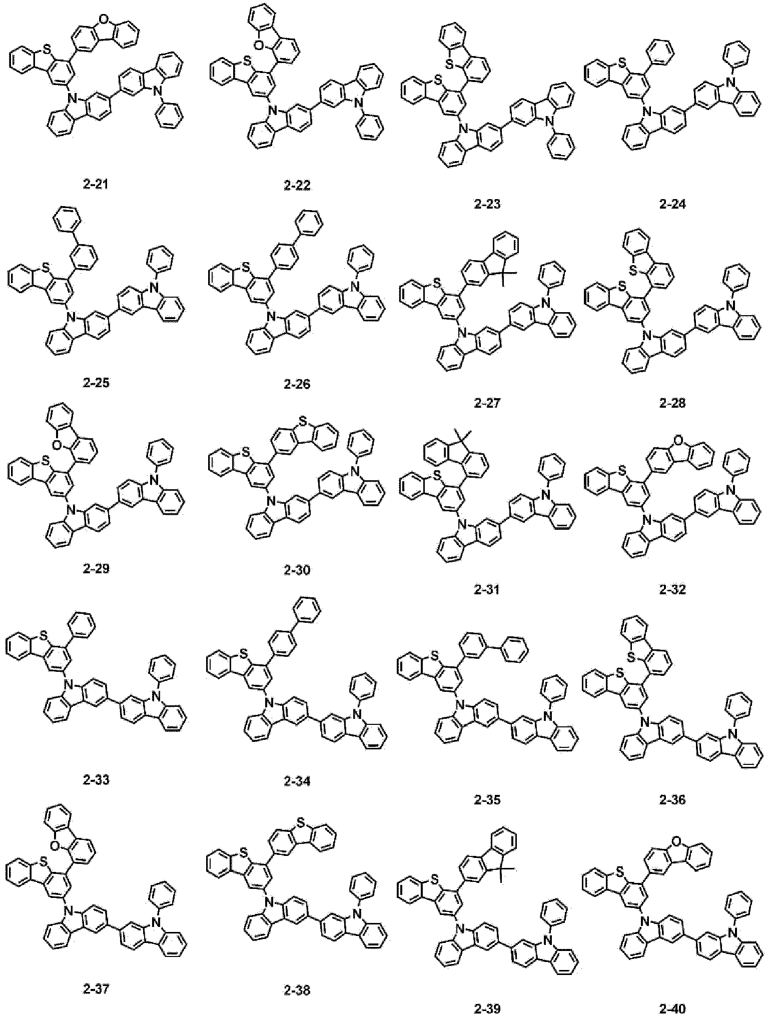

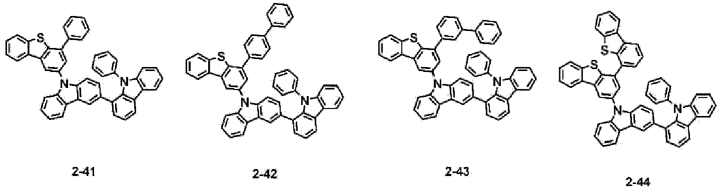

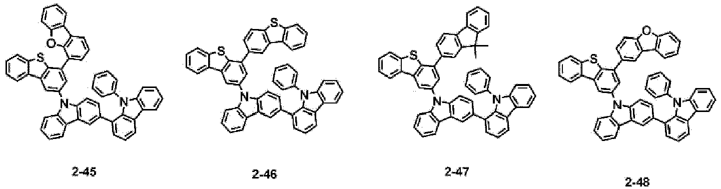

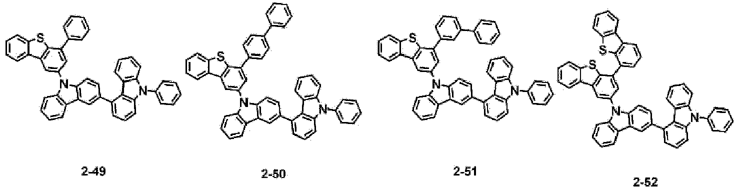

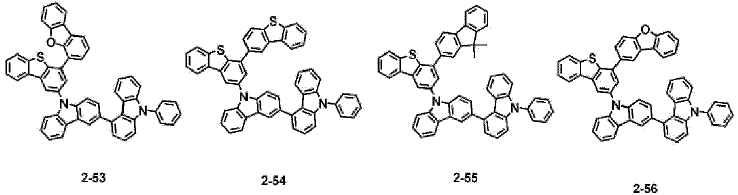

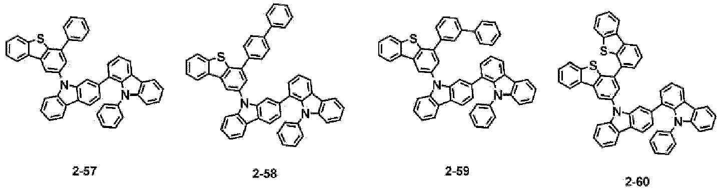

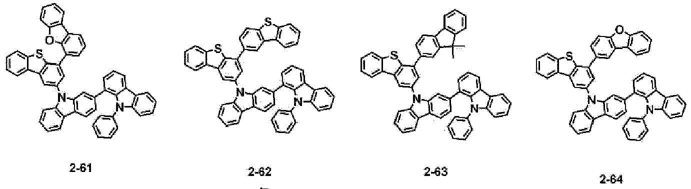

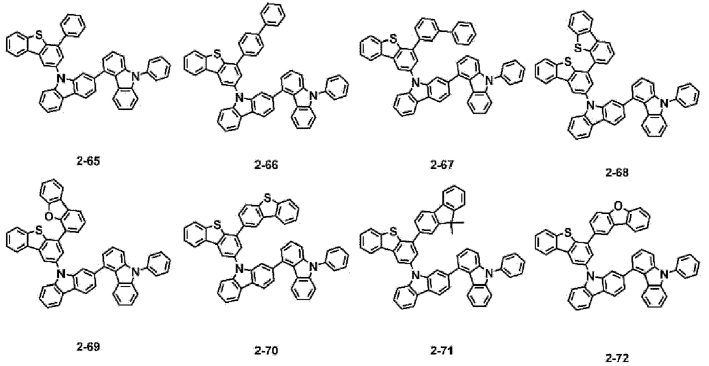

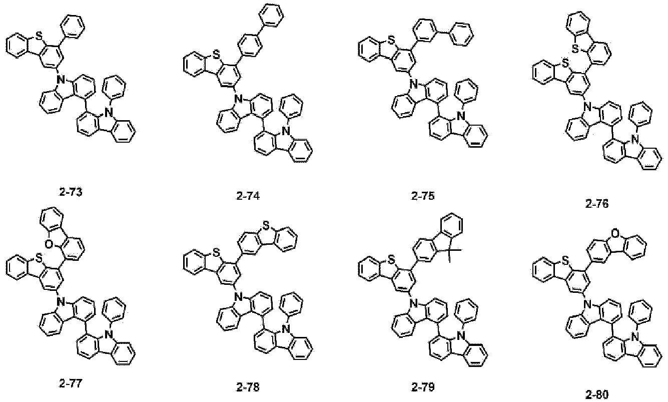

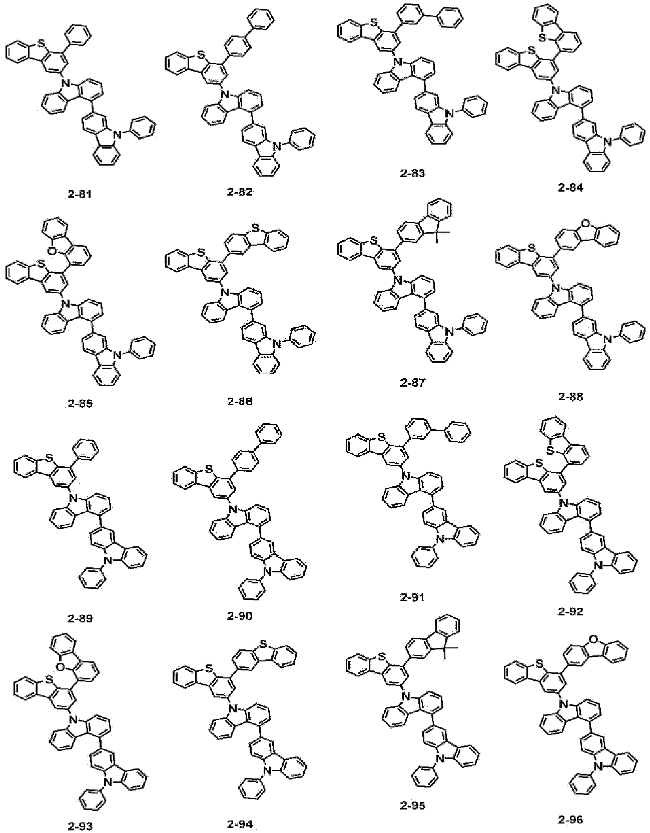

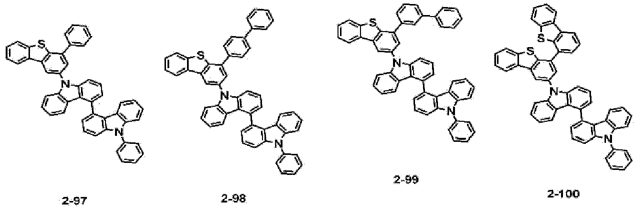

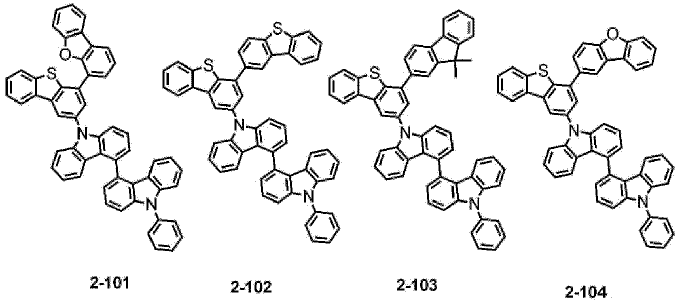

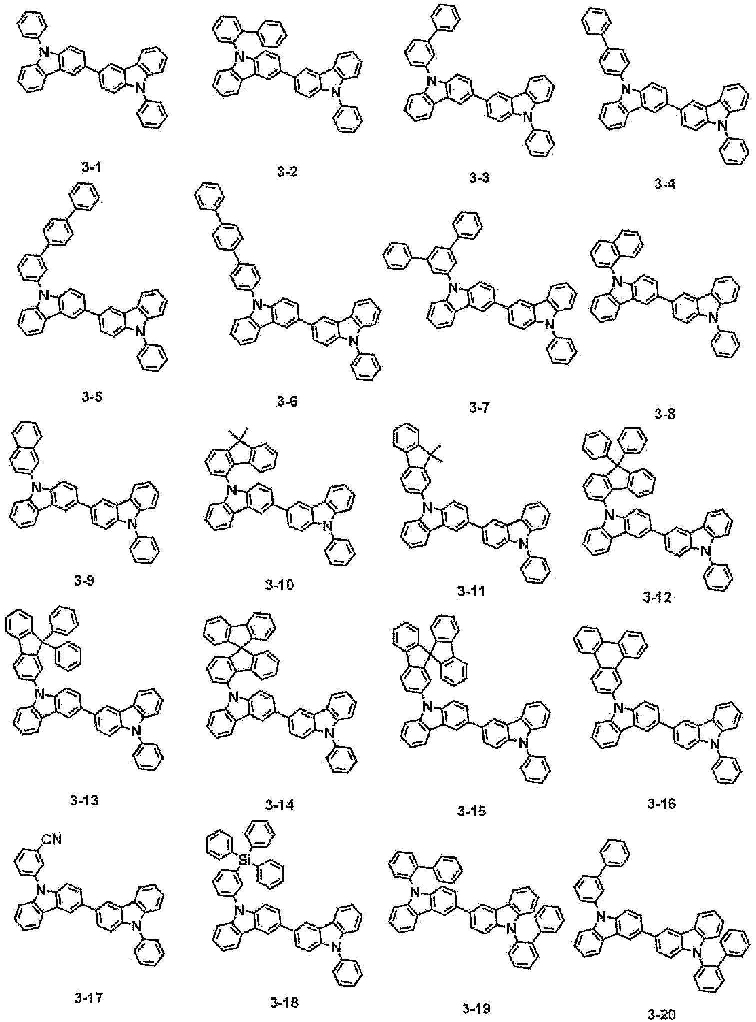

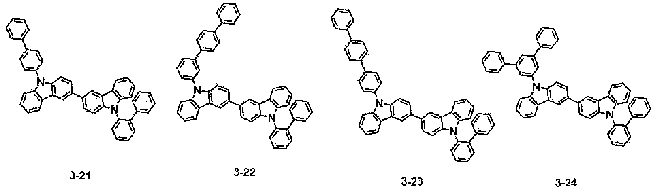

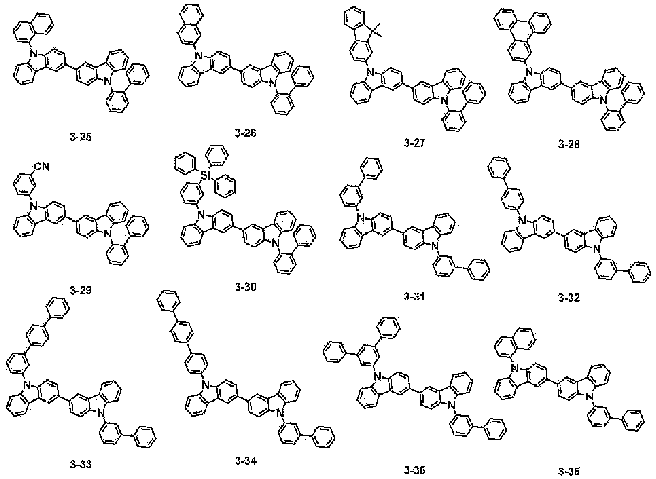

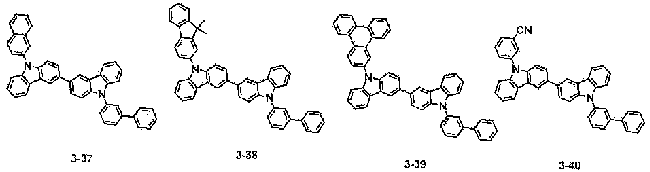

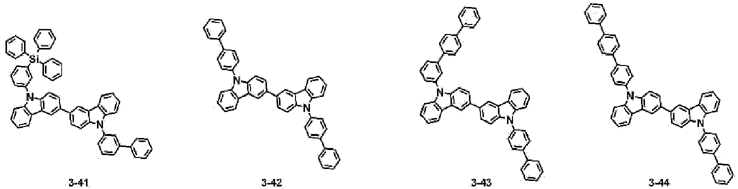

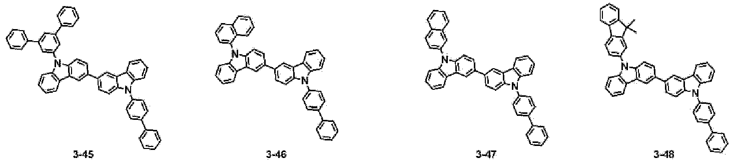

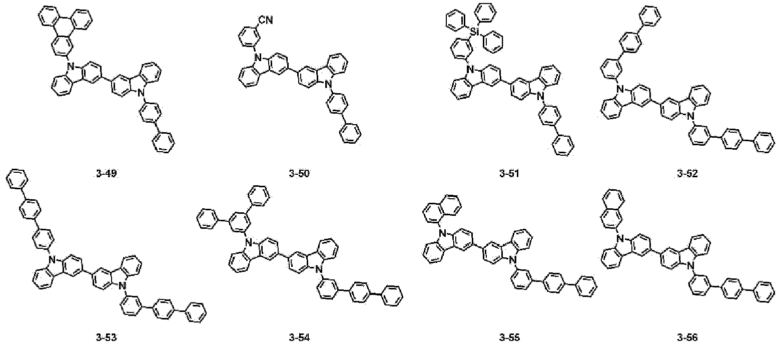

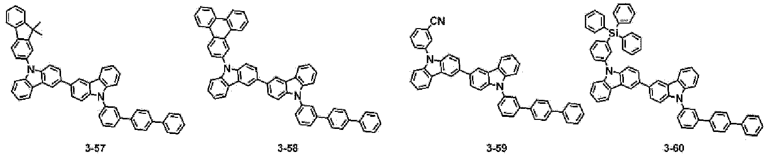

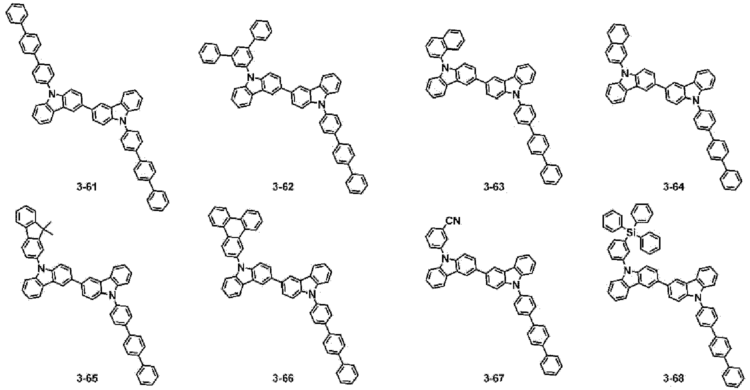

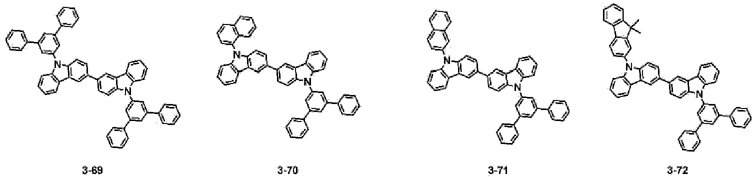

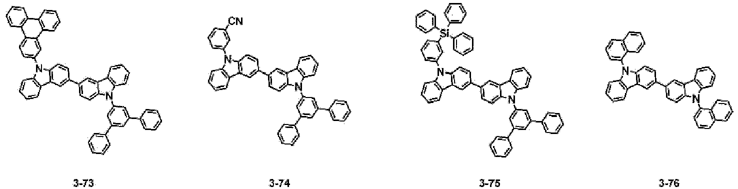

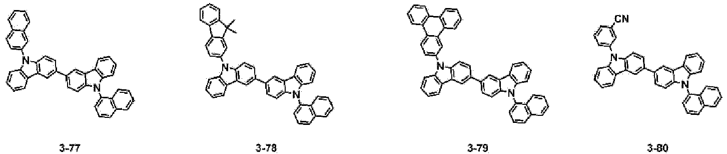

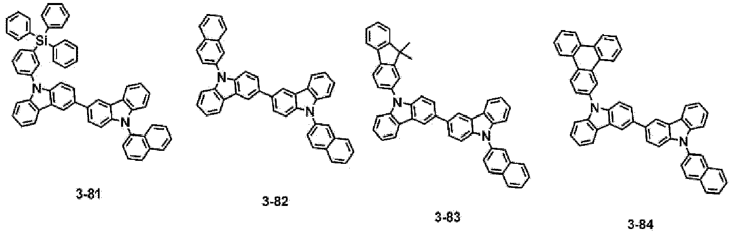

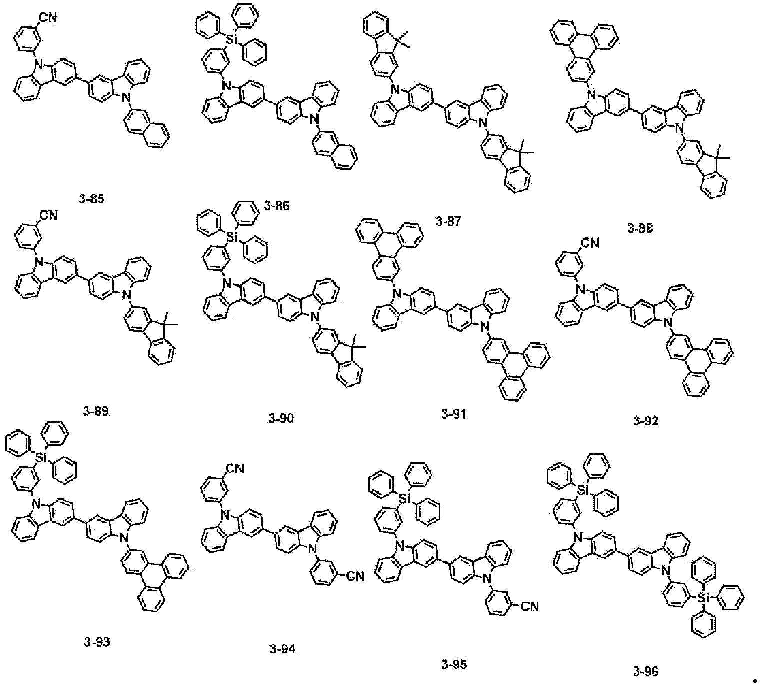

10. The organic light emitting device of Claim 7, wherein Rc and Rd are hydrogen.

11. The organic light emitting device of Claim 6, wherein the organic material layer includes a light emitting layer, and the light emitting layer includes the heterocyclic compound.

12. The organic light emitting device of Claim 6, wherein the organic material layer includes a light emitting layer, the light emitting layer includes a host material, and the host material includes the heterocyclic compound.

13. The organic light emitting device of Claim 6, further comprising one, two or more layers selected from the group consisting of a light emitting layer, a hole injection layer, a hole transfer layer, an electron injection layer, an electron transfer layer, an electron blocking layer and a hole blocking layer.

14. A composition for an organic material layer of an organic light emitting device, the composition comprising:
the heterocyclic compound of any one of Claims 1 to 5; and
a heterocyclic compound represented by the following Chemical Formula 2: 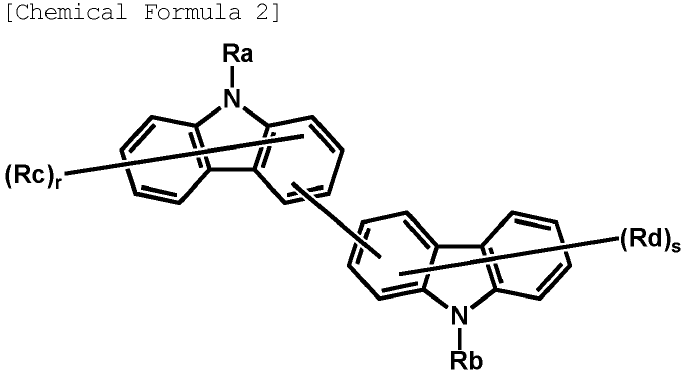 in Chemical Formula 2,
Rc and Rd are the same as or different from each other, and each independently selected from the group consisting of hydrogen; deuterium; a halogen group; -CN; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -Si(R10) (R11) (R12); - P(=O) (R10) (R11); and a substituted or unsubstituted amine group, or two or more groups adjacent to each other bond to each other to form a substituted or unsubstituted C6 to C60 aromatic hydrocarbon ring or a substituted or unsubstituted C2 to C60 heteroring;
R10, R11 and R12 are the same as or different from each other, and each independently hydrogen; deuterium; -CN; a substituted or unsubstituted C1 to C60 alkyl group; a substituted or unsubstituted C6 to C60 aryl group; or a substituted or unsubstituted C2 to C60 heteroaryl group;
Ra and Rb are the same as or different from each other, and each independently a substituted or unsubstituted C6 to C60 aryl group; or a substituted or unsubstituted C2 to C60 heteroaryl group;
r and s are each an integer of 0 to 7; and
when r and s are each 2 or greater, substituents in each parenthesis are the same as or different from each other.

15. The composition for an organic material layer of an organic light emitting device of Claim 14, wherein the heterocyclic compound:the heterocyclic compound represented by Chemical Formula 2 have a weight ratio of 1:10 to 10:1 in the composition.

16. A method for manufacturing an organic light emitting device, the method comprising:
preparing a substrate;
forming a first electrode on the substrate;
forming one or more organic material layers on the first electrode; and
forming a second electrode on the organic material layer,
wherein the forming of organic material layers includes forming one or more organic material layers using the composition for an organic material layer of Claim 14.

17. The method for manufacturing an organic light emitting device of Claim 16, wherein the forming of organic material layers is forming using a thermal vacuum deposition method after pre-mixing the heterocyclic compound and the heterocyclic compound of Chemical Formula 2.
